(19) **Europäisches Patentamt / European Patent Office / Office européen des brevets**

(11) **EP 3 394 017 B1**

(12) **FASCICULE DE BREVET EUROPEEN**

(45) Date de publication et mention de la délivrance du brevet:
**25.10.2023 Bulletin 2023/43**

(21) Numéro de dépôt: **16819037.9**

(22) Date de dépôt: **14.12.2016**

(51) Classification Internationale des Brevets (IPC):
**C07C 17/25** (2006.01)   **C07C 17/38** (2006.01)
**C07C 19/10** (2006.01)   **C07C 21/18** (2006.01)
**C07C 17/386** (2006.01)   **C09K 5/04** (2006.01)

(52) Classification Coopérative des Brevets (CPC):
(C-Sets disponibles)
**C07C 17/386;** C09K 5/045; C09K 2205/122;
C09K 2205/126                                           (Cont.)

(86) Numéro de dépôt international:
**PCT/EP2016/080944**

(87) Numéro de publication internationale:
**WO 2017/108518 (29.06.2017 Gazette 2017/26)**

(54) **PROCÉDÉ DE PRÉPARATION DU 2,3,3,3-TETRAFLUORO-1-PROPÈNE ET RECYCLAGE DU 2-CHLORO-3,3,3-TRIFLUOROPROPÈNE EXEMPT D'IMPURETÉS**

VERFAHREN ZUR HERSTELLUNG VON 2,3,3,3-TETRAFLUOR-1-PROPEN UND ZUM RECYCLING VON 2-CHLOR-3,3,3-TRIFLUORPROPEN, DAS FREI VON VERUNREINIGUNGEN IST

METHOD FOR PREPARING 2,3,3,3-TETRAFLUORO-1-PROPENE AND RECYCLING 2-CHLORO-3,3,3-TRIFLUOROPROPENE FREE OF IMPURITIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.12.2015 FR 1563164**

(43) Date de publication de la demande:
**31.10.2018 Bulletin 2018/44**

(73) Titulaire: **Arkema France**
**92700 Colombes (FR)**

(72) Inventeurs:
• **BABA-AHMED, Abdelatif**
**69190 Saint-fons (FR)**
• **COLLIER, Bertrand**
**69230 Saint-genis-laval (FR)**
• **DEUR-BERT, Dominique**
**69390 Charly (FR)**

• **WENDLINGER, Laurent**
**69510 Soucieu En Jarrest (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**EP-A1- 0 921 109          EP-B1- 0 743 934
WO-A1-2008/054781     WO-A1-2014/147311
WO-A1-2014/147312     US-A- 5 470 442
US-B2- 7 371 309**

(52) Classification Coopérative des Brevets (CPC):
(Cont.)

C-Sets
**C07C 17/386, C07C 19/10**

## Description

### Domaine technique de l'invention

L'invention se rapporte à un procédé de préparation du 2,3,3,3-tétrafluoro-1-propène et est définie dans les revendications. Plus particulièrement, l'invention se rapporte à un procédé de préparation de 2,3,3,3-tétrafluoro-1-pro-pène incluant la purification et le recyclage du 2-chloro-3,3,3-trifluoropropène également issu de la réaction.

### Arrière-plan technologique de l'invention

Les hydrofluorocarbures (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. Les HFO ont été identifiés comme des alternatives souhaitables au HCFC du fait de leurs faibles valeurs d'ODP (Ozone Depletion Potential ou potentiel d'appauvrissement de la couche d'ozone) et de GWP (Global Warming Potential ou potentiel de réchauffement climatique).

La plupart des procédés de fabrication des hydrofluorooléfines font appel à une réaction de fluoration et/ou de déshydrohalogénation. Ce type de réaction est effectué en phase gazeuse et génère des impuretés qu'il faut par conséquent éliminer pour obtenir le composé désiré dans un degré de pureté suffisant pour les applications visées.

Par exemple, dans le cadre de la production de 2,3,3,3-tétrafluoro-1-propène (HFO-1234yf), la présence d'impuretés telles que le 1-chloro-3,3,3-trifluoro-1-propene (1233zd), 1,3,3,3-tetrafluoro-1-propene (1234ze) et le 1,1,1,3,3-pentafluoropropane (245fa) sont observées. Ces impuretés sont des isomères des composés principaux visant à être obtenus par le procédé de production du 2,3,3,3-tétrafluoro-1-propène outre ce dernier, i.e. 2-chloro-3,3,3-trifluoro-1-propene (1233xf) et 1,1,1,2,2-pentafluoropropane (245cb). Compte tenu des points d'ébullition respectifs du 1-chloro-3,3,3-trifluoro-1-propene (1233zd), 1,3,3,3-tetrafluoro-1-propene (1234ze) et le 1,1,1,3,3-pentafluoropropane (245fa), ceux-ci peuvent s'accumuler dans la boucle des produits recyclés au réacteur et ainsi empêcher la formation des produits d'intérêt.

La purification de ce type de mélange réactionnel peut être effectuée par différentes techniques connues de l'art antérieur, telles que par exemple la distillation. Cependant lorsque les composés à purifier ont des points d'ébullition trop proches ou lorsque ceux-ci forment des compositions azéotropiques ou quasi-azéotropiques, la distillation n'est pas un procédé efficace. Des procédés de distillation extractive ont ainsi été décrits.

On connaît par EP 0 864 554 un procédé de purification d'un mélange comprenant 1,1,1,3,3-pentafluoropropane (245fa) et 1-chloro-3,3,3-trifluoro-trans-1-propene (1233zd) par distillation en présence d'un solvant ayant un point d'ébullition supérieur à celui de 1-chloro-3,3,3-trifluoro-trans-1-propene.

On connaît par WO 03/068716 un procédé de récupération de pentafluoroéthane à partir d'un mélange comprenant du pentafluoroéthane et du chloropentafluoroéthane par distillation en présence d'hexafluoropropène.

On connaît aussi par WO 98/19982 un procédé de purification du 1,1-difluoroéthane par distillation extractive. Le procédé consiste à mettre en contact un agent d'extraction avec un mélange de 1,1-difluoroéthane et de chlorure de vinyle. L'agent d'extraction est choisi parmi les hydrocarbures, les alcools, les chlorocarbures ayant un point d'ébullition compris entre 10°C et 120°C.

On connaît par WO 2008/054781 un procédé de production d'au moins un composé sélectionné parmi le groupe consistant en CF3CF2CH3, CF3CF=CH2 et CF3CCl=CH2.

Comme mentionné par WO 98/19982, la sélection de l'agent d'extraction peut s'avérer complexe en fonction des produits à séparer. Il existe donc toujours un besoin pour la mise en oeuvre d'un procédé particulier de purification du 2-chloro-3,3,3-trifluoropropène.

### Résumé de l'invention

Dans un procédé de production du 2,3,3,3-tetrafluoro-1-propène, le choix de conditions opérationnelles particulières permet de favoriser la présence de certaines impuretés ou d'isomères de celles-ci. La présence d'impuretés telles que 1-chloro-3,3,3-trifluoro-1-propene (1233zd) et 1,1,1,3,3-pentafluoropropane (245fa) peut être observée. Ces impuretés peuvent provenir de réactions secondaires induites par des composés produits intermédiairement pendant la production du 2,3,3,3-tetrafluoro-1-propène, et peuvent présenter des propriétés physiques telles que la séparation de celles-ci avec le 2-chloro-3,3,3-trifluoropropene (1233xf) peut s'avérer complexe. Il y a donc un intérêt à traiter la boucle de réaction pour limiter la présence des impuretés. En outre, La présente invention permet, outre la production de 2,3,3,3-tetrafluoro-1-propène, la récupération et le recyclage du 2-chloro-3,3,3-trifluoropropene (1233xf) produit avec une excellente pureté.

Selon un premier aspect, la présente invention fournit un procédé de purification du 2-chloro-3,3,3-trifluoropropène (1233xf) à partir d'une première composition comprenant du 2-chloro-3,3,3-trifluoropropène et 1,1,1,3,3-pentafluoropropane (245fa), ledit procédé comprenant les étapes de:

a) mise en contact de ladite première composition avec au moins un agent d'extraction organique pour former une seconde composition ;
b) distillation extractive de ladite seconde composition pour former :

i) une troisième composition comprenant ledit agent d'extraction organique et 1,1,1,3,3-pentafluoropropane (245fa),
ii) un courant comprenant le 2-chloro-3,3,3-trifluoropropène;

c) récupération et séparation de ladite troisième composition pour former un courant comprenant ledit agent d'extraction organique et un courant comprenant 1,1,1,3,3-pentafluoropropane (245fa); caractérisé en ce que ledit agent d'extraction organique est sélectionné parmi le groupe consistant en éthanedial, propanone, methylacetate, chloromethoxymethane, isobutanal, methanol, isopropylformate, methylglyoxal, 2,2,2-trifluoroethanol, 1,1,1-trifluoro-2-propanol, ethylacetate, ethanol, butanone, n-propylformate, 2-propanol, acetonitrile, pentafluoro-1-propanol, tert-butanol, 1,2-dimethoxyethane, isopropylacetate, 2-methylbutanal, 2-methoxyethanamine, tert-butylacetate, propionitrile, 2-allyloxyethanol, ethylpropionate, dioxane, 3-pentanone, 2-pentanone, trimethoxymethane, 3,3-dimethyl-2-butanone, 1,3-dioxane, 2,2,3,3-tetraflouro-1-propanol, sec-butylacetate, n-methyl-1,2-ethanediamine, 1,3,5-trioxane, 4-methyl-2-pentanone, 1,2-diaminoethane, butyronitrile, 1-methoxy2-propanol, 1,2-propanediamine, perfluorobutanoicacid, 1-(dimethylamino)-2-propanol, 2-methoxyethanol, 4,4,4-trifluorobutanol, diethylcarbonate, n-butylacetate, 2-hexanone, 3-fluoropropanol, 5-hexen-2-one, 2-methoxy1-propanol, 1-ethoxy-2-propanol, 2-(dimethylamino)-ethanol, ethoxyethanol, 2-ethoxy-1-propanol, 1,3-propanediamine, 3,3,4,4,5,5,6,6-octafluoro-1-pentanol, valeronitrile, (methyleneamino)acetonitrile, 3-furfural, 4-methyl-2-hexanone, 1-methoxy-2-acetoxypropane, methylhexanoate, 2-propoxyethanol, 1-propoxy-2-propanol, dimethylethanolamine, 2-heptanone, dimethylformamide, 2-isopropoxyethanol, cyclohexanone, 2-ethoxyethanolacetate, 3-hydroxy-butanal, 2-(methylamino)-ethanol, 4-methoxy-4-methyl-pentan-2-one, 3-ethoxy-1-propanol, 3-methoxy-1-butanol, furfural, diglyme, 3-methoxypropanenitrile, 2-(ethylamino)ethanol, diacetone alcohol, 1-butoxy-2-propanol, 2-furanmethanol, methylacetoacetate, 2-aminopropanol, n,n-dimethylpropanamide, 2-amino-1-butanol, 2-methyl-2-nitro-1-propanol, 2-propanol-1-methoxy-propanoate, cycloheptanone, dimethylmalonate, 1,1,3,3-tetramethoxypropane, dimethylsulfate, dimethylsulfoxide, 2-butoxyethanolacetate, diethylsulfoxide, 2-(2-methoxyethoxy)ethanol, 1,2-butanediol, diethyleneglycolmonoethylether, trimethylphosphate, 2-methyl-2,4-pentanediol, methylbenzoate, diethylmalonate.

De préférence, le courant comprenant l'agent d'extraction organique peut être recyclé à l'étape a). De préférence, le courant comprenant 1,1,1,3,3-pentafluoropropane (245fa) peut être incinéré ou récupéré pour pouvoir purifier un ou plusieurs des produits le constituant.

Selon un mode de réalisation préféré, ledit agent d'extraction organique est sélectionné parmi le groupe consistant en éthanedial, propanone, methylacetate, methylglyoxal, ethylacetate, butanone, propionitrile, dioxane, trimethoxymethane, 1,3-dioxane, 1,3,5-trioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, methylacetoacetate, n,n-dimethylpropanamide, dimethylmalonate, diethylsulfoxide, 2-(2-methoxyethoxy)ethanol, trimethylphosphate, diethylmalonate ; de préférence ledit agent d'extraction organique est choisi parmi le groupe consistant en propanone, methylacetate, ethylacetate, butanone, dioxane, trimethoxymethane, 1,3-dioxane, 1,3,5-trioxane, 1,2-diaminoethane, 1-methoxy-2-propanol.

Selon un autre mode de réalisation, la présente invention fournit un procédé de purification du 2-chloro-3,3,3-trifluoropropène (1233xf) à partir d'une première composition comprenant du 2-chloro-3,3,3-trifluoropropène et E-1-chloro-3,3,3-trifluoro-1-propene (1233zdE), ledit procédé comprenant les étapes de:

a) mise en contact de ladite première composition avec au moins un agent d'extraction organique pour former une seconde composition ;
b) distillation extractive de ladite seconde composition pour former :

i) une troisième composition comprenant ledit agent d'extraction organique et E-1-chloro-3,3,3-trifluoro-1-propene (1233zdE),
ii) un courant comprenant le 2-chloro-3,3,3-trifluoropropène;

c) récupération et séparation de ladite troisième composition pour former un courant comprenant ledit agent d'extraction organique et un courant comprenant E-1-chloro-3,3,3-trifluoro-1-propene (1233zdE) ; caractérisé en ce que ledit agent d'extraction organique est sélectionné parmi le groupe consistant en isopropylmethylamine, ethanedial, 2-chloro-2-methylpropane, 2-propanethiol, 2-ethoxy-propane, 1-chloro-2,2-difluoropropane, methyl-t-butylether, diethylamine, propanone, methylacetate, 1,1-dichloroethane, 4-methoxy-2-methyl-2-butanethiol, 2-bromopropane, chloromethoxymethane, difluorodiethylsilane, 2-butanamine, n-methylpropylamine, tert-butylthiol, isobutanal, tetrahydrofurane, 1-propanethiol, 2-chlorobutane, isopropylformate, diisopropylether, 1-bromopropane, methylglyoxal, 2-ethoxy-2-methyl-propane, 2-bromo-2-methylpropane, 1-chloro-3-fluoropropane, 1,1,1-trifluoro-2-propanol, 1-butylamine, ethylacetate, 1-chlorobutane, ethanol, butanone, n-propylformate, 2-ethoxy-butane, 2-propanol, acetonitrile, tert-butanol, 1-methoxy-2-methyl-butane, cyclohexene, 2,2-dimethoxypropane, 2-chloro-2-methylbutane, 1,2-dichloroethane, 1-ethoxy-2-methylpropane, diisopropylamine, 2-butanethiol, 1,2-dimethoxyethane, 3-methyl-2-butanamine, diethoxymethane, 1,1-dichloropropane, 2-methyl-1-propanethiol, isopropylacetate, 1,2-dichloro-2-fluoropropane, di-n-propylether,, 3-pentylamine, n-methylbutylamine, 2-bromobutane, diethylsulfide, 1-ethoxybutane, 1-fluorohexane, 1-methoxy-2-propanamine, 2,3-dichloro-1-propene, 2-methylbutanal, 2-methoxyethanamine, 1,2-dichloropropane, propanol, tert-butylacetate, propionitrile, 3-chloropentane, trichloroacetaldehyde, 2-allyloxyethanol, butanethiol, isoamylchloride, 1-methoxy-pentane, ethylpropionate, 2-butanol, 1,2-dimethoxypropane, isopropyl-isobutyl-ether, 1-chloro-4-fluorobutane, 2,4,4-trimethyl-1-pentene, 1-bromo-3-fluoropropane, dioxane, 1-bromobutane, 3-pentanone, 1,1-diethoxyethane, 2-pentanone, 2-methyl-2-butanol, 2-methoxy-1propanamine, trimethoxymethane, 2,2-dichlorobutane, cis-1,3-dichloropropene, n-pentylamine, 3,3-dimethyl-2-butanone, 1,3-dioxane, piperidine, isobutanol, 2-bromo-2-methylbutane, dipropylamine, 2-ethoxyethanamine, triethylfluorosilane, 1-methylcyclohexene, sec-butylacetate, trans-1,3-dichloropropene, 2,2-dimethyl-1-propanol, 1,1,1-trichloro-3-fluoropropane, n-methyl-1,2-ethanediamine, 2,2-diethoxypropane, 1,3,5-trioxane, 1-chloro-3,3-dimethylbutane, pyridine, n-methylmorpholine, 3-pentanol, 4-methyl-2-pentanone, 1,2-diaminoethane, isobutyl-tert-butylether, 2-bromopentane, butyronitrile, 1-butanol, 2,3-dichlorobutane, sec-butyl-tert-butylether, 1-methoxy2-propanol, 1,2-propanediamine, 2,6-dimethyl-5-heptenal, 1-bromo-3-methylbutane, 1,3-dichloro-trans-2-butene, 1,3-dichloropropane, 1-(dimethylamino)-2-propanol, tetrahydrothiophene, 3-methyl-3-pentanol, 1,1-diethoxypropane, 1,2,2-trichloropropane, 1-chloro-2-methyl-2-propanol, 2-methoxyethanol, 1,2-dichlorobutane, 4,4,4-trifluorobutanol, 2-ethylbutylamine, diethylcarbonate, n-butylacetate, 1-pentanethiol, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxy1-propanol, 1,2-dichloro-2-butene, hexanal, 1-ethoxy-2-propanol, 4-methyl-2-pentanol, bromoaceticacidmethylester, 1,2-octanediol, 4-methyl-2-hexanamine, hexylamine, methoxycyclohexane, 2-(dimethylamino)-ethanol, 1,3-dichlorobutane, cyclohexylamine, n-ethyl-2-dimethylaminoethylamine, ethoxyethanol, 3-hexanol, 2-hexanol, 2-methylpyrazine, 2-ethoxy-1-propanol, 1-pentanol, n-ethyl-morpholine, 1-methylpiperazine, 1,4-dimethylbenzene, 1,3-propanediamine, di-n-butylether, valeronitrile, (methyleneamino)acetonitrile, 2-heptanamine, 2,3-dimethylbutanol, 1-ethoxy-hexane, 1-chloro-3-bromopropane, n,n-diethylethylenediamine, 3-furfural, 2,6-dimethylpyridine, 4-methyl-2-hexanone, 1,1,1-triethoxyethane, styrene, 1-methoxy-2-acetoxypropane, 4-methylpyridine, n,n'-diethyl-1,2-ethanediamine, 2,6-dimethylmorpholine, 2-ethyl-1-butanol, 1,2-dichloropentane, 2-methyl-1-pentanol, methylhexanoate, 2-propoxyethanol, 1-propoxy-2-propanol, dimethylethanolamine, isopropylchloroacetate, 2-heptanone, 1-hexanethiol, 1,2-propanedithiol, heptanal, dimethylformamide, 2,6-dimethylpyrazine, 2-isopropoxyethanol, diethyldisulfide, 2-methylpiperazine, 1-methylcyclohexanol, 1-bromohexane, cyclohexanone, 1-heptanamine, 2,3-dimethylpyrazine, 2-ethoxyethanolacetate, 3-hydroxy-butanal, 1-hexanol, 2-(methylamino)-ethanol, 1,4-butanediamine, 2,4-dimethylpyridine, 2-pyrimidinamine, 2-heptanol, 2-methoxy-3-methylpyrazine, dibutylamine, 4-methoxy-4-methyl-pentan-2-one, 1,4-dichloro-trans-2-butene, 3-ethoxy-1-propanol, cyclohexanol, 1,4-dichlorobutane, 3-methoxy-1-butanol, furfural, diglyme, 1,1,1-trichloro-2-propanol, 2-(diethylamino)-ethanol, 3-methoxypropanenitrile, 2,2-diethoxyethanamine, 1,3,5-trimethylbenzene, 2-methoxy-n-(2-methoxyethyl)ethanamine, 2-chloropyridine, bromocyclohexane, 2,3-heptanedione, 2-(ethylamino)ethanol, 3-methylcyclohexanol, 1,3-dibromopropane, 2-methylcyclohexanol, 3-octanone, diacetone alcohol, diethylaminopropylamine, 2-ethylhexylamine, 1,3-propanedithiol, ethoxybenzene, 1-butoxy-2-propanol, 2-furanmethanol, 2-butoxyethanol, methylacetoacetate, 2-octanone, 2-aminopropanol, 1,4-dichlorobenzene, methylheptanoate, triethylenediamine, n,n-dimethylpropanamide, 2-chlorophenol, 2-amino-1-butanol, 1-heptanol, 2-methyl-2-nitro-1-propanol, 2-propanol-1-methoxy-propanoate, 1,5-pentanediamine, 2-octanol, cycloheptanone, 3,4-dimethylpyridine, 1-octanamine, benzylmethylamine, dimethylmalonate, 1-chlorooctane, cyclohexanemethanol, 1,1,3,3-tetramethoxypropane, 2-(ethylthio)-ethanol, 1-ethoxy-2-methylbenzene, aniline, 2-ethyl-1-hexanol, tert-butylcyclohexylamine, dihexylphthalate, 1-fluorodecane, 2-chloro-1,4-dimethylbenzene, dimethylsulfate, dimethylsulfoxide, diethylpropanolamine, 2-methylphenol, 2-butoxyethanolacetate, diethylsulfoxide, 1-octanol, 2-bromopyridine, 2-(2-methoxyethoxy)ethanol, 1,2-butanediol, 2-bromophenol, 4-methylbenzenemethanamine, diethyleneglycolmonoethylether, 1,2,4,5-tetramethylbenzene, 2-propylcyclohexanone, 1,4-dibromobutane, trimethylphosphate, 2-methyl-2,4-pentanediol, 1,3-diethenylbenzene, methylbenzoate, 1-octanethiol, diethylmalonate, 2-methoxypyrimidine.

Selon un mode de réalisation préféré, ledit agent d'extraction organique est sélectionné parmi le groupe consistant en isopropylmethylamine, methyl-t-butylether, diethylamine, propanone, methylacetate, 2-butanamine, n-methylpropylamine, tetrahydrofurane, 1-butylamine, ethylacetate, butanone, n-propylformate, dimethoxypropane, diisopropylamine, 1,2-dimethoxyethane, 3-methyl-2-butanamine, diethoxymethane, isopropylacetate, 3-pentylamine, n-methylbutylamine, 1-methoxy-2-propanamine, 2-methoxyethanamine, tert-butylacetate, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 1,2-diaminoethane, 1-methoxy2-propanol, 1,2-propanediamine, 2,6-dimethyl-5-heptenal, 1-(dimethylamino)-2-propanol, 3-methyl-3-pentanol, 2-ethylbutylamine, diethylcarbonate, n-butylacetate, 2-hexanone, n-ethylethylenediamine, 2-methoxy1-propanol, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-ethoxy-1-propanol, 1-methylpiperazine, 1,3-propanediamine, 2-heptanamine, n,n-diethylethylenediamine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol, 2-heptanone, dimethylformamide, 2-isopropoxyethanol, 2-methylpiperazine, cyclohexanone, 1-heptanamine, 2-ethoxyethanolacetate, 1,4-butanediamine, 2,4-dimethylpyridine, 2-methoxy-3-methylpyrazine, 4-methoxy-4-methyl-pentan-2-one, 3-ethoxy-1-propanol, 3-methoxy-1-butanol, diglyme, 2-(diethylamino)-ethanol, 2,2-diethoxyethanamine, 2-methoxy-n-(2-methoxyethyl)ethanamine, 2-(ethylamino)ethanol, 3-octanone, diacetone alcohol, diethylaminopropylamine, 2-ethylhexylamine, 1-butoxy-2-propanol, 2-butoxyethanol, 2-octanone, methylheptanoate, triethylenediamine, n,n-dimethylpropanamide, 2-propanol-1-methoxy-propanoate, 1,5-pentanediamine, cycloheptanone, 3,4-dimethylpyridine, 1-octanamine, benzylmethylamine, 1,1,3,3-tetramethoxypropane, dihexylphthalate, diethylpropanolamine, 2-butoxyethanolacetate, diethylsulfoxide, 2-(2-methoxyethoxy)ethanol, 4-methylbenzenemethanamine, diethyleneglycolmonoethylether, 2-propylcyclohexanone, trimethylphosphate, 2-methyl-2,4-pentanediol, methylbenzoate, diethylmalonate, 2-methoxypyrimidine ; de préférence ledit agent d'extraction organique est choisi parmi le groupe consistant en diethylamine, propanone, methylacetate, tetrahydrofurane, ethylacetate, butanone, diethoxymethane, isopropylacetate, tert-butylacetate, dioxane, 3-pentanone, 1,1-diethoxyethane, 2-pentanone, n-pentylamine, 1,3-dioxane, sec-butylacetate, 1,2-diaminoethane, 1-methoxy2-propanol, n-butylacetate, 1-ethoxy-2-propanol.

Selon un mode de réalisation préféré, l'agent d'extraction organique séparé à l'étape c) est recyclé à l'étape a).

Selon un mode de réalisation préféré, ledit courant comprenant le 2-chloro-3,3,3-trifluoropropène formé à l'étape b) est récupéré en tête de colonne de distillation et recyclé optionnellement dans un procédé de production du 2,3,3,3-tétrafluoropropène.

Selon un mode de réalisation particulier, ledit agent d'extraction organique peut être choisi parmi le groupe consistant en propanone, methylacetate, ethylacetate, butanone, dioxane, trimethoxymethane, 1,3-dioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, n,n-dimethylpropanamide, diethylsulfoxide, 2-(2-methoxyethoxy)ethanol, trimethylphosphate, diethylmalonate ; de préférence ledit agent d'extraction organique est choisi parmi le groupe consistant propanone, methylacetate, ethylacetate, butanone, dioxane, trimethoxymethane, 1,3-dioxane, 1,2-diaminoethane, 1-methoxy2-propanol, 3-methoxy-1-butanol, diacetone alcohol. De préférence, ce mode de réalisation particulier peut permettre de séparer efficacement et simultanément le 2-chloro-3,3,3-trifluoropropène, de E-1-chloro-3,3,3-trifluoro-1-propene (1233zdE) et 1,1,1,3,3-pentafluoropropane (245fa).

Selon un mode de réalisation particulier, la première composition est une composition azéotropique ou quasi-azéotropique comprenant 2-chloro-3,3,3-trifluoropropène et E-1-chloro-3,3,3-trifluoro-1-propene (1233zdE) ; ou la première composition est une composition azéotropique ou quasi-azéotropique comprenant 2-chloro-3,3,3-trifluoropropène et 1,1,1,3,3-pentafluoropropane (245fa).

Selon un second aspect de la présente invention, un procédé de production de 2,3,3,3-tetrafluoro-1-propène est fourni. Ledit procédé comprend les étapes de :

A) dans un réacteur, fluoration en présence d'un catalyseur d'un composé de formule $CX(Y)_2\text{-}CX(Y)_m\text{-}CH_mXY$ (I) dans laquelle X et Y représentent indépendamment H, F, ou Cl et m = 0 ou 1 ; et/ou fluoration en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant, de préférence issu d'une purge de la boucle réactionnelle de recyclage, comprenant du 1,1,1,2,2-pentafluoropropane, du 2-chloro-3,3,3-trifluoropropène, et au moins un des composés choisi parmi le groupe consistant en E-1-chloro-3,3,3-trifluoro-1-propene (1233zdE) et 1,1,1,3,3-pentafluoropropane (245fa),

C) distillation du courant récupéré à l'étape B) et récupération en tête de colonne de distillation d'un courant comprenant du 1,1,1,2,2-pentafluoropropane et en bas de colonne de distillation d'un courant comprenant du 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa) rt

D) mise en oeuvre du procédé de purification du 2-chloro-3,3,3-trifluoropropène selon la présente invention à partir du courant récupéré à l'étape C) et comprenant du 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa); et

E) recyclage à l'étape A) du courant comprenant le 2-chloro-3,3,3-trifluoropropène formé et récupéré à l'étape b) du procédé de purification mis en oeuvre à l'étape D).

Selon un mode de réalisation préféré, le courant comprenant du 1,1,1,2,2-pentafluoropropane récupéré à l'étape C) en tête de colonne de distillation peut être recyclé, par exemple dans la boucle réactionnelle, c'est-à-dire à l'étape A).

De préférence, le procédé de production de 2,3,3,3-tetrafluoro-1-propène est effectué dans un dispositif comprenant un réacteur et une boucle réactionnelle de recyclage. Cette dernière pouvant être purgée.

Selon un mode de réalisation préféré, le courant récupéré à l'étape B) comprenant également HF, 1,1,1,2,2-pentafluoropropane et 1,3,3,3-tetrafluoro-1-propene (1234ze), ceux-ci étant, préalablement à la mise en oeuvre de la distillation de l'étape C), traités selon les étapes suivantes :

i) séparation à basse température de ladite composition liquide pour former une première phase riche en HF et une seconde phase organique contenant 1,3,3,3-tetrafluoro-1-propene (1234ze), 1,1,1,2,2-pentafluoropropane et 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa);

ii) distillation de ladite seconde phase organique pour former et récupérer, avantageusement en tête de colonne de distillation, un premier courant comprenant 1,1,1,2,2-pentafluoropropane et 1,3,3,3-tetrafluoro-1-propene (1234ze), et pour former et récupérer, avantageusement en bas de colonne de distillation, un second courant comprenant du 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa);

iii) récupération dudit second courant et mise en oeuvre de l'étape D) à partir de celui-ci.

Selon un autre aspect, l'invention fournit une composition comprenant du 2-chloro-3,3,3-trifluoropropène (1233xf), E-1-chloro-3,3,3-trifluoro-1-propene (1233zdE) et un agent d'extraction organique sélectionné parmi le groupe consistant en isopropylmethylamine, methyl-t-butylether, diethylamine, propanone, methylacetate, 2-butanamine, n-methylpropylamine, tetrahydrofurane, 1-butylamine, ethylacetate, butanone, n-propylformate, -dimethoxypropane, diisopropylamine, 1,2-dimethoxyethane, 3-methyl-2-butanamine, diethoxymethane, isopropylacetate, 3-pentylamine, n-methylbutylamine, 1-methoxy-2-propanamine, 2-methoxyethanamine, tert-butylacetate, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 1,2-diaminoethane, 1-methoxy2-propanol, 1,2-propanediamine, 2,6-dimethyl-5-heptenal, 1-(dimethylamino)-2-propanol, 3-methyl-3-pentanol, 2-ethylbutylamine, diethylcarbonate, n-butylacetate, 2-hexanone, n-ethylethylenediamine, 2-methoxy1-propanol, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-ethoxy-1-propanol, 1-methylpiperazine, 1,3-propanediamine, 2-heptanamine, n,n-diethylethylenediamine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol, 2-heptanone, dimethylformamide, 2-isopropoxyethanol, 2-methylpiperazine, cyclohexanone, 1-heptanamine, 2-ethoxyethanolacetate, 1,4-butanediamine, 2,4-dimethylpyridine, 2-methoxy-3-methylpyrazine, 4-methoxy-4-methyl-pentan-2-one, 3-ethoxy-1-propanol, 3-methoxy-1-butanol, diglyme, 2-(diethylamino)-ethanol, 2,2-diethoxyethanamine, 2-methoxy-n-(2-methoxyethyl)ethanamine, 2-(ethylamino)ethanol, 3-octanone, diacetone alcohol, diethylaminopropylamine, 2-ethylhexylamine, 1-butoxy-2-propanol, 2-butoxyethanol, 2-octanone, methylheptanoate, triethylenediamine, n,n-dimethylpropanamide, 2-propanol-1-methoxy-propanoate, 1,5-pentanediamine, cycloheptanone, 3,4-dimethylpyridine, 1-octanamine, benzylmethylamine, 1,1,3,3-tetramethoxypropane, dihexylphthalate, diethylpropanolamine, 2-butoxyethanolacetate, diethylsulfoxide, 2-(2-methoxyethoxy)ethanol, 4-methylbenzenemethanamine, diethyleneglycolmonoethylether, 2-propylcyclohexanone, trimethylphosphate, 2-methyl-2,4-pentanediol, methylbenzoate, diethylmalonate, 2-methoxypyrimidine ; en particulier ledit agent d'extraction organique est choisi parmi le groupe consistant en diethylamine, propanone, methylacetate, tetrahydrofurane, ethylacetate, butanone, diethoxymethane, isopropylacetate, tert-butylacetate, dioxane, 3-pentanone, 1,1-diethoxyethane, 2-pentanone, n-pentylamine, 1,3-dioxane, sec-butylacetate, 1,2-diaminoethane, 1-methoxy2-propanol, n-butylacetate, 1-ethoxy-2-propanol.

Selon un autre aspect, l'invention fournit une composition comprenant du 2-chloro-3,3,3-trifluoropropène (1233xf), 1,1,1,3,3-pentafluoropropane (245fa) et un agent d'extraction organique sélectionné parmi le groupe consistant en éthanedial, propanone, methylacetate, methylglyoxal, ethylacetate, butanone, propionitrile, dioxane, trimethoxymethane, 1,3-dioxane, 1,3,5-trioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, methylacetoacetate, n,n-dimethylpropanamide, dimethylmalonate, diethylsulfoxide, 2-(2-methoxyethoxy)ethanol, trimethylphosphate, diethylmalonate ; en particulier ledit agent d'extraction organique est sélectionné parmi le groupe consistant en propanone, methylacetate, ethylacetate, butanone, dioxane, trimethoxymethane, 1,3-dioxane, 1,3,5-trioxane, 1,2-diaminoethane, 1-methoxy-2-propanol.

Selon un autre aspect, l'invention fournit une composition azéotropique ou quasi-azéotropique comprenant 45 à 65 mole% de 2-chloro-3,3,3-trifluoropropène (1233xf) et de 35 à 55 mole% de 1,1,1,3,3-pentafluoropropane (245fa) sur base de la composition totale.

Aussi décrit est une composition comprenant 2-chloro-3,3,3-trifluoropropène (1233xf), 1,1,1,3,3-pentafluoropropane (245fa), E-1-chloro-3,3,3-trifluoro-1-propene (1233zdE) et un agent d'extraction organique sélectionné parmi le groupe consistant en propanone, methylacetate, ethylacetate, butanone, dioxane, trimethoxymethane, 1,3-dioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, n,n-dimethylpropanamide, diethylsulfoxide, 2-(2-methoxyethoxy)ethanol, trimethylphosphate, diethylmalonate ; de préférence un agent d'extraction organique sélectionné parmi le groupe consistant en propanone, methylacetate, ethylacetate, butanone, dioxane, trimethoxymethane, 1,3-dioxane, 1,2-diaminoethane, 1-methoxy2-propanol, 3-methoxy-1-butanol, diacetone alcohol.

Brève description des figures

Les figures 1a-d représentent schématiquement un dispositif mettant en oeuvre un procédé de purification du 2-chloro-3,3,3-trifluoropropène selon des modes de réalisation particuliers de la présente invention.
La figure 2 représente schématiquement un dispositif mettant en oeuvre un procédé de production du 2,3,3,3-tetrafluoropropene selon un mode de réalisation particulier de la présente invention.

## Description détaillée de l'invention

Le terme « hydrocarbure » tel qu'utilisé ici se réfère à des composés linéaires ou branchés d'alcane en $C_1$-$C_{20}$, cycloalcane en $C_3$-$C_{20}$, alcène en $C_5$-$C_{20}$, cycloalcène en $C_5$-$C_{20}$, arène en $C_6$-$C_{18}$. Par exemple, le terme alcane se réfère à des composés de formule $C_nH_{2n+2}$ dans lequel n est compris entre 1 et 20. Le terme alcane en $C_1$-$C_{20}$ englobe par exemple le pentane, hexane, heptane, octane, nonane, décane ou des isomères de ceux-ci. Le terme alcène en $C_5$-$C_{20}$ se réfère à des composés hydrocarbonés comprenant une ou plusieurs doubles liaisons carbone-carbone et comprenant de 5 à 20 atomes de carbone. Le terme cycloalcane en $C_3$-$C_{20}$ se réfère à un cycle hydrocarboné saturé comprenant de 3 à 20 atomes de carbone. Le terme aryle en $C_6$-$C_{18}$ se réfère à des composés hydrocarbonés cycliques et aromatiques comprenant de 6 à 18 atomes de carbone. Le terme cycloalcène en $C_5$-$C_{20}$ se réfère à des composés hydrocarbonés cycliques comprenant de 5 à 20 atomes de carbone et comprenant une ou plusieurs doubles liaisons carbone-carbone.

Le terme « alkyle » désigne un radical monovalent issu d'un alcane, linéaire ou branché, comprenant de 1 à 20 atomes de carbone. Le terme « cycloalkyle » désigne un radical monovalent issu d'un cycloalcane comprenant de 3 à 20 atomes de carbone. Le terme « aryle » désigne un radical monovalent issu d'un arène comprenant de 6 à 18 atomes de carbone. Le terme « alkényle » désigne un radical monovalent de 2 à 20 atomes de carbone et au moins une double liaison carbone-carbone. Le terme « alkynyle » désigne un radical monovalent de 2 à 20 atomes de carbone et au moins une triple liaison carbone-carbone. Le terme « halogène » se réfère à un groupement -F, -Cl, -Br ou -I. Le terme « cycloalkényle » se réfère à un radical monovalent issu d'un cycloalcène comprenant de 3 à 20 atomes de carbone. Les substituants alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ peuvent être substitués ou non par un ou plusieurs substituants -OH, halogène, $-P(O)(OR^a)_2$, $-NR^aC(O)R^b$, $-C(O)NR^aR^b$ -CN, $-NO_2$, $-NR^aR^b$, $-OR^a$, $-SR^a$, $-CO_2R^a$, $-OC(O)OR^a$, $-OC(O)R^a$, -C(O)H, $-C(O)R^a$, $-S(O)R^a$ dans lequel $R^a$ et $R^b$ sont indépendamment l'un de l'autre hydrogène, alkyle en $C_1$-$C_{20}$ non substitué, alkényle en $C_2$-$C_{20}$ non substitué, alkynyle en $C_2$-$C_{20}$ non substitué, cycloalkyle en $C_3$-$C_{20}$ non substitué, cycloalkényle en $C_3$-$C_{20}$ non substitué, aryle en $C_6$-$C_{18}$ non substitué. Dans les substituants $-NR^aR^b$, $R^a$ et $R^b$ peuvent former avec l'atome d'azote auquel ils sont rattachés un hétérocycle saturé ou insaturé, aromatique ou non, comprenant de 5 à 10 chainons.

Le terme « hydrohalocarbures » se réfère à des composés de formule $R^aX$ dans laquelle $R^a$ est sélectionné parmi alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ et X représente un atome de chlore, de fluor, de brome ou d'iode. Les substituants alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ peuvent être substitués

ou non par un ou plusieurs substituants -OH, halogène, -P(O)(OR$^a$)$_2$, -NR$^a$C(O)R$^b$, -C(O)NR$^a$R$^b$ -CN, -NO$_2$, - NR$^a$R$^b$, -OR$^a$, -SR$^a$, -CO$_2$R$^a$, -OC(O)OR$^a$, -OC(O)R$^a$, -C(O)H, -C(O)R$^a$, -S(O)R$^a$ dans lequel R$^a$ et R$^b$ sont tels que définis ci-dessus.

Le terme « alcool » se réfère à des hydrocarbures ou hydrohalocarbures tels que définis ci-dessus dans lequel au moins un atome d'hydrogène est remplacé par un groupement hydroxyle -OH.

Le terme « cétone » se réfère à des hydrocarbures comprenant au moins un ou plusieurs groupements fonctionnels carbonyle R$^C$-C(O)-R$^d$ dans lequel R$^c$ et R$^d$ sont indépendamment l'un de l'autre un alkyle en C$_1$-C$_{20}$, alkényle en C$_2$-C$_{20}$, alkynyle en C$_2$-C$_{20}$, cycloalkyle en C$_3$-C$_{20}$, cycloalkényle en C$_3$-C$_{20}$, aryle en C$_6$-C$_{18}$ et peuvent être substitués ou non par un ou plusieurs substituants -OH, halogène, -P(O)(OR$^a$)$_2$, -NR$^a$C(O)R$^b$, -C(O)NR$^a$R$^b$ -CN, -NO$_2$, -NR$^a$R$^b$, -OR$^a$, -SR$^a$, - CO$_2$R$^a$, -OC(O)OR$^a$, -OC(O)R$^a$, -C(O)H, -C(O)R$^a$, -S(O)R$^a$ dans lequel R$^a$ et R$^b$ sont, R$^c$ et R$^d$ pouvant être liés entre eux pour former avec le groupement carbonyle auquel ils sont rattachés une cétone cyclique comprenant de 4 à 10 chainons, de préférence de 4 à 7 chainons. La cétone cyclique peut également comprendre une ou plusieurs doubles liaisons carbone-carbone. La cétone cyclique peut également être substituée ou non par un ou plusieurs substituants tels que définis ci-dessus.

Le terme « amine » se réfère à des hydrocarbures comprenant au moins un ou plusieurs groupements fonctionnels amine -NR$^c$R$^d$ dans lequel R$^c$ et R$^d$ sont tels que définis ci-dessus, R$^c$ et R$^d$ pouvant être liés entre eux pour former avec l'atome d'azote auquel ils sont rattachés un hétérocycle aromatique ou non comprenant de 4 à 10 chainons.

Le terme « esters » se réfère à des composés de formule R$^c$-C(O)-O-R$^d$ dans lequel R$^c$ et R$^d$ sont tels que définis ci-dessus, R$^c$ et R$^d$ pouvant être liés entre eux pour former avec le groupement ester un cycle comprenant de 4 à 20 atomes de carbone.

Le terme « éther » se réfère à des composés de formule R$^c$-O-R$^d$ dans lequel R$^c$ et R$^d$ sont tels que définis ci-dessus, R$^c$ et R$^d$ pouvant être liés entre eux pour former avec l'atome d'oxygène auquel ils sont rattachés un hétérocycle comprenant de 4 à 20 atomes de carbone.

Le terme « aldéhyde » se réfère à des composés comprenant au moins un ou plusieurs groupements fonctionnels -C(O)-H.

Le terme « nitrile » se réfère à des composés comprenant au moins un ou plusieurs groupements fonctionnels -CN.

Le terme « carbonate » se réfère à des composés de formule R$^c$-O-C(O)-O-R$^d$ dans lequel R$^c$ et R$^d$ sont tels que définis ci-dessus.

Le terme « sulfoxide » concerne des composés de formule R$^c$S(O)R$^d$ dans laquelle R$^c$ et R$^d$ sont tels que définis ci-dessus.

Le terme « sulfate » concerne des composés de formule R$^c$S(O)$_2$R$^d$.

Le terme « thioalkyle » concerne des composés de formule R$^c$SR$^d$ dans laquelle R$^c$ et R$^d$ sont tels que définis ci-dessus.

Le terme « amide » concerne des composés de formule R$^c$C(O)NR$^e$R$^d$ dans laquelle R$^c$ et R$^d$ sont tels que définis ci-dessus, R$^e$ étant défini par les mêmes substituants que R$^c$, R$^c$ et R$^d$ pouvant être liés entre eux pour former avec le groupement amide -C(O)N- auquel ils sont rattachés une amide cyclique comprenant de 4 à 10 chainons, de préférence de 4 à 7 chainons. L'amide cyclique peut également comprendre une ou plusieurs doubles liaisons carbone-carbone. L'amide cyclique peut également être substituée ou non par un ou plusieurs substituants tels que définis ci-dessus.

Le terme « hétérocycle » désigne un cycle carboné comprenant de 4 à 10 chainons dont au moins un des chainons est un hétéroatome sélectionné parmi le groupe consistant en O, S, P et N. L'hétérocycle peut comprendre un ou plusieurs double liaison carbone-carbone ou une ou plusieurs double liaison carbone-hétéroatome ou une ou plusieurs double liaison hétéroatomehétéroatome. De préférence, l'hétérocycle peut comprendre 1, 2, 3, 4 ou 5 hétéroatomes tel que défini ci-dessus. En particulier, l'hétérocycle peut comprendre 1, 2 ou 3 hétéroatomes sélectionné parmi l'oxygène, l'azote ou le soufre. De préférence, l'hétérocycle peut être un cycle carboné comprenant de 4 à 6 chaînons dont 1, 2 ou 3 chaînons sont des hétéroatomes sélectionnés par O ou N ; de préférence O. L'hétérocycle peut être ou non substitué par un ou plusieurs substituants choisi parmi -OH, halogène, -P(O)(OR$^a$)$_2$, -NR$^a$C(O)R$^b$, -C(O)NR$^a$R$^b$ -CN, - NO$_2$, -NR$^a$R$^b$, -OR$^a$, -SR$^a$, -CO$_2$R$^a$, -OC(O)OR$^a$, -OC(O)R$^a$, -C(O)H, -C(O)R$^a$, -S(O)R$^a$ dans lequel R$^a$ et R$^b$ sont tels que définis ci-dessus.

Le terme « phosphate » se réfère à un composé de formule (R$^c$O)$_3$P(O) dans laquelle R$^c$ est tel que défini ci-dessus.

Le terme « composition azéotropique » désigne un mélange liquide de deux ou plusieurs composés se comportant comme une substance unique, et qui bout à température fixe en gardant une composition en phase liquide identique à celle de la phase gaz. Le terme « composition quasi-azéotropique » désigne un mélange liquide de deux ou plusieurs composés ayant un point d'ébullition constant ou qui a tendance à ne pas se fractionner lorsqu'il est soumis à l'ébullition ou l'évaporation.

Selon un premier aspect, l'invention se rapporte à un procédé de purification du 2-chloro-3,3,3-trifluoropropene (1233xf). Le procédé de purification est effectué à partir d'une première composition comprenant du 2-chloro-3,3,3-trifluoropropène et au moins un des composés choisi parmi le groupe consistant en E-1-chloro-3,3,3-trifluoro-1-propene (1233zdE) et 1,1,1,3,3-pentafluoropropane (245fa).

De préférence, ledit procédé comprend les étapes de :

a) mise en contact de ladite première composition avec au moins un agent d'extraction organique pour former une seconde composition ;

b) distillation extractive de ladite seconde composition pour former :

i) une troisième composition comprenant ledit agent d'extraction organique et ledit au moins un des composés choisi parmi le groupe consistant en E-1-chloro-3,3,3-trifluoro-1-propene (1233zdE) et 1,1,1,3,3-pentafluoropropane (245fa),

ii) un courant comprenant le 2-chloro-3,3,3-trifluoropropène;

c) récupération et séparation de ladite troisième composition pour former un courant comprenant ledit agent d'extraction organique et un courant comprenant ledit au moins un des composés choisi parmi le groupe consistant en E-1-chloro-3,3,3-trifluoro-1-propene (1233zdE) et 1,1,1,3,3-pentafluoropropane (245fa).

Alternativement, l'étape c) peut être optionnelle.

De préférence, le courant comprenant ledit agent organique est recyclé à l'étape a).

De préférence, le courant comprenant ledit au moins un des composés choisi parmi le groupe consistant en E-1-chloro-3,3,3-trifluoro-1-propene (1233zdE) et 1,1,1,3,3-pentafluoropropane (245fa) est incinéré ou récupéré pour extraire et purifier un des constituants de celui-ci.

Ladite première composition peut comprendre du 2-chloro-3,3,3-trifluoropropène et au moins un, au moins deux ou l'ensemble des composés choisi parmi le groupe consistant en E-1-chloro-3,3,3-trifluoro-1-propene (1233zdE) et 1,1,1,3,3-pentafluoropropane (245fa).

Ladite première composition peut comprendre entre 50 et 99,99 % en poids de 2-chloro-3,3,3-trifluoropropène sur base du poids total de la première composition, avantageusement entre 55 et 99,9% en poids, de préférence entre 55 et 95% et en particulier entre 60 et 90% en poids de 2-chloro-3,3,3-trifluoropropène sur base du poids total de la première composition.

Lorsqu'elle en contient, ladite première composition peut comprendre entre 0,01 et 50 % en poids de E-1-chloro-3,3,3-trifluoro-1-propene (1233zdE) sur base du poids total de la première composition, avantageusement entre 0,01 et 40 % en poids, de préférence entre 0,01 et 30 % et en particulier entre 0,01 et 20 % en poids de E-1-chloro-3,3,3-trifluoro-1-propene (1233zdE) sur base du poids total de la première composition.

Lorsqu'elle en contient, ladite première composition peut comprendre entre 0,01et 50 % en poids de 1,1,1,3,3-pentafluoropropane (245fa) sur base du poids total de la première composition, avantageusement entre 0,01 et 40 % en poids, de préférence entre 0,01 et 30% et en particulier entre 0,01 et 20% en poids de 1,1,1,3,3-pentafluoropropane (245fa) sur base du poids total de la première composition.

Lorsqu'elle en contient, ladite première composition peut comprendre entre 0,01 et 15 % en poids de 1,1,1,3,3,3-hexafluoropropane (236fa) sur base du poids total de la première composition, avantageusement entre 0,01 et 10 % en poids, de préférence entre 0,01 et 7% et en particulier entre 0,01 et 5 % en poids de 1,1,1,3,3,3-hexafluoropropane (236fa) sur base du poids total de la première composition.

Selon un mode de réalisation particulier, ledit agent d'extraction organique est un solvant choisi parmi le groupe consistant en hydrocarbure, hydrohalocarbure, alcool, cétone, amine, ester, éther, aldéhyde, nitrile, carbonate, sulfoxide, sulfate, thioalkyle, amide, hétérocycle et phosphate. Ledit agent d'extraction organique peut aussi être l'acide perfluorobutanoïque. De préférence, ledit agent d'extraction organique est un solvant choisi parmi le groupe consistant en alcool, cétone, amine, ester, éther, aldéhyde, carbonate, sulfoxide, amide, hétérocycle et phosphate.

Ledit agent d'extraction organique se réfère à un composé comportant au moins un atome de carbone.

De préférence, les hydrocarbures sont sélectionnés parmi le groupe consistant en cyclohexene, 1,3,5-triethylbenzene, 2,4,4-trimethyl-1-pentene, 1-methylcyclohexene, 1,4-dimethylbenzene, styrene, 1,3,5-trimethylbenzene, 1,2,4,5-tetramethylbenzene, 1,3-diethenylbenzene.

De préférence, les hydrohalocarbures sont sélectionnés parmi le groupe consistant en 2-chloro-2-methylpropane, 1-chloro-2,2-difluoropropane, 1,1-dichloroethane 2-bromopropane, 2-chlorobutane, 1-bromopropane, 2-bromo-2-methylpropane, 1-chloro-3-fluoropropane, 1-chlorobutane, 2-chloro-2-methylbutane, 1,2-dichloroethane, 1,1-dichloropropane, 1,2-dichloro-2-fluoropropane, 2-bromobutane, 1-fluorohexane,2,3-dichloro-1-propene, 1,2-dichloropropane, 3-chloropentane, trichloroacetaldehyde, isoamylchloride, 1-chloro-4-fluorobutane, 1-bromo-3-fluoropropane, 1-bromobutane, 2,2-dichlorobutane, cis-1,3-dichloropropene, 2-bromo-2-methylbutane, trans-1,3-dichloropropene, 1,1,1-trichloro-3-fluoropropane, 1-chloro-3,3-dimethylbutane, 2-bromopentane, 2,3-dichlorobutane, 1-bromo-3-methylbutane, 1,3-dichloro-trans-2-butene, 1,3-dichloropropane, 1,2,2-trichloropropane, 1,2-dichlorobutane, 2,3-dichloro-2-methylbutane, 1-bromopentane, 1,2-dichloro-2-butene, 1,3-dichlorobutane, 1-chloro-3-bromopropane, 1,2-dichloropentane, 1-bromohexane, 1,2,3-trichloropropane, 1,4-dichloro-trans-2-butene, 1,4-dichlorobutane, 2-chloropyridine, bromocyclohexane, 1,3-dibromopropane, 1,4-dichlorobenzene, 1-chlorooctane, 1-fluorodecane, 2-chloro-1,4-dimethylbenzene, 1,4-dibromobutane.

De préférence, les alcools sont sélectionnés parmi le groupe consistant en méthanol, 1,1,1-trifluoro-2-propanol, ethanol, 2-propanol, tert-butanol, 2,2-difluoroethanol, propanol, 2-allyloxyethanol, 2-butanol, 2,2,3,3-tetraflouro-1-propanol, 1-methoxy2-propanol, 1-(dimethylamino)-2-propanol, 4,4,4-trifluorobutanol, 3-fluoropropanol, 2-chloroethanol, 2-methoxy1-propanol, 1-ethoxy-2-propanol, 2-chloro-1-propanol, 2-(dimethylamino)-ethanol, 2-ethoxy-1-propanol, 2-propoxyethanol, 1-propoxy-2-propanol, 2-isopropoxyethanol, 2-(methylamino)-ethanol, 3-ethoxy-1-propanol, 3-methoxy-1-butanol, 2-(diethylamino)-ethanol, 2-(ethylamino)ethanol, 1-butoxy-2-propanol, 2-furanmethanol, 2-butoxyethanol, 2-aminopropanol, 2-amino-1-butanol, 2-methyl-2-nitro-1-propanol, 2-(ethylthio)-ethanol, propyleneglycol, 2-(2-methoxyethoxy)ethanol, 1,2-butanediol, glycol, 2-methyl-2,4-pentanediol.

De préférence, les cétones sont sélectionnées parmi le groupe consistant en propanone, butanone, 3-pentanone, 2-pentanone, 3,3-dimethyl-2-butanone, 4-methyl-2-pentanone, 2-hexanone, 5-hexen-2-one, 4-methyl-2-hexanone, 2-heptanone, cyclohexanone, 4-methoxy-4-methyl-pentan-2-one, 2,3-heptanedione, 3-octanone, diacetone alcohol, 2-octanone, cycloheptanone, dihydroxyacetone, 2-propylcyclohexanone.

De préférence, les amines sont sélectionnées parmi le groupe consistant en 2-methoxyethanamine, n-methylhydroxylamine, 2-ethoxyethanamine, n-methyl-1,2-ethanediamine, 1,2-diaminoethane, 1,2-propanediamine, 1,3-propanediamine, dimethylethanolamine, 1,4, butanediamine, ethanolamine, diethylpropanolamine.

De préférence, les esters sont sélectionnés parmi le groupe consistant en méthylacétate, isopropylformate, ethylacetate, n-propylformate, isopropylacetate, tert-butylacetate, ethylpropionate, sec-butylacetate, diethylcarbonate, n-butylacetate, methylhexanoate,, isopropylchloroacetate, 2-ethoxyethanolacetate, methylacetoacetate, methylheptanoate, 2-propanol-1-methoxy-propanoate, dimethylmalonate, dihexylphthalate, dimethylsulfate, 2-butoxyethanolacetate, trimethylphosphate, methylbenzoate, diethylmalonate.

De préférence, les éthers sont sélectionnés parmi le groupe consistant en chloromethoxymethane, 1,2-dimethoxyethane, trimethoxymethane, 2-methoxyethanol, 2-chloro-1,1-dimethoxyethane, ethoxyethanol, 1-methoxy-2-acetoxypropane, 1,1,3,3-tetramethoxypropane, diethyleneglycolmonoethylether.

De préférence, les aldéhydes sont sélectionnés parmi le groupe consistant en éthanedial, isobutanal, methylglyoxal, 2-methylbutanal, hexanal, heptanal, 3-hydroxy-butanal, furfural.

De préférence, les nitriles sont sélectionnés parmi le groupe consistant en acétonitrile, propionitrile, butyronitrile, valeronitrile, (methyleneamino)acetonitrile, 3-methoxypropanenitrile.

De préférence, le carbonate peut être le diéthylcarbonate.

De préférence, les sulfoxides sont sélectionnés parmi le groupe consistant en diméthylsulfoxide et diéthylsulfoxide.

De préférence, le sulfate peut être diméthylsulfate.

De préférence, les amides incluent le diméthylformamide, 2,2,2-trifluoro-acétamide,n,n-dimethylpropanamide.

De préférence, le thioalkyle peut être le 3-mercapto-1,2-propanediol.

De préférence, les hétérocycles sont choisis parmi le groupe consistant en tétrahydrofurane, dioxane, 1,3-dioxane, 1,3,5-trioxane, 3-furfural.

De préférence, le phosphate peut être par exemple trimethylphosphate.

L'agent d'extraction organique à utiliser peut être sélectionné en fonction des composés présents dans ladite première composition. Ainsi, l'agent d'extraction organique peut être sélectionné en fonction du facteur de séparation et de la capacité d'absorption établi pour une composition particulière. Outre ces deux critères, le choix de l'agent d'extraction organique peut se baser optionnellement sur d'autres critères commerciaux ou environnementaux, tels que par exemple le coût de l'agent d'extraction organique, sa disponibilité sur le marché, ses propriétés toxiques ou inflammable. De plus, selon un mode de réalisation particulier, afin d'optimiser le fonctionnement des colonnes de distillation utilisées à l'étape b) et c) du présent procédé de purification du 2-chloro-3,3,3-trifluoropropene, le point d'ébullition de l'agent d'extraction organique peut être de 50°C à 200°C, avantageusement de 50°C à 190°C, de préférence de 50°C à 180°C, en particulier 50°C à 160°C, et de manière privilégiée de 50°C à 150°C, de manière plus privilégiée de 75°C à 150°C.

Également décrit est un agent d'extraction organique qui a un facteur de séparation $S_{1,2}$ supérieur ou égal à 1,1, ledit facteur de séparation étant calculé par la formule $S_{1,2} = (\gamma_{1,S}*P1)/(\gamma_{2,S}*P2)$ dans laquelle

$\gamma_{1,S}$ représente le coefficient d'activité du 2-chloro-3,3,3-trifluoropropene dans ledit agent d'extraction organique à dilution infinie,
P1 représente la pression de vapeur saturante du 2-chloro-3,3,3-trifluoropropene,

$\gamma_{2,S}$ représente le coefficient d'activité dudit au moins un des composés choisi parmi le groupe consistant en E-1-chloro-3,3,3-trifluoro-1-propene (1233zdE), 1,1,1,3,3-pentafluoropropane (245fa) dans ledit agent d'extraction organique à dilution infinie, P2 représente la pression de vapeur saturante de dudit au moins un des composés choisi parmi le groupe consistant en E-1-chloro-3,3,3-trifluoro-1-propene (1233zdE), 1,1,1,3,3-pentafluoropropane (245fa) et 1,1,1,3,3,3-hexafluoropropane (236fa).

Dans la présente demande, la pression de vapeur saturante est considérée pour une température de 25°C. Avantageusement, le facteur de séparation $S_{1,2}$ est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

Ledit agent d'extraction organique peut avoir une capacité d'absorption $C_{2,S}$ supérieure ou égale à 0,20, ladite capacité d'absorption étant calculé par la formule $C_{2,S} = 1/(\gamma_{2,S})$ dans laquelle $\gamma_{2,S}$ représente le coefficient d'activité dudit au moins un des composés choisi parmi le groupe consistant en 1-chloro-3,3,3-trifluoro-1-propene (1233zd), 1,1,1,3,3-pentafluoropropane (245fa) et 1,1,1,3,3,3-hexafluoropropane (236fa) dans ledit agent d'extraction organique à dilution infinie. Avantageusement, la capacité d'absorption $C_{2,S}$ est supérieure ou égale à 0,40, de préférence supérieure ou égale à 0,60, plus préférentiellement supérieure ou égale à 0,80, en particulier supérieure ou égale à 1,0, plus particulièrement supérieure ou égale à 1,4, de manière privilégiée supérieure ou égale à 1,6. Ainsi, ledit agent d'extraction organique peut avoir un facteur de séparation $S_{1,2}$ supérieur ou égal à 1,1, avantageusement supérieur ou égal à 1,2, de préférence supérieur ou égal à 1,4, plus préférentiellement supérieur ou égal à 1,6, en particulier supérieur ou égal à 1,8 et plus particulièrement supérieur ou égal à 2,0 ; et une capacité d'absorption $C_{2,S}$ supérieure ou égale à 0,20, avantageusement supérieure ou égale à 0,40, de préférence supérieure ou égale à 0,60, plus préférentiellement supérieure ou égale à 0,8, en particulier supérieure ou égale à 1,0, plus particulièrement supérieure ou égale à 1,4, de manière privilégiée supérieure ou égale à 1,6.

De préférence, ledit agent d'extraction organique peut être sélectionné parmi le groupe consistant en propanone, methylacetate, ethylacetate, butanone, dioxane, trimethoxymethane, 1,3-dioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, n,n-dimethylpropanamide, diethylsulfoxide, 2-(2-methoxyethoxy)ethanol, trimethylphosphate, diethylmalonate. En particulier, ledit agent d'extraction organique peut être sélectionné parmi le groupe consistant en propanone, methylacetate, ethylacetate, butanone, dioxane, trimethoxymethane, 1,3-dioxane, 1,2-diaminoethane, 1-methoxy2-propanol, 3-methoxy-1-butanol, diacetone alcohol.

Également décrit est une première composition comprenant 2-chloro-3,3,3-trifluoropropene (1233xf) et au moins un des composés choisi parmi le groupe consistant en E-1-chloro-3,3,3-trifluoro-1-propene (1233zdE), 1,1,1,3,3-pentafluoropropane (245fa) et 1,1,1,3,3,3-hexafluoropropane (236fa). Ladite première composition peut être une composition comprenant 2-chloro-3,3,3-trifluoropropene (1233xf) et au moins deux ou l'ensemble des composés choisi parmi le groupe consistant en E-1-chloro-3,3,3-trifluoro-1-propene (1233zdE), 1,1,1,3,3-pentafluoropropane (245fa) et 1,1,1,3,3,3-hexafluoropropane (236fa). La teneur en chacun des composés dans la première composition azéotropique ou quasi-azéotropique est telle qu'exprimée ci-dessus.

En fonction du ou des composés à éliminer dans ladite première composition, ledit facteur de séparation et ladite capacité d'absorption pourront être calculés pour un couple binaire particulier consistant en 2-chloro-3,3,3-trifluoropropene (1233xf) et un des composés choisi parmi le groupe consistant en E-1-chloro-3,3,3-trifluoro-1-propene (1233zdE) et 1,1,1,3,3-pentafluoropropane (245fa). Ainsi, pour sélectionner ledit agent d'extraction organique apte à être utilisé dans l'étape b) de distillation extractive, le facteur de séparation et la capacité d'absorption peuvent être calculés par exemple pour un couple binaire 2-chloro-3,3,3-trifluoropropene (1233xf) et 1,1,1,3,3-pentafluoropropane (245fa) si ladite première composition comprend ces deux composés ou pour un couple binaire 2-chloro-3,3,3-trifluoropropene (1233xf) et E-1-chloro-3,3,3-trifluoro-1-propene (1233zdE) si ladite première composition comprend ces deux composés. Le facteur de séparation $S_{1,2}$ permet de déterminer la capacité d'un agent d'extraction organique à séparer deux ou plusieurs composés. La capacité d'absorption $C_{2,S}$ permet de déterminer la quantité de solvant à utiliser pour obtenir la séparation entre les composés considérés. Pour l'ensemble des premières compositions détaillées ci-dessous, ledit agent d'extraction organique peut avoir un facteur de séparation $S_{1,2}$ supérieur ou égal à 1,1, avantageusement supérieur ou égal à 1,2, de préférence supérieur ou égal à 1,4, plus préférentiellement supérieur ou égal à 1,6, en particulier supérieur ou égal à 1,8 et plus particulièrement supérieur ou égal à 2,0 ; et/ou ledit agent d'extraction organique peut avoir une capacité d'absorption $C_{2,S}$ supérieure ou égale à 0,25, avantageusement supérieure ou égale à 0,5, de préférence supérieure ou égale à 0,75, plus préférentiellement supérieure ou égale à 1,0, plus particulièrement supérieure ou égale à 1,4, de manière privilégiée supérieure ou égale à 1,6.

Selon un mode de réalisation préféré, ladite première composition peut être une composition comprenant 2-chloro-3,3,3-trifluoropropene (1233xf) et 1,1,1,3,3-pentafluoropropane (245fa). La teneur en chacun des composés dans cette composition particulière est telle qu'exprimée ci-dessus en référence aux teneurs individuelles de chacun des composés. Ainsi, la seconde composition peut comprendre 2-chloro-3,3,3-trifluoropropene (1233xf) et 1,1,1,3,3-pentafluoropropane (245fa) et ledit agent d'extraction organique. Pour séparer la première composition comprenant 2-chloro-3,3,3-trifluoropropene (1233xf) et 1,1,1,3,3-pentafluoropropane (245fa), un agent d'extraction organique ayant un facteur de séparation $S_{1,2}$ supérieur ou égal à 1,1 et une capacité d'absorption $C_{2,S}$ supérieure ou égale à 0,2 peut être utilisé ; ledit agent d'extraction organique peut être sélectionné parmi le groupe consistant en éthanedial, 2-chloro-1,1,1,3-tetrafluoropropane, 1,1,1,2,2,3,4,5,5,5-decafluoropentane, 1-chloro-2,2-difluoropropane, propanone, methylacetate, ethyl1,1,2,2-tetrafluoroethylether, chloromethoxymethane, isobutanal, methanol, tetrahydrofurane, isopropylformate, methylglyoxal, 2,2,2-trifluoroethanol, 1,1,1-trifluoro-2-propanol, ethylacetate, ethanol, butanone, n-propylformate, 2-propanol, acetonitrile, pentafluoro-1-propanol, tert-butanol, 1,2-dimethoxyethane, isopropylacetate, 2-methylbutanal, 2-methoxyethanamine, 2,2-difluoroethanol, propanol, tert-butylacetate, propionitrile, 2-allyloxyethanol, ethylpropionate, 2-butanol, dioxane, 3-pentanone, 2-pentanone, trimethoxymethane, n-methylhydroxylamine, 3,3-dimethyl-2-butanone, 1,3-dioxane, 2,2,3,3-tetraflouro-1-propanol, 2-ethoxyethanamine, sec-butylacetate, n-methyl-1,2-ethanediamine, 1,3,5-trioxane, 4-methyl-2-pentanone, 1,2-diaminoethane, butyronitrile, 1-methoxy2-propanol, 1,2-propanediamine, perfluorobutanoicacid, 1-(dimethylamino)-2-propanol, 2-methoxyethanol, 4,4,4-trifluorobutanol, diethylcarbonate, n-butylacetate, 2-chloro-1,1-dimethoxyethane, 2-hexanone, 3-fluoropropanol, 5-hexen-2-one, 2-methoxy1-propanol, hexanal, 1-ethoxy-2-propanol, 2-chloro-1-propanol, 2-(dimethylamino)-ethanol, ethoxyethanol, 2-ethoxy-1-propanol, 1,3-propanediamine, 3,3,4,4,5,5,6,6-octafluoro-1-pentanol, valeronitrile, (methyleneamino)acetonitrile, 3-furfural, 4-methyl-2-hexanone, 1-methoxy-2-acetoxypropane, methylhexanoate, 2-propoxyethanol, 1-propoxy-2-propanol, dimethylethanolamine, isopropylchloroacetate, 2-heptanone, heptanal, dimethylformamide, 2-isopropoxyethanol, cyclohexanone, 2-ethoxyethanolacetate, 3-hydroxy-butanal, 2-(methylamino)-ethanol, 1,4-butanediamine, 4-methoxy-4-methyl-pentan-2-one, 3-ethoxy-1-propanol, 3-methoxy-1-butanol, furfural, diglyme, 2,2,2-trifluoro-acetamide, 2-(diethylamino)-ethanol, 3-methoxypropanenitrile, 2,3-heptanedione, 2-(ethylamino)ethanol, 3-octanone, diacetone alcohol, 1-butoxy-2-propanol, 2-furanmethanol, 2-butoxyethanol, ethanolamine, methylacetoacetate, 2-octanone, 2-aminopropanol, methylheptanoate, n,n-dimethylpropanamide, 2-amino-1-butanol, 2-methyl-2-nitro-1-propanol, 2-propanol-1-methoxy-propanoate, cycloheptanone, dihydroxyacetone, dimethylmalonate, 1,1,3,3-tetramethoxypropane, 2-(ethylthio)-ethanol, dihexylphthalate, propyleneglycol, dimethylsulfate, dimethylsulfoxide, diethylpropanolamine, 2-butoxyethanolacetate, diethylsulfoxide, 2-(2-methoxyethoxy)ethanol, 1,2-butanediol, diethyleneglycolmonoethylether, trimethylphosphate, 2-methyl-2,4-pentanediol, methylbenzoate, diethylmalonate.

Avantageusement, ledit agent d'extraction organique peut avoir un facteur de séparation $S_{1,2}$ supérieur ou égal à 1,2 et une capacité d'absorption $C_{2,S}$ supérieure ou égale à 0,4 ; et être sélectionné parmi le groupe consistant en éthanedial, propanone, methylacetate, chloromethoxymethane, isobutanal, methanol, isopropylformate, methylglyoxal, 2,2,2-trifluoroethanol, 1,1,1-trifluoro-2-propanol, ethylacetate, ethanol, butanone, n-propylformate, 2-propanol, acetonitrile, pentafluoro-1-propanol, tert-butanol, 1,2-dimethoxyethane, isopropylacetate, 2-methylbutanal, 2-methoxyethanamine, tert-butylacetate, propionitrile, 2-allyloxyethanol, ethylpropionate, dioxane, 3-pentanone, 2-pentanone, trimethoxymethane, 3,3-dimethyl-2-butanone, 1,3-dioxane, 2,2,3,3-tetraflouro-1-propanol, sec-butylacetate, n-methyl-1,2-ethanediamine, 1,3,5-trioxane, 4-methyl-2-pentanone, 1,2-diaminoethane, butyronitrile, 1-methoxy2-propanol, 1,2-propanediamine, perfluorobutanoicacid, 1-(dimethylamino)-2-propanol, 2-methoxyethanol, 4,4,4-trifluorobutanol, diethylcarbonate, n-butylacetate, 2-hexanone, 3-fluoropropanol, 5-hexen-2-one, 2-methoxy1-propanol, 1-ethoxy-2-propanol, 2-(dimethylamino)-ethanol, ethoxyethanol, 2-ethoxy-1-propanol, 1,3-propanediamine, 3,3,4,4,5,5,6,6-octafluoro-1-pentanol, valeronitrile, (methyleneamino)acetonitrile, 3-furfural, 4-methyl-2-hexanone, 1-methoxy-2-acetoxypropane, methylhexanoate, 2-propoxyethanol, 1-propoxy-2-propanol, dimethylethanolamine, 2-heptanone, dimethylformamide, 2-isopropoxyethanol, cyclohexanone, 2-ethoxyethanolacetate, 3-hydroxy-butanal, 2-(methylamino)-ethanol, 4-methoxy-4-methyl-pentan-2-one, 3-ethoxy-1-propanol, 3-methoxy-1-butanol, furfural, diglyme, 3-methoxypropanenitrile, 2-(ethylamino)ethanol, diacetone alcohol, 1-butoxy-2-propanol, 2-furanmethanol, methylacetoacetate, 2-aminopropanol, n,n-dimethylpropanamide, 2-amino-1-butanol, 2-methyl-2-nitro-1-propanol, 2-propanol-1-methoxy-propanoate, cycloheptanone, dimethylmalonate, 1,1,3,3-tetramethoxypropane, dimethylsulfate, dimethylsulfoxide, 2-butoxyethanolacetate, diethylsulfoxide, 2-(2-methoxyethoxy)ethanol, 1,2-butanediol, diethyleneglycolmonoethylether, trimethylphosphate, 2-methyl-2,4-pentanediol, methylbenzoate, diethylmalonate.

De préférence, ledit agent d'extraction organique peut avoir un facteur de séparation $S_{1,2}$ supérieur ou égal à 1,4 et une capacité d'absorption $C_{2,S}$ supérieure ou égale à 0,6 ; et être sélectionné parmi le groupe consistant en éthanedial, propanone, methylacetate, isobutanal, isopropylformate, methylglyoxal, ethylacetate, butanone, n-propylformate, acetonitrile, 1,2-dimethoxyethane, isopropylacetate, 2-methoxyethanamine, propionitrile, 2-allyloxyethanol, ethylpropionate, dioxane, 3-pentanone, 2-pentanone, trimethoxymethane, 3,3-dimethyl-2-butanone, 1,3-dioxane, sec-butylacetate, 1,3,5-trioxane, 1,2-diaminoethane, butyronitrile, 1-methoxy2-propanol, 1,2-propanediamine, 1-(dimethylamino)-2-propanol, 2-methoxyethanol, diethylcarbonate, 5-hexen-2-one, 2-methoxy1-propanol, 1-ethoxy-2-propanol, 2-(dimethylamino)-ethanol, ethoxyethanol, 2-ethoxy-1-propanol, 1,3-propanediamine, 3,3,4,4,5,5,6,6-octafluoro-1-pentanol, (methyleneamino)acetonitrile, 3-furfural, 1-methoxy-2-acetoxypropane, dimethylethanolamine, dimethylformamide, 2-isopropoxyethanol, cyclohexanone, 2-ethoxyethanolacetate, 3-hydroxy-butanal, 2-(methylamino)-ethanol, 3-ethoxy-1-propanol, 3-methoxy-1-butanol, furfural, 3-methoxypropanenitrile, diacetone alcohol, methylacetoacetate, 2-aminopropanol, n,n-dimethylpropanamide, 2-propanol-1-methoxy-propanoate, dimethylmalonate, dimethylsulfoxide, diethylsulfoxide, 2-(2-methoxyethoxy)ethanol, diethyleneglycolmonoethylether, trimethylphosphate, 2-methyl-2,4-pentanediol, diethylmalonate.

En particulier, ledit agent d'extraction organique peut avoir un facteur de séparation $S_{1,2}$ supérieur ou égal à 1,6 et une capacité d'absorption $C_{2,S}$ supérieure ou égale à 0,8 ; et être sélectionné parmi le groupe consistant en éthanedial, propanone, methylacetate, methylglyoxal, ethylacetate, butanone, propionitrile, dioxane, trimethoxymethane, 1,3-dioxane, 1,3,5-trioxane, 1,2-diaminoethane, 1-methoxy2-propanol, 2-methoxyethanol, diethylcarbonate, 2-methoxy1-propanol, 3-furfural, 1-methoxy-2-acetoxypropane, dimethylformamide, 3-methoxy-1-butanol, furfural, 3-methoxypropanenitrile, diacetone alcohol, methylacetoacetate, n,n-dimethylpropanamide, dimethylmalonate, dimethylsulfoxide, diethylsulfoxide, 2-(2-methoxyethoxy)ethanol, trimethylphosphate, diethylmalonate.

Plus particulièrement, ledit agent d'extraction organique peut avoir un facteur de séparation $S_{1,2}$ supérieur ou égal à 1,6 et une capacité d'absorption $C_{2,S}$ supérieure ou égale à 1,0 ; et être sélectionné parmi le groupe consistant en éthanedial, propanone, methylacetate, methylglyoxal, ethylacetate, butanone, propionitrile, dioxane, trimethoxymethane, 1,3-dioxane, 1,3,5-trioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, methylacetoacetate, n,n-dimethylpropanamide, dimethylmalonate, diethylsulfoxide, 2-(2-methoxyethoxy)ethanol, trimethylphosphate, diethylmalonate.

De manière privilégiée, ledit agent d'extraction organique est sélectionné parmi le groupe consistant en propanone, methylacétate, ethylacétate, butanone, dioxane, triméthoxymethane, 1,3-dioxane, 1,3,5-trioxane, 1,2-diaminoethane, 1-methoxy-2-propanol.

Selon un mode de réalisation préféré, ladite première composition peut être une composition comprenant 2-chloro-3,3,3-trifluoropropene (1233xf) et E-1-chloro-3,3,3-trifluoro-1-propene (1233zdE). La teneur en chacun des composés dans cette composition particulière est telle qu'exprimée ci-dessus en référence aux teneurs individuelles de chacun des composés. Ainsi, la seconde composition peut comprendre 2-chloro-3,3,3-trifluoropropene (1233xf) et E-1-chloro-3,3,3-trifluoro-1-propene (1233zdE) et ledit agent d'extraction organique. Pour séparer la première composition comprenant 2-chloro-3,3,3-trifluoropropene (1233xf) et E-1-chloro-3,3,3-trifluoro-1-propene (1233zdE), un agent d'extraction organique ayant un facteur de séparation $S_{1,2}$ supérieur ou égal à 1,1 et une capacité d'absorption $C_{2,S}$ supérieure ou égale à 0,2 peut être utilisé ; ledit agent d'extraction organique peut être sélectionné parmi le groupe consistant en isopropylmethylamine, ethanedial, 1,2-dichloro-1,1,3,3,3-pentafluoropropane, 1-chloro-1,2,2,3-tetrafluoropropane, perfluorocyclohexane, 1,1-dichloro-1,2,2,3,3-pentafluoropropane, 2-chloro-2-methylpropane, 3-chloro-1,1,1,3-tetrafluoropropane, 1,1-dichloro-2,2,3,3,3-pentafluoropropane, 2-chloro-1,1,1,3-tetrafluoropropane, 2-propanethiol, 1,3-dichloro-1,1,2,3,3-pentafluoropropane, 1,2-dichloro-3,3,3-trifluoropropene, 1,3-dichloro-1,1,2,2,3-pentafluoropropane, 2-ethoxypropane, 2,2-dichloro-1,1,1,3-tetrafluoropropane, 1-chloro-2,2-difluoropropane, methyl-t-butylether, diethylamine, propanone, perfluoro-n-hexane, methylacetate, 1,1-dichloroethane, ethyl1,1,2,2-tetrafluoroethylether, 4-methoxy-2-methyl-2-butanethiol, 2-bromopropane, chloromethoxymethane, 1,2-dichloro-1,2,3,3,4,4-hexafluorocyclobutane, 1,1-dichloro-2,2-difluoroethane, 2,2-dichloro-1,1,3,3-tetrafluoropropane, trichloromethane, difluorodiethylsilane, 2-butanamine, 2,3-dichloro-1,1,1,2-tetrafluoropropane, n-methylpropylamine, 3-methylpentane, 1,1-dichloro-1,2,2,3-tetrafluoropropane, tert-butylthiol, isobutanal, methanol, tetrahydrofurane, 1,3-dichloro-1,1,2,2-tetrafluoropropane, 1,1,1-trichloro-2,2-difluoroethane, 1,2-dichloro-1,2,3,3-tetrafluoropropane, 1-propanethiol, chlorobromomethane, 2-chlorobutane, isopropylformate, diisopropylether, 1,3-dichloro-1,1,3,3-tetrafluoropropane, hexane, 1,3-dichloro-1,2,2,3-tetrafluoropropane, 1,2-dichloro-1,1,2,3-tetrafluoropropane, 3-bromopropene, 2,3-dichloro-1,1,1,3-tetrafluoropropane, 1,1-dichloro-2-fluoroethane, 1-bromopropane, 1,1-difluoro-1,2,2-trichloroethane, methylglyoxal, 1,1,2-trichloro-1,2-difluoroethane, iodoethane, 2-ethoxy-2-methyl-propane, 2-bromo-2-methylpropane, 1,2-dichloro-1-fluoroethane, 1,1,1-trichloroethane, 2,2,2-trifluoroethanol, 1-chloro-3-fluoropropane, 1,1,1-trifluoro-2-propanol, 2,3-dichloro-1,1,1-trifluoropropane, perfluoromethylcyclohexane, tetrachloromethane, 1-butylamine, ethylacetate, 1-chlorobutane, ethanol, butanone, 2,4-dimethylpentane, cyclohexane, n-propylformate, 2-ethoxy-butane, 2-propanol, acetonitrile, pentafluoro-1-propanol, tert-butanol, perfluoroheptane, 1,3-dichloro-1,1,2-trifluoropropane, 1-methoxy-2-methyl-butane, 1,1-dichloro-2,2,3-trifluoropropane, cyclohexene, 2,2-dimethoxypropane, 1,3,3-trichloro-1,1,2,2-tetrafluoropropane, 2-chloro-2-methylbutane, 1,2-dichloroethane, 1-ethoxy-2-methylpropane, diisopropylamine, 2-butanethiol, 1,2-dimethoxyethane, 1,1,1-trichloro-2,2,3,3-tetrafluoropropane, 3-methyl-2-butanamine, 1,1,3-trichloro-1,2,2,3-tetrafluoropropane, 1,3-dichloro-1,2,2-trifluoropropane, trichloroethene, diethoxymethane, 1,1-dichloropropane, 2-methyl-1-propanethiol, isopropylacetate, 1,2-dichloro-2-fluoropropane, 2-iodopropane, dichlorobromomethane, di-n-propylether, 3-pentylamine, n-methylbutylamine, 2-bromobutane, 2,2-difluorotetrachloroethane, diethylsulfide, 1-ethoxybutane, 1,1,2,2-tetrachloro-1,2-difluoro-ethane, 1-fluorohexane, 1-methoxy-2-propanamine, 1,3-dichloro-1,2,3-trifluoropropane, 2,3-dichloro-1-propene, 2-methylbutanal, 2-methoxyethanamine, 1,2-dichloropropane, propanol, tert-butylacetate, propionitrile, 3-chloropentane, trichloroacetaldehyde, 2-allyloxyethanol, butanethiol, isoamylchloride, 1-methoxy-pentane, ethylpropionate, 2-butanol, 1,2-dimethoxypropane, isopropyl-isobutyl-ether, 1,1,1-trichloro-2,2,3-trifluoropropane, methylcyclohexane, 1-chloro-4-fluorobutane, 2,4,4-trimethyl-1-pentene, 1-bromo-3-fluoropropane, dioxane, 1-bromobutane, 3-pentanone, 1,1,2-trichloro-2-fluoroethane, 1,1-diethoxyethane, 2-pentanone, 2-methyl-2-butanol, 1-iodopropane, 2-methoxy-1propanamine, 1,1,3-trichloro-1,2,2-trifluoropropane, 1,1,3-trichloro-2,2,3-trifluoropropane, trimethoxymethane, 2,2-dichlorobutane, cis-1,3-dichloropropene, n-pentylamine, 1,1-dichloro-2,2-difluoroethylmethylether, 2,2,4-trimethyl-2-pentene, bromotrichloromethane, n-methylhydroxylamine, perfluorooctane, 1,1,1,2-tetrachloro-2-fluoroethane, 3,3-dimethyl-2-butanone, 1,3-dioxane, piperidine, 1-bromo-2-chloroethane, isobutanol, 2-bromo-2-methylbutane, dipropylamine, 2,2,3,3-tetraflouro-1-propanol, 2-ethoxyethanamine, triethylfluorosilane, 1-methylcyclohexene, sec-butylacetate, trans-1,3-dichloropropene, 2-fluorotoluene, 2,2-dimethyl-1-propanol, 1,1,2-trichloroethane, 1,1,1-trichloro-3-fluoropropane, n-methyl-1,2-ethanediamine, 2,2-diethoxypropane, 1,3,5-trioxane, 3,3,3-trichloro-1-propene, 1-chloro-3,3-dimethylbutane, pyridine, 2,3-dimethylhexane, 1,1,1,2-tetrachloro-3,3,3-trifluoropropane, n-methylmorpholine, 3-pentanol, 4-methyl-2-pentanone, 1,2-diaminoethane, isobutyl-tert-butylether, 2-bromopentane, butyronitrile, 1-butanol, trichloroacetylchloride, 3-mercapto-1,2-propanediol, 2,3-dichlorobutane, sec-butyl-tert-butylether, 1-methoxy2-propanol, 1,1,3,3-tetrachloro-1,2,2-trifluoropropane, 1,2-propanediamine, 2,6-dimethyl-5-heptenal, perfluorobutanoicacid, 1,1,1,3-tetrachloro-2,2,3-trifluoropropane, 1-bromo-3-methylbutane, 1,3-dichloro-trans-2-butene, 1,3-dichloropropane, 1-(dimethylamino)-2-propanol, tetrahydrothiophene, tetrachloroethene, 3-methyl-3-pentanol, 1,2-dibromo-1-fluoroethane, 1,1-diethoxypropane, 1,2,2-trichloropropane, 1-chloro-2-methyl-2-propanol, 2-methoxyethanol, 1,2-dichlorobutane, 4,4,4-trifluorobutanol, 2-ethylbutylamine, perfluorononane, octane, diethylcarbonate, n-butylacetate, 1-pentanethiol, 1,2,2,3-tetrachloro-3,3-difluoropropane, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxy1propanol, 1,2-dichloro-2-butene, 1-iodobutane, hexanal, 1-ethoxy-2-propanol, 1,2-dibromoethane, 4-methyl-2-pentanol, chlorobenzene, ethylcyclohexane, bromoaceticacidmethylester, perfluorooctylbromide, 1,1,2-trichloropropane, 1,2-octanediol, 4-methyl-2-hexanamine, hexylamine, 2-chloro-1-propanol, methoxycyclohexane, 2-(dimethylamino)-ethanol, 1,3-dichlorobutane, cyclohexylamine, n-ethyl-2-dimethylaminoethylamine, ethoxyethanol, pentachlorofluoroethane, 3-hexanol, 2-hexanol, 2-methylpyrazine, 2-ethoxy-1-propanol, 1-pentanol, n-ethyl-morpholine, 1-methylpiperazine, 1,4-dimethylbenzene, 1,3-dimethylbenzene, 1,3-propanediamine, di-n-butylether, 3,3,4,4,5,5,6,6-octafluoro-1-pentanol, valeronitrile, (methyleneamino)acetonitrile, 1,2-dibromopropane, 1,2,3-trichloropropene, 2-heptanamine, 1,2,3-trimethylcyclohexane, 2,3-dimethylbutanol, 1-ethoxy-hexane, 1-chloro-3-bromopropane, perfluoro-n-decane, n,n-diethylethylenediamine, 3-furfural, 2,6-dimethylpyridine, 1,1,3,3-tetrachloro-1-fluoropropane, 1,2-dimethylbenzene, 4-methyl-2-hexanone, 1,1,2,2,3-pentchloro-3,3-difluoropropane, 1,1,1-triethoxyethane, styrene, 1-methoxy-2-acetoxypropane, 4-methylpyridine, n,n'-diethyl-1,2-ethanediamine, 1,1,2,2-tetrachloroethane, 2,6-dimethylmorpholine, 2-ethyl-1-butanol, 1,2-dichloropentane, 2-methyl-1-pentanol, methylhexanoate, 2-propoxyethanol, 2-aminophenol, 1-propoxy-2-propanol, dimethylethanolamine, isopropylchloroacetate, n-nonane, 2-heptanone, 1-hexanethiol, 1,1,1,3,3-pentachloro-2,2-difluoropropane, 1,2-propanedithiol, heptanal, dimethylformamide, 2,6-dimethylpyrazine, 2-isopropoxyethanol, diethyldisulfide, 2-methylpiperazine, 1-methylcyclohexanol, 1-bromohexane, cyclohexanone, n,n-di-2-propenyl-2-propen-1-amine, hexachloroethane, 1-heptanamine, 2,3-dimethylpyrazine, 2-ethoxyethanolacetate, 1,2,3-trichloropropane, 3-hydroxybutanal, 1-hexanol, 2-(methylamino)-ethanol, 1,4-butanediamine, 2,4-dimethylpyridine, 1-chloro-2-methylbenzene, 2-pyrimidinamine, 2-heptanol, 2-methoxy-3-methylpyrazine, dibutylamine, pentachloroethane, 4-methoxy-4-methyl-pentan-2-one, 1,4-dichloro-trans-2-butene, 3-ethoxy-1-propanol, 1,1,1,3,3-pentachloro-3-fluoropropane, cyclohexanol, 1,4-dichlorobutane, 3-methoxy-1-butanol, furfural, 3-chlorotoluene, diglyme, 1-chloro-4-methylbenzene, 1,1,1-trichloro-2-propanol, 2-(diethylamino)-ethanol, 3-methoxypropanenitrile, 2,2-diethoxyethanamine, 1,3,5-trimethylbenzene, 2-methoxy-n-(2-methoxyethyl)ethanamine, n,n,n',n'-tetraethylmethanediamine, 2-chloropyridine, bromocyclohexane, 2,3-heptanedione, 2-(ethylamino)ethanol, 3-methylcyclohexanol, 1,3-dibromopropane, 2-methylcyclohexanol, 3-octanone, diacetone alcohol, diethylaminopropylamine, 2-ethylhexylamine, 1,3-propanedithiol, thiophenol, 1,2,4-trimethylbenzene, ethoxybenzene, 1-butoxy-2-propanol, 2-furanmethanol, 2-butoxyethanol, ethanolamine, methylacetoacetate, 2-octanone, 2-aminopropanol, 1,4-dichlorobenzene, methylheptanoate, triethylenediamine, n-decane, n,n-dimethylpropanamide, 2-chlorophenol, 2-amino-1-butanol, 1,3-dichloro-2-propanol, 1-heptanol, 2-methyl-2-nitro-1-propanol, 2-propanol-1-methoxy-propanoate, perfluoro-n-dodecane, 1,5-pentanediamine, 1-methyl-2-isopropylbenzene, 2-octanol, cycloheptanone, 1,1,1,2,3-pentachloropropane, 3,4-dimethylpyridine, 1-octanamine, 1,2-dichlorobenzene, 1,1,1,2,2,3-hexachloro-3-fluoropropane, benzylmethylamine, n,n-dimethylbenzylamine, dimethylmalonate, phenol, diiodomethane, 1-chlorooctane, cyclohexanemethanol, 1,1,3,3-tetramethoxypropane, 1-chloro-2,4-dimethylbenzene, 2-(ethylthio)-ethanol, 1-ethoxy-2-methylbenzene, aniline, 1-bromo-4-methylbenzene, 2-ethyl-1-hexanol, tert-butylcyclohexylamine, dihexylphthalate, 1-fluorodecane, 2-chloro-1,4-dimethylbenzene, propyleneglycol, dimethylsulfate, dimethylsulfoxide, diethylpropanolamine, 2-methylphenol, 2-butoxyethanolacetate, diethylsulfoxide, 1-octanol, 2-bromopyridine, 2-(2-methoxyethoxy)ethanol, 1,2-butanediol, 2-bromophenol, 4-methylbenzenemethanamine, m-toluenethiol, 1,1,1,2,2,3,3-heptachloro-3-fluoropropane, 1-bromo-4-chlorobenzene, diethyleneglycolmonoethylether, 1,2,4,5-tetramethylbenzene, 2-propylcyclohexanone, 1,4-dibromobutane, trimethylphosphate, 2-methyl-2,4-pentanediol, 1,3-diethenylbenzene, methylbenzoate, 1-octanethiol, diethylmalonate, 2-methoxypyrimidine.

Avantageusement, ledit agent d'extraction organique peut avoir un facteur de séparation $S_{1,2}$ supérieur ou égal à 1,2 et une capacité d'absorption $C_{2,S}$ supérieure ou égale à 0,4 ; et être sélectionné parmi le groupe consistant en isopropylmethylamine, ethanedial, 2-chloro-2-methylpropane, 2-propanethiol, 2-ethoxy-propane, 1-chloro-2,2-difluoropropane, methyl-t-butylether, diethylamine, propanone, methylacetate, 1,1-dichloroethane, 4-methoxy-2-methyl-2-butanethiol, 2-bromopropane, chloromethoxymethane, difluorodiethylsilane, 2-butanamine, n-methylpropylamine, tert-butylthiol, isobutanal, tetrahydrofurane, 1-propanethiol, 2-chlorobutane, isopropylformate, diisopropylether, 1-bromopropane, methylglyoxal, 2-ethoxy-2-methyl-propane, 2-bromo-2-methylpropane, 1-chloro-3-fluoropropane, 1,1,1-trifluoro-2-propanol, 1-butylamine, ethylacetate, 1-chlorobutane, ethanol, butanone, n-propylformate, 2-ethoxy-butane, 2-propanol, acetonitrile, tert-butanol, 1-methoxy-2-methyl-butane, cyclohexene, 2,2-dimethoxypropane, 2-chloro-2-methylbutane, 1,2-dichloroethane, 1-ethoxy-2-methylpropane, diisopropylamine, 2-butanethiol, 1,2-dimethoxyethane, 3-methyl-2-butanamine, diethoxymethane, 1,1-dichloropropane, 2-methyl-1-propanethiol, isopropylacetate, 1,2-dichloro-2-fluoropropane, di-n-propylether,, 3-pentylamine, n-methylbutylamine, 2-bromobutane, diethylsulfide, 1-ethoxybutane, 1-fluorohexane, 1-methoxy-2-propanamine, 2,3-dichloro-1-propene, 2-methylbutanal, 2-methoxyethanamine, 1,2-dichloropropane, propanol, tert-butylacetate, propionitrile, 3-chloropentane, trichloroacetaldehyde, 2-allyloxyethanol, butanethiol, isoamylchloride, 1-methoxy-pentane, ethylpropionate, 2-butanol, 1,2-dimethoxypropane, isopropyl-isobutyl-ether, 1-chloro-4-fluorobutane, 2,4,4-trimethyl-1-pentene, 1-bromo-3-fluoropropane, dioxane, 1-bromobutane, 3-pentanone, 1,1-diethoxyethane, 2-pentanone, 2-methyl-2-butanol, 2-methoxy-1propanamine, trimethoxymethane, 2,2-dichlorobutane, cis-1,3-dichloropropene, n-pentylamine, 3,3-dimethyl-2-butanone, 1,3-dioxane, piperidine, isobutanol, 2-bromo-2-methylbutane, dipropylamine, 2-ethoxyethanamine, triethylfluorosilane, 1-methylcyclohexene, sec-butylacetate, trans-1,3-dichloropropene, 2,2-dimethyl-1-propanol, 1,1,1-trichloro-3-fluoropropane, n-methyl-1,2-ethanediamine, 2,2-diethoxypropane, 1,3,5-trioxane, 1-chloro-3,3-dimethylbutane, pyridine, n-methylmorpholine, 3-pentanol, 4-methyl-2-pentanone, 1,2-diaminoethane, isobutyl-tert-butylether, 2-bromopentane, butyronitrile, 1-butanol, 2,3-dichlorobutane, sec-butyl-tert-butylether, 1-methoxy2-propanol, 1,2-propanediamine, 2,6-dimethyl-5-heptenal, 1-bromo-3-methylbutane, 1,3-dichloro-trans-2-butene, 1,3-dichloropropane, 1-(dimethylamino)-2-propanol, tetrahydrothiophene, 3-methyl-3-pentanol, 1,1-diethoxypropane, 1,2,2-trichloropropane, 1-chloro-2-methyl-2-propanol, 2-methoxyethanol, 1,2-dichlorobutane, 4,4,4-trifluorobutanol, 2-ethylbutylamine, diethylcarbonate, n-butylacetate, 1-pentanethiol, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxy1-propanol, 1,2-dichloro-2-butene, hexanal, 1-ethoxy-2-propanol, 4-methyl-2-pentanol, bromoaceticacidmethylester, 1,2-octanediol, 4-methyl-2-hexanamine, hexylamine, methoxycyclohexane, 2-(dimethylamino)-ethanol, 1,3-dichlorobutane, cyclohexylamine, n-ethyl-2-dimethylaminoethylamine, ethoxyethanol, 3-hexanol, 2-hexanol, 2-methylpyrazine, 2-ethoxy-1-propanol, 1-pentanol, n-ethyl-morpholine, 1-methylpiperazine, 1,4-dimethylbenzene, 1,3-propanediamine, di-n-butylether, valeronitrile, (methyleneamino)acetonitrile, 2-heptanamine, 2,3-dimethylbutanol, 1-ethoxy-hexane, 1-chloro-3-bromopropane, n,n-diethylethylenediamine, 3-furfural, 2,6-dimethylpyridine, 4-methyl-2-hexanone, 1,1,1-triethoxyethane, styrene, 1-methoxy-2-acetoxypropane, 4-methylpyridine, n,n'-diethyl-1,2-ethanediamine, 2,6-dimethylmorpholine, 2-ethyl-1-butanol, 1,2-dichloropentane, 2-methyl-1-pentanol, methylhexanoate, 2-propoxyethanol, 1-propoxy-2-propanol, dimethylethanolamine, isopropylchloroacetate, 2-heptanone, 1-hexanethiol, 1,2-propanedithiol, heptanal, dimethylformamide, 2,6-dimethylpyrazine, 2-isopropoxyethanol, diethyldisulfide, 2-methylpiperazine, 1-methylcyclohexanol, 1-bromohexane, cyclohexanone, 1-heptanamine, 2,3-dimethylpyrazine, 2-ethoxyethanolacetate, 3-hydroxy-butanal, 1-hexanol, 2-(methylamino)-ethanol, 1,4-butanediamine, 2,4-dimethylpyridine, 2-pyrimidinamine, 2-heptanol, 2-methoxy-3-methylpyrazine, dibutylamine, 4-methoxy-4-methyl-pentan-2-one, 1,4-dichloro-trans-2-butene, 3-ethoxy-1-propanol, cyclohexanol, 1,4-dichlorobutane, 3-methoxy-1-butanol, furfural, diglyme, 1,1,1-trichloro-2-propanol, 2-(diethylamino)-ethanol, 3-methoxypropanenitrile, 2,2-diethoxyethanamine, 1,3,5-trimethylbenzene, 2-methoxy-n-(2-methoxyethyl)ethanamine, 2-chloropyridine, bromocyclohexane, 2,3-heptanedione, 2-(ethylamino)ethanol, 3-methylcyclohexanol, 1,3-dibromopropane, 2-methylcyclohexanol, 3-octanone, diacetone alcohol, diethylaminopropylamine, 2-ethylhexylamine, 1,3-propanedithiol, ethoxybenzene, 1-butoxy-2-propanol, 2-furanmethanol, 2-butoxyethanol, methylacetoacetate, 2-octanone, 2-aminopropanol, 1,4-dichlorobenzene, methylheptanoate, triethylenediamine, n,n-dimethylpropanamide, 2-chlorophenol, 2-amino-1-butanol, 1-heptanol, 2-methyl-2-nitro-1-propanol, 2-propanol-1-methoxy-propanoate, 1,5-pentanediamine, 2-octanol, cycloheptanone, 3,4-dimethylpyridine, 1-octanamine, benzylmethylamine, dimethylmalonate, 1-chlorooctane, cyclohexanemethanol, 1,1,3,3-tetramethoxypropane, 2-(ethylthio)-ethanol, 1-ethoxy-2-methylbenzene, aniline, 2-ethyl-1-hexanol, tert-butylcyclohexylamine, dihexylphthalate, 1-fluorodecane, 2-chloro-1,4-dimethylbenzene, dimethylsulfate, dimethylsulfoxide, diethylpropanolamine, 2-methylphenol, 2-butoxyethanolacetate, diethylsulfoxide, 1-octanol, 2-bromopyridine, 2-(2-methoxyethoxy)ethanol, 1,2-butanediol, 2-bromophenol, 4-methylbenzenemethanamine, diethyleneglycolmonoethylether, 1,2,4,5-tetramethylbenzene, 2-propylcyclohexanone, 1,4-dibromobutane, trimethylphosphate, 2-methyl-2,4-pentanediol, 1,3-diethenylbenzene, methylbenzoate, 1-octanethiol, diethylmalonate, 2-methoxypyrimidine.

De préférence, ledit agent d'extraction organique peut avoir un facteur de séparation $S_{1,2}$ supérieur ou égal à 1,4 et une capacité d'absorption $C_{2,S}$ supérieure ou égale à 0,6 ; et être sélectionné parmi le groupe consistant en isopropylmethylamine, 2-ethoxy-propane, methyl-t-butylether, diethylamine, propanone, methylacetate, 4-methoxy-2-methyl-2-butanethiol, 2-butanamine, n-methylpropylamine, isobutanal, tetrahydrofurane, Isopropylformate, diisopropylether, methylglyoxal, 2-ethoxy-2-methyl-propane, 1-butylamine, ethylacetate, butanone, n-propylformate, 2-ethoxy-butane, 2-propanol, tert-butanol, 1-methoxy-2-methyl-butane, 2,2-dimethoxypropane, 1-ethoxy-2-methylpropane, diisopropylamine, 1,2-dimethoxyethane, 3-methyl-2-butanamine, diethoxymethane, isopropylacetate, di-n-propylether, 3-pentylamine, n-methylbutylamine, 1-ethoxybutane, 1-methoxy-2-propanamine, 2-methylbutanal, 2-methoxyethanamine, tert-butylacetate, propionitrile, 2-allyloxyethanol, 1-methoxy-pentane, ethylpropionate, 2-butanol, 1,2-dimethoxypropane, isopropyl-isobutyl-ether, dioxane, 3-pentanone, 1,1-diethoxyethane, 2-pentanone, 2-methyl-2-butanol, 2-methoxy-1propanamine, trimethoxymethane, n-pentylamine, 3,3-dimethyl-2-butanone, 1,3-dioxane, piperidine, isobutanol, dipropylamine, 2-ethoxyethanamine, sec-butylacetate, 2,2-dimethyl-1-propanol, n-methyl-1,2-ethanediamine, 2,2-diethoxypropane, 1,3,5-trioxane, pyridine, n-methylmorpholine, 3-pentanol, 4-methyl-2-pentanone, 1,2-diaminoethane, butyronitrile, 1-butanol, sec-butyl-tert-butylether, 1-methoxy2-propanol, 1,2-propanediamine, 2,6-dimethyl-5-heptenal, 1-(dimethylamino)-2-propanol, 3-methyl-3-pentanol, 1,1-diethoxypropane, 2-methoxyethanol, 2-ethylbutylamine, diethylcarbonate, n-butylacetate, 2-chloro-1,1-dimethoxyethane, 2-hexanone, n-ethylethylenediamine, 5-hexen-2-one, 2-methylpyridine, 2-methoxy1-propanol, hexanal, 1-ethoxy-2-propanol, 4-methyl-2-pentanol, 1,2-octanediol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 3-hexanol, 2-hexanol, 2-methylpyrazine, 2-ethoxy-1-propanol, 1-pentanol, n-ethyl-morpholine, 1-methylpiperazine, 1,3-propanediamine, di-n-butylether, valeronitrile, (methyleneamino)acetonitrile, 2-heptanamine, 2,3-dimethylbutanol, 1-ethoxy-hexane, n,n-diethylethylenediamine, 3-furfural, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, 2-ethyl-1-butanol, 2-methyl-1-pentanol, methylhexanoate, 2-propoxyethanol, 1-propoxy-2-propanol, dimethylethanolamine, isopropylchloroacetate, 2-heptanone, heptanal, dimethylformamide, 2,6-dimethylpyrazine, 2-isopropoxyethanol, 2-methylpiperazine, 1-methylcyclohexanol, cyclohexanone, 1-heptanamine, 2,3-dimethylpyrazine, 2-ethoxyethanolacetate, 1-hexanol, 2-(methylamino)-ethanol, 1,4-butanediamine, 2,4-dimethylpyridine, 2-heptanol, 2-methoxy-3-methylpyrazine, dibutylamine, 4-methoxy-4-methyl-pentan-2-one, 3-ethoxy-1-propanol, cyclohexanol, 3-methoxy-1-butanol, furfural, diglyme, 2-(diethylamino)-ethanol, 3-methoxypropanenitrile, 2,2-diethoxyethanamine, 2-methoxy-n-(2-methoxyethyl)ethanamine, 2,3-heptanedione, 2-(ethylamino)ethanol, 3-methylcyclohexanol, 2-methylcyclohexanol, 3-octanone, diacetone alcohol, diethylaminopropylamine, 2-ethylhexylamine, 1-butoxy-2-propanol, 2-butoxyethanol, methylacetoacetate, 2-octanone, 2-aminopropanol, methylheptanoate, triethylenediamine, n,n-dimethylpropanamide, 2-amino-1-butanol, 1-heptanol, 2-propanol-1-methoxy-propanoate, 1,5-pentanediamine, 2-octanol, cycloheptanone, 3,4-dimethylpyridine, 1-octanamine, benzylmethylamine, dimethylmalonate, cyclohexanemethanol, 1,1,3,3-tetramethoxypropane, 2-(ethylthio)-ethanol, 2-ethyl-1-hexanol, tert-butylcyclohexylamine, dihexylphthalate, diethylpropanolamine, 2-butoxyethanolacetate, diethylsulfoxide, 1-octanol, 2-(2-methoxyethoxy)ethanol, 4-methylbenzenemethanamine, diethyleneglycolmonoethylether, 2-propylcyclohexanone, trimethylphosphate, 2-methyl-2,4-pentanediol, methylbenzoate, diethylmalonate, 2-methoxypyrimidine.

En particulier, ledit agent d'extraction organique peut avoir un facteur de séparation $S_{1,2}$ supérieur ou égal à 1,6 et une capacité d'absorption $C_{2,S}$ supérieure ou égale à 0,8 ; et être sélectionné parmi le groupe consistant en isopropylmethylamine, methyl-t-butylether, diethylamine, propanone, methylacetate, 2-butanamine, n-methylpropylamine, tetrahydrofurane, 1-butylamine, ethylacetate, butanone, n-propylformate, tert-butanol, 2,2-dimethoxypropane, diisopropylamine, 1,2-dimethoxyethane, 3-methyl-2-butanamine, diethoxymethane, isopropylacetate, 3-pentylamine, n-methylbutylamine, 1-methoxy-2-propanamine, 2-methoxyethanamine, tert-butylacetate, 2-allyloxyethanol, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 3-pentanol, 1,2-diaminoethane, 1-methoxy2-propanol, 1,2-propanediamine, 2,6-dimethyl-5-heptenal, 1-(dimethylamino)-2-propanol, 3-methyl-3-pentanol, 2-ethylbutylamine, diethylcarbonate, n-butylacetate, 2-hexanone, n-ethylethylenediamine, 2-methoxy1-propanol, 1-ethoxy-2-propanol, 1,2-octanediol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-ethoxy-1-propanol, 1-methylpiperazine, 1,3-propanediamine, 2-heptanamine, n,n-diethylethylenediamine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol, dimethylethanolamine, 2-heptanone, dimethylformamide, 2-isopropoxyethanol, 2-methylpiperazine, cyclohexanone, 1-heptanamine, 2-ethoxyethanolacetate, 2-(methylamino)-ethanol, 1,4-butanediamine, 2,4-dimethylpyridine, 2-heptanol, 2-methoxy-3-methylpyrazine, 4-methoxy-4-methyl-pentan-2-one, 3-ethoxy-1-propanol, cyclohexanol, 3-methoxy-1-butanol, diglyme, 2-(diethylamino)-ethanol, 2,2-diethoxyethanamine, 2-methoxy-n-(2-methoxyethyl)ethanamine, 2-(ethylamino)ethanol, 2-methylcyclohexanol, 3-octanone, diacetone alcohol, diethylaminopropylamine, 2-ethylhexylamine, 1-butoxy-2-propanol, 2-butoxyethanol, 2-octanone, methylheptanoate, triethylenediamine, n,n-dimethylpropanamide, 2-amino-1-butanol, 2-propanol-1-methoxy-propanoate, 1,5-pentanediamine, cycloheptanone, 3,4-dimethylpyridine, 1-octanamine, benzylmethylamine, dimethylmalonate, 1,1,3,3-tetramethoxypropane, dihexylphthalate, diethylpropanolamine, 2-butoxyethanolacetate, diethylsulfoxide, 2-(2-methoxyethoxy)ethanol, 4-methylbenzenemethanamine, diethyleneglycolmonoethylether, 2-propylcyclohexanone, trimethylphosphate, 2-methyl-2,4-pentanediol, methylbenzoate, diethylmalonate, 2-methoxypyrimidine.

Plus particulièrement, ledit agent d'extraction organique peut avoir un facteur de séparation $S_{1,2}$ supérieur ou égal à 1,6 et une capacité d'absorption $C_{2,S}$ supérieure ou égale à 1,0 ; et peut être sélectionné parmi le groupe consistant en isopropylmethylamine, methyl-t-butylether, diethylamine, propanone, methylacetate, 2-butanamine, n-methylpropylamine, tetrahydrofurane, 1-butylamine, ethylacetate, butanone, n-propylformate, -dimethoxypropane, diisopropylamine, 1,2-dimethoxyethane, 3-methyl-2-butanamine, diethoxymethane, isopropylacetate, 3-pentylamine, n-methylbutylamine, 1-methoxy-2-propanamine, 2-methoxyethanamine, tert-butylacetate, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 1,2-diaminoethane, 1-methoxy2-propanol, 1,2-propanediamine, 2,6-dimethyl-5-heptenal, 1-(dimethylamino)-2-propanol, 3-methyl-3-pentanol, 2-ethylbutylamine, diethylcarbonate, n-butylacetate, 2-hexanone, n-ethylethylenediamine, 2-methoxy1-propanol, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-ethoxy-1-propanol, 1-methylpiperazine, 1,3-propanediamine, 2-heptanamine, n,n-diethylethylenediamine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol, 2-heptanone, dimethylformamide, 2-isopropoxyethanol, 2-methylpiperazine, cyclohexanone, 1-heptanamine, 2-ethoxyethanolacetate, 1,4-butanediamine, 2,4-dimethylpyridine, 2-methoxy-3-methylpyrazine, 4-methoxy-4-methyl-pentan-2-one, 3-ethoxy-1-propanol, 3-methoxy-1-butanol, diglyme, 2-(diethylamino)-ethanol, 2,2-diethoxyethanamine, 2-methoxy-n-(2-methoxyethyl)ethanamine, 2-(ethylamino)ethanol, 3-octanone, diacetone alcohol, diethylaminopropylamine, 2-ethylhexylamine, 1-butoxy-2-propanol, 2-butoxyethanol, 2-octanone, methylheptanoate, triethylenediamine, n,n-dimethylpropanamide, 2-propanol-1-methoxypropanoate, 1,5-pentanediamine, cycloheptanone, 3,4-dimethylpyridine, 1-octanamine, benzylmethylamine, 1,1,3,3-tetramethoxypropane, dihexylphthalate, diethylpropanolamine, 2-butoxyethanolacetate, diethylsulfoxide, 2-(2-methoxyethoxy)ethanol, 4-methylbenzenemethanamine, diethyleneglycolmonoethylether, 2-propylcyclohexanone, trimethylphosphate, 2-methyl-2,4-pentanediol, methylbenzoate, diethylmalonate, 2-methoxypyrimidine.

De manière privilégiée, ledit agent d'extraction organique peut être sélectionné parmi le groupe consistant en diethylamine, propanone, methylacetate, tetrahydrofurane, ethylacetate, butanone, diethoxymethane, isopropylacetate, tert-butylacetate, dioxane, 3-pentanone, 1,1-diethoxyethane, 2-pentanone, n-pentylamine, 1,3-dioxane, sec-butylacetate, 1,2-diaminoethane, 1-methoxy2-propanol, n-butylacetate, 1-ethoxy-2-propanol.

Ladite première composition peut être une composition azéotropique ou quasi-azéotropique comprenant 2-chloro-3,3,3-trifluoropropene (1233xf) et au moins un des composés choisi parmi le groupe consistant en E-1-chloro-3,3,3-trifluoro-1-propene (1233zdE) et 1,1,1,3,3-pentafluoropropane (245fa).

Ladite première composition peut être une composition azéotropique ou quasi-azéotropique comprenant 2-chloro-3,3,3-trifluoropropene (1233xf) et au moins deux ou l'ensemble des composés choisi parmi le groupe consistant en E-1-chloro-3,3,3-trifluoro-1-propene (1233zdE) et 1,1,1,3,3-pentafluoropropane (245fa).

La teneur en chacun des composés dans la première composition azéotropique ou quasi-azéotropique est telle qu'exprimée ci-dessus.

En particulier, ladite première composition peut être une composition azéotropique ou quasi-azéotropique comprenant 2-chloro-3,3,3-trifluoropropene (1233xf) et 1,1,1,3,3-pentafluoropropane (245fa). La teneur en chacun des composés dans cette composition particulière est telle qu'exprimée ci-dessus en référence aux teneurs individuelles de chacun des composés. Ladite composition azéotropique ou quasi-azéotropique est obtenue à une température et une pression donnée. De préférence, ladite composition azéotropique ou quasi-azéotropique peut comprendre de 45 à 65 mole% de 2-chloro-3,3,3-trifluoropropene (1233xf) et de 35 à 55 mole% de 1,1,1,3,3-pentafluoropropane (245fa) sur base de la composition totale. Ainsi, la seconde composition peut comprendre 2-chloro-3,3,3-trifluoropropene (1233xf) et 1,1,1,3,3-pentafluoropropane (245fa) et ledit agent d'extraction organique tel que défini ci-dessus lorsque le facteur de séparation et la capacité d'absorption sont calculés avec le 1,1,1,3,3-pentafluoropropane (245fa) comme second composé du couple binaire.

Selon un mode de réalisation particulier, ladite première composition peut être une composition azéotropique ou quasi-azéotropique comprenant 2-chloro-3,3,3-trifluoropropene (1233xf) et E-1-chloro-3,3,3-trifluoropropene (1233zdE). La teneur en chacun des composés dans cette composition particulière est telle qu'exprimée ci-dessus en référence aux teneurs individuelles de chacun des composés. Ainsi, la seconde composition peut comprendre 2-chloro-3,3,3-trifluoropropene (1233xf), E-1-chloro-3,3,3-trifluoropropene (1233zdE) et ledit agent d'extraction organique, ayant de préférence un facteur de séparation $S_{1,2}$ supérieur ou égal à 1,1, avantageusement supérieur ou égal à 1,2, de préférence supérieur ou égal à 1,4, plus préférentiellement supérieur ou égal à 1,6, en particulier supérieur ou égal à 1,8 et plus particulièrement supérieur ou égal à 2,0 ; et/ou ledit agent d'extraction organique peut avoir une capacité d'absorption $C_{2,S}$ supérieure ou égale à 0,20, avantageusement supérieure ou égale à 0,40, de préférence supérieure ou égale à 0,60, plus préférentiellement supérieure ou égale à 0,80, en particulier supérieur ou égal à 1,0, plus particulièrement supérieure ou égale à 1,4, de manière privilégiée supérieure ou égale à 1,6. Ledit agent d'extraction organique peut être tel que défini ci-dessus lorsque le facteur de séparation et la capacité d'absorption sont calculés avec le E-1-chloro-3,3,3-trifluoropropene (1233zdE) comme second composé du couple binaire.

Selon un mode de réalisation préféré, ladite troisième composition comprenant le l'agent d'extraction organique et ledit au moins un des composés choisi parmi le groupe consistant en E-1-chloro-3,3,3-trifluoro-1-propene (1233zdE) et 1,1,1,3,3-pentafluoropropane (245fa) peut être soumis à une distillation pour séparer l'agent d'extraction organique et ledit au moins un des composés choisi parmi le groupe consistant en E-1-chloro-3,3,3-trifluoro-1-propene (1233zdE) et 1,1,1,3,3-pentafluoropropane (245a). Ledit au moins un des composés choisi parmi le groupe consistant en E-1-chloro-3,3,3-trifluoro-1-propene (1233zdE) et 1,1,1,3,3-pentafluoropropane (245fa). peut être envoyé vers un incinérateur ou un dispositif de purification d'un des composés.

Selon un mode de réalisation particulier, le courant comprenant le 2-chloro-3,3,3-trifluoropropène séparé à l'étape b) du présent procédé est recyclé pour être utilisé dans un procédé de production du 2,3,3,3-tetrafluoropropène. Ceci permet d'améliorer l'efficacité global du procédé puisque le 2-chloro-3,3,3-trifluoropropène peut former par fluoration du 2,3,3,3-tetrafluoropropène. De préférence, dans ce mode de réalisation, des traces d'agent d'extraction organique peuvent être présentes dans le courant comprenant le 2-chloro-3,3,3-trifluoropropène. De préférence, l'agent d'extraction organique est sélectionné de sorte à éviter ou minimiser la désactivation du catalyseur utilisé dans le procédé de production du 2,3,3,3-tetrafluoropropène.

Le présente procédé permet donc de purifier le 2-chloro-3,3,3-trifluoropropène. Avantageusement, la teneur en au moins un des composés choisi parmi le groupe consistant en E-1-chloro-3,3,3-trifluoro-1-propene (1233zdE) et 1,1,1,3,3-pentafluoropropane (245fa) dans le courant comprenant du 2-chloro-3,3,3-trifluoropropène, obtenu à l'étape b) du présent procédé de purification, est inférieure à la teneur de celui-ci ou de ceux-ci dans ladite première composition. Par exemple, la teneur en l'un quelconque des composés peut être diminuée de 50%, avantageusement de 75%, de préférence de 90%, en particulier de 95%, plus particulièrement de 98%. Les teneurs sont exprimées en pourcentage en poids. De préférence, le courant comprenant le 2-chloro-3,3,3-trifluoropropène obtenu à l'étape b) du présent procédé de purification peut être dépourvu dudit au moins un des composés choisi parmi le groupe consistant en E-1-chloro-3,3,3-trifluoro-1-propene (1233zdE) et 1,1,1,3,3-pentafluoropropane (245fa) lorsque celui-ci ou ceux-ci sont présents dans ladite première composition. Le terme « dépourvu » signifie que le courant comprenant le 2-chloro-3,3,3-trifluoropropène comprend moins de 50 ppm, avantageusement moins de 20 ppm, de préférence moins de 10 ppm du composé considéré sur base du poids total du courant.

Selon un mode de réalisation préféré, ladite première composition mise en oeuvre à l'étape a) du présent procédé est obtenue au cours de la mise en oeuvre d'un procédé de production du 2,3,3,3-tetrafluoropropene, à partir d'une purge de la boucle. La purge de la boucle réactionnelle ou une partie de celle-ci peut être préalablement purifiée avant d'être utilisée dans le présent procédé de purification du 2-chloro-3,3,3-trifluoropropène, par exemple si celle-ci est issue d'une réaction de fluoration ou de déhydrofluoration. Dans ce cas, la purge de la boucle réactionnelle ou une partie de celle-ci comprendrait du HF, 1,1,1,2,2-pentafluoropropane (245cb), 1,3,3,3-tetrafluoro-1-propene (1234ze), 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa). Ainsi, le présent procédé peut comprendre préalablement à l'étape a) les étapes :

i') séparation à basse température de ladite composition liquide pour former une première phase riche en HF et une seconde phase organique contenant 1,1,1,2,2-pentafluoropropane (245cb), 1,3,3,3-tetrafluoro-1-propene (1234ze), 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa);
ii') distillation de ladite seconde phase organique pour former et récupérer, avantageusement en tête de colonne de distillation, un premier courant contenant 1,1,1,2,2-pentafluoropropane (245cb) et 1,3,3,3-tetrafluoro-1-propene (1234ze), et pour former et récupérer, avantageusement en bas de colonne de distillation, un second courant comprenant du 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa);
iii') récupération dudit second courant correspondant à ladite première composition utilisé à l'étape a) du présent procédé de purification du 2-chloro-3,3,3-trifluoropropène (1233xf).

Le premier courant contenant 1,1,1,2,2-pentafluoropropane (245cb) et 1,3,3,3-tetrafluoro-1-propene (1234ze) formé et récupéré à l'étape ii') peut être recyclé dans la boucle réactionnelle, c'est-à-dire recyclé à l'étape A) ci-dessous.

Optionnellement, si le second courant récupéré à l'étape ii') contient des impuretés lourdes, celles-ci peuvent être éliminées par distillation. Dans ce cas, les impuretés lourdes sont récupérées en bas de colonne de distillation, et le second courant correspondant à ladite première composition utilisé à l'étape a) du présent procédé de purification du 2-chloro-3,3,3-trifluoropropène (1233xf) est récupéré en tête de colonne de distillation. Les impuretés lourdes peuvent être ultérieurement incinérées.

Selon un second aspect, la présente invention fournit un procédé de production du 2,3,3,3-tetrafluoro-1-propène. En outre, ce procédé peut inclure la purification du 2-chloro-3,3,3-trifluoropropene en vue de son recyclage vers un réacteur de fluoration pour la production de 2,3,3,3-tetrafluoro-1-propène. Ainsi, la présente invention fournit un procédé de production de 2,3,3,3-tetrafluoro-1-propène comprenant les étapes de :

A) dans un réacteur, fluoration en présence d'un catalyseur d'un composé de formule $CX(Y)_2\text{-}CX(Y)_m\text{-}CH_mXY$ (I) dans laquelle X et Y représentent indépendamment H, F, ou Cl et m = 0 ou 1 ; et/ou fluoration en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 1,1,1,2,2,-pentafluoropropane, du 2-chloro-3,3,3-trifluoropropène, et au moins un des composés choisi parmi le groupe consistant en E-1-chloro-3,3,3-trifluoro-1-propene (1233zdE) et 1,1,1,3,3-pentafluoropropane (245fa).

C) distillation du courant récupéré à l'étape B) et récupération en tête de colonne de distillation d'un courant comprenant du 1,1,1,2,2-pentafluoropropane et en bas de colonne de distillation d'un courant comprenant du 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa)

D) mise en oeuvre du procédé de purification du 2-chloro-3,3,3-trifluoropropène selon la présente invention à partir du courant récupéré à l'étape C) et comprenant du 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa); et

E) recyclage à l'étape A) du courant comprenant le 2-chloro-3,3,3-trifluoropropène formé et récupéré à l'étape b) du procédé de purification mis en oeuvre à l'étape D).

Le courant comprenant du 1,1,1,2,2-pentafluoropropane récupéré à l'étape C) peut être recyclé à l'étape A).

Ainsi, le procédé de production de 2,3,3,3-tetrafluoro-1-propène comprenant les étapes de :

A) dans un réacteur, fluoration en présence d'un catalyseur d'un composé de formule $CX(Y)_2\text{-}CX(Y)_m\text{-}CH_mXY$ (I) dans laquelle X et Y représentent indépendamment H, F, ou Cl et m = 0 ou 1 ; et/ou fluoration en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 1,1,1,2,2-pentafluoropropane, du 2-chloro-3,3,3-trifluoropropène, et au moins un des composés choisi parmi le groupe consistant en E-1-chloro-3,3,3-trifluoro-1-propene (1233zdE) et 1,1,1,3,3-pentafluoropropane (245fa).

C) distillation du courant récupéré à l'étape B) et récupération en tête de colonne de distillation d'un courant comprenant du 1,1,1,2,2-pentafluoropropane et en bas de colonne de distillation d'un courant comprenant du 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa)

D) mise en oeuvre du procédé de purification du 2-chloro-3,3,3-trifluoropropène selon la présente invention à partir du courant récupéré à l'étape C) et comprenant du 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa), comprenant les étapes de :

a) mise en contact avec au moins un agent d'extraction organique du courant comprenant 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa) pour former une seconde composition ;
b) distillation extractive de ladite seconde composition pour former :

i) une troisième composition comprenant ledit agent d'extraction organique et ledit au moins un des composés choisi parmi le groupe consistant en E-1-chloro-3,3,3-trifluoro-1-propene (1233zdE) et 1,1,1,3,3-pentafluoropropane (245fa),

ii) un courant comprenant le 2-chloro-3,3,3-trifluoropropène;

c) récupération et séparation de ladite troisième composition comprenant un courant comprenant ledit agent d'extraction organique et un courant comprenant ledit au moins un des composés choisi parmi le groupe consistant en E-1-chloro-3,3,3-trifluoro-1-propene (1233zdE) et 1,1,1,3,3-pentafluoropropane (245fa),

E) recyclage à l'étape A) du courant comprenant le 2-chloro-3,3,3-trifluoropropène formé et récupéré à l'étape b) du procédé de purification mis en oeuvre à l'étape D).

De préférence, l'agent d'extraction organique séparé à l'étape c) peut être recyclé à l'étape a).

De préférence, le courant comprenant ledit au moins un des composés choisi parmi le groupe consistant en E-1-chloro-3,3,3-trifluoro-1-propene (1233zdE) et 1,1,1,3,3-pentafluoropropane (245fa) séparé à l'étape c) peut être récupéré pour être incinéré ou pour purifier un ou plusieurs constituants de celui-ci, par exemple E-1-chloro-3,3,3-trifluoro-1-propene (1233zdE) ou 1,1,1,3,3-pentafluoropropane (245fa).

Selon un mode de réalisation préféré, le courant récupéré à l'étape B) comprend également HF, et 1,3,3,3-tetrafluoro-1-propene (1234ze), ceux-ci étant, préalablement à la mise en oeuvre de la distillation de l'étape C), traité, selon les étapes suivantes :

i') séparation à basse température dudit courant pour former une première phase riche en HF et une seconde phase organique contenant 1,1,1,2,2-pentafluoropropane (245cb), 1,3,3,3-tetrafluoro-1-propene (1234ze), 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa);

ii') distillation de ladite seconde phase organique pour former et récupérer, avantageusement en tête de colonne de distillation, un premier courant contenant 1,1,1,2,2-pentafluoropropane (245cb) et 1,3,3,3-tetrafluoro-1-propene (1234ze), et pour former et récupérer, avantageusement en bas de colonne de distillation, un second courant comprenant du 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa);

iii') récupération dudit second courant et mise en oeuvre de l'étape D) à partir de celui-ci.

Le premier courant contenant 1,1,1,2,2-pentafluoropropane (245cb) et 1,3,3,3-tetrafluoro-1-propene (1234ze) formé et récupéré à l'étape ii') peut être recyclé à l'étape A).

Plus particulièrement, l'étape A) est effectuée à partir de 1,1,2,3-tetrachloropropène, le 2,3,3,3,-tetrachloropropène, le 1,1,3,3-tetrachloropropène, le 1,3,3,3-tetrachloropropène, le 1,1,1,2,3-pentachloropropane, le 1,1,1,3,3-pentachloropropane, le 1,1,2,2,3-pentachloropropane, le 1,2-dichloro-3,3,3-trifluoropropane, le 2-chloro-2,3,3,3-tetrafluoropropane, le 1,1,1,2,2-pentafluoropropane, le 1-chloro-1,3,3,3-tetrafluoropropane et le 1,1,1,3,3-pentafluoropropane, de préférence à partir de 1,1,1,2,3-pentachloropropane, 1,1,2,3,tetrachloropropène, du 1,1,1,2,2-pentafluoropropane et/ou du 2-chloro-3,3,3-trifluoro-1-propène ; en particulier à partir du 1,1,1,2,3-pentachloropropane (240db).

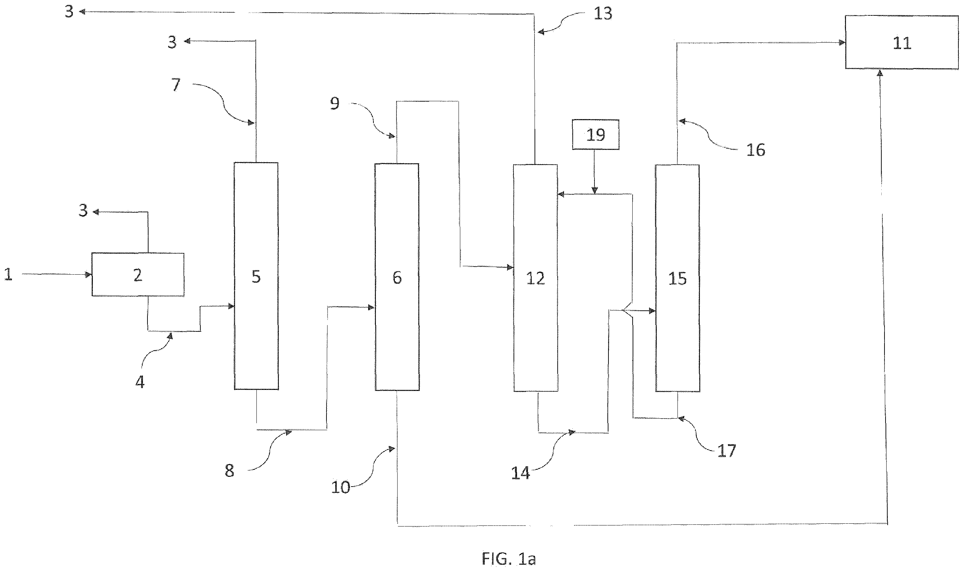

La figure 1a représente schématiquement un dispositif mettant en oeuvre un procédé de purification du 2-chloro-3,3,3-trifluoropropene (1233xf) selon un mode de réalisation particulier de l'invention. Une composition comprenant HF, 1,1,1,2,2-pentafluoropropane (245cb), 1,3,3,3-tetrafluoro-1-propene (1234ze), 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa) est fourni en 1. La composition peut également comprendre des impuretés lourdes. La composition est refroidie en 2 pour former une phase supérieure comprenant HF et une phase organique inférieure comprenant 1,1,1,2,2-pentafluoropropane (245cb), 1,3,3,3-tetrafluoro-1-propene (1234ze), 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa); et des impuretés lourdes. La phase supérieure comprenant HF est recyclée vers 3, c'est-à-dire vers le réacteur de fluoration catalytique tandis que la phase organique inférieure est transférée par le conduit 4 vers une colonne de distillation 5. La distillation effectuée en 5 permet de récupérer en tête de colonne de distillation via le conduit 7 un courant comprenant 1,1,1,2,2-pentafluoropropane (245cb) et 1,3,3,3-tetrafluoro-1-propene (1234ze) qui peuvent être recyclés vers 3, c'est-à-dire vers le réacteur de fluoration catalytique. Le courant récupéré en bas de colonne de distillation 5 comprend 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa); et des impuretés lourdes. Ce courant est acheminé via le conduit 8 vers la colonne de distillation 6 pour éliminer les impuretés lourdes. Ces dernières sont récupérées en bas de colonne de distillation 6 et acheminées vers un dispositif 11 via le conduit 10. Le dispositif 11 peut être un incinérateur, un oxydateur thermique ou un dispositif de purification. Un courant comprenant 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa) est récupéré en tête de colonne de distillation 6 et acheminé via le conduit 9 vers une colonne de distillation extractive 12. Cette dernière colonne de distillation 12 permet de purifier le 2-chloro-3,3,3-trifluoropropène (1233xf) qui est récupéré en tête de colonne de distillation pour être recyclé via le conduit 13 vers 3, c'est-à-dire vers le réacteur de fluoration catalytique. La colonne de distillation extractive 12 est alimentée en agent d'extraction organique 19 via le conduit 17. Ledit agent d'extraction organique et ledit au moins un des composés choisi parmi le groupe consistant en E-1-chloro-3,3,3-trifluoro-1-propene (1233zdE) et 1,1,1,3,3-pentafluoropropane (245fa) sont récupérés en bas de colonne de distillation extractive 12 et transférés vers une colonne de distillation 15 via le conduit 14 pour les séparer. L'agent d'extraction organique est récupéré en bas de colonne de distillation 15 et recyclé dans la colonne de distillation extractive 12 via le conduit 17. Ledit au moins un des composés choisi parmi le groupe consistant en E-1-chloro-3,3,3-trifluoro-1-propene (1233zdE) et 1,1,1,3,3-pentafluoropropane (245fa) est transféré vers un dispositif 11 via le conduit 16.

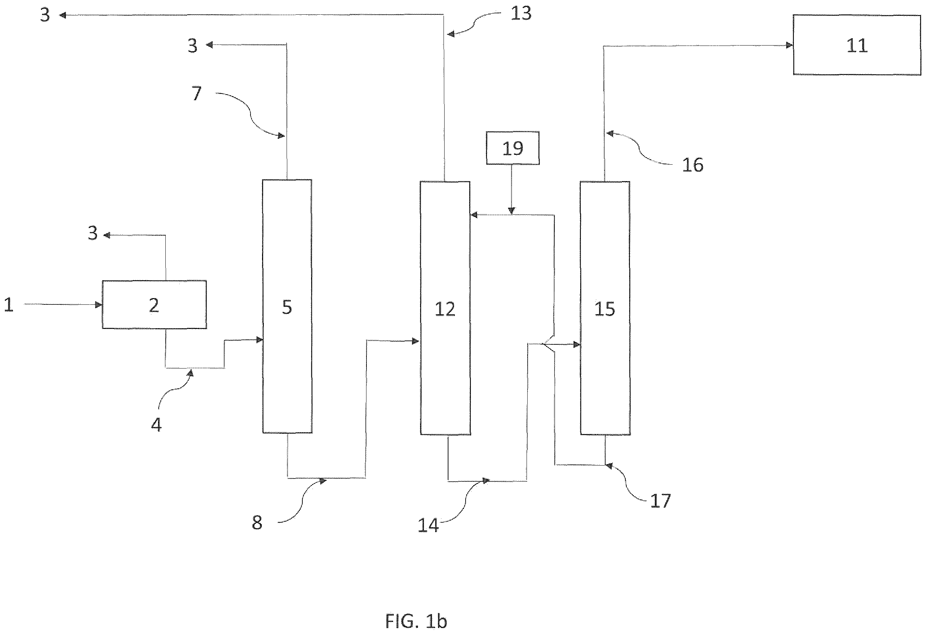

La figure 1b représente schématiquement un dispositif mettant en oeuvre un procédé de purification du 2-chloro-3,3,3-trifluoropropène (1233xf) à partir d'une composition qui ne comprend pas d'impuretés lourdes. Dans ce cas particulier, le courant récupéré en bas de colonne de distillation 5 comprend 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa). Ce courant est alors directement acheminé vers la colonne de distillation extractive 12 via le conduit 8.

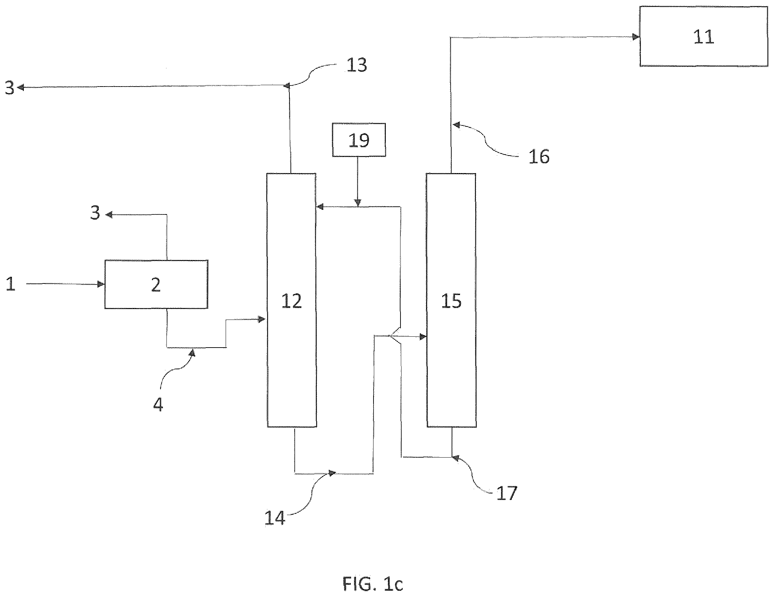

Si la phase organique inférieure récupéré en 2 comprend 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa) mais est dépourvu de 1,1,1,2,2-pentafluoropropane (245cb) et 1,3,3,3-tetrafluoro-1-propene (1234ze), alors la phase organique inférieure est acheminée vers la colonne de distillation extractive 12 via le conduit 4 comme illustré à la figure 1c.

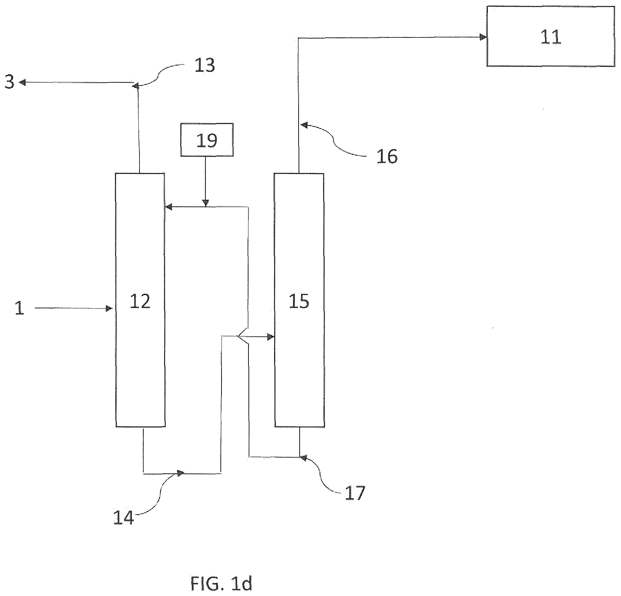

Lorsque la composition acheminée en 1 comprend essentiellement 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa), celle-ci peut être directement soumis à la distillation extractive comme illustré à la fig. 1d en présence d'agent d'extraction organique 19. La composition est ainsi transférée vers la colonne de distillation extractive 12 pour séparer et récupérer le 2-chloro-3,3,3-trifluoropropène (1233xf) en tête de la colonne de distillation 12, pour être recyclé vers 3 via le conduit 13. L'agent d'extraction organique 19 et ledit au moins un des composés choisi parmi le groupe consistant en E-1-chloro-3,3,3-trifluoro-1-propene (1233zdE) et 1,1,1,3,3-pentafluoropropane (245fa) sont traités comme expliqué en relation avec la Fig. 1a.

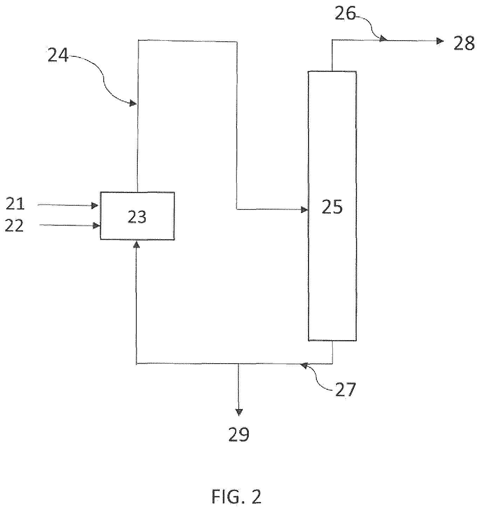

La Fig. 2 illustre schématiquement un dispositif mettant en oeuvre un procédé de production du 2,3,3,3-tetrafluoropropène selon un mode de réalisation particulier de la présente invention. L'acide fluorhydrique 21 est mis en contact avec du 1,1,1,2,3-pentachloropropane (240db) 22 dans un réacteur 23. Un courant comprenant HF, 2,3,3,3-tetrafluoropropène, HCl, 2-chloro-3,3,3-trifluoropropène (1233xf), au moins un des composés sélectionné parmi le groupe consistant en E-1-chloro-3,3,3-trifluoro-1-propene (1233zdE) et 1,1,1,3,3-pentafluoropropane (245fa) est récupéré en sortie de réacteur et acheminé vers une colonne de distillation 25. Le courant peut également comprendre des impuretés lourdes, 1,1,1,2,2-pentafluoropropane (245cb) et 1,3,3,3-tetrafluoro-1-propene (1234ze). Un courant comprenant HCl et 2,3,3,3-tetrafluoropropène est récupéré en tête de colonne de distillation et acheminé via le conduit 26 vers un dispositif de purification 28 apte à séparer HCl et 2,3,3,3-tetrafluoropropène, par exemple une colonne de distillation. Une partie du courant obtenu en bas de colonne de distillation, de préférence moins de 50% en poids sur base du poids total du courant obtenu en bas de colonne de distillation, est acheminé vers le dispositif de purification 29 du 2-chloro-3,3,3-trifluoro-1-propene (1233xf) via le conduit 27. Le dispositif de purification 29 peut être l'un quelconque des dispositifs illustrés aux Fig. 1a-1d.

Le catalyseur utilisé dans le présent procédé de production de 2,3,3,3-tétrafluoropropène peut être par exemple à base d'un métal comprenant un oxyde de métal de transition ou un dérivé ou un halogénure ou un oxyhalogénure d'un tel métal. On peut citer par exemple $FeCl_3$, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple $AlF_3$ et $Al_2O_3$, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, l.1-5 et 28-32), au document EP 939071 (paragraphe [0022]), au document WO 2008/054781 (en p.9 l.22-p.10 l.34), et au document WO 2008/040969 (revendication 1), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de nickel.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, l.30-p.7 l.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :

- avec un rapport molaire HF / composé de formule (I) et/ou (II) de 3:1 à 150:1, de préférence de 4:1 à 125:1 et de manière plus particulièrement préférée de 5:1 à 100:1 ;
- avec un temps de contact de 3 à 100 s, de préférence 4 à 75 s et plus particulièrement 5 à 50 s (volume de catalyseur divisé par le flux entrant total, ajusté à la température et à la pression de fonctionnement) ;
- à une pression allant de la pression atmosphérique à 20 bar, de préférence de 2 à 18 bar et plus particulièrement de 3 à 15 bars;
- à une température (température du lit de catalyseur) de 200 à 450°C, de préférence de 250 à 400°C, et plus particulièrement de 280 à 380°C.

La durée de l'étape de réaction est typiquement de 10 à 8000 heures, de préférence de 50 à 5000 heures et de manière plus particulièrement préférée de 70 à 1000 heures.

Un agent oxydant, de préférence l'oxygène, peut éventuellement être ajouté lors de la réaction de fluoration. Le rapport molaire oxygène / composés organiques peut être de 0,005 à 2, de préférence de 0,01 à 1,5. L'oxygène peut être introduit pur ou sous forme d'air ou de mélange oxygène / azote. On peut également remplacer l'oxygène par du chlore.

**Méthode de sélection de l'agent d'extraction organique**

La sélection de l'agent d'extraction organique est déterminée par l'utilisation du modèle Cosmo-RS implémenté dans le logiciel COSMOTHERM. Pour ce couple binaire sélectionné, un facteur de séparation est calculé pour chacun des solvants étudiés par l'équation suivante :

$$S_{1,2} = (\gamma_{1,S} * P1)/(\gamma_{2,S} * P2)$$

dans laquelle

$\gamma_{1,S}$ représente le coefficient d'activité du premier composé 1 dans l'agent d'extraction organique considéré à dilution infinie,

P1 représente la pression de vapeur saturante du premier composé 1,

$\gamma_{2,S}$ représente le coefficient d'activité du second composé 2 du couple binaire dans l'agent d'extraction organique considéré à dilution infinie,

P2 représente la pression de vapeur saturante du second composé 2.

Une capacité d'absorption est également calculée pour chacun des solvants étudiés et pour un couple binaire (1,2) considéré. La capacité d'absorption est calculée par la formule $C_{2,S} = 1/(\gamma_{2,S})$ dans laquelle $\gamma_{2,S}$ représente le coefficient d'activité du second composé du couple binaire considéré dans ledit agent d'extraction organique étudié à dilution infinie.

Les calculs sont répétés pour chaque agent d'extraction organique étudié. Des valeurs minimales de facteur de séparation et de capacité d'absorption sont identifiées afin de permettre une séparation suffisante entre le premier composé et le second composé du couple binaire (1,2) considéré. La pression de vapeur saturante est considérée pour une température de 25°C.

## Exemples

### Exemple 1

La séparation entre le 2-chloro-3,3,3-trifluoropropene (1233xf) et le 1,1,1,3,3-pentafluoropropane (245fa) est considérée. Sur base des informations obtenues par le modèle Cosmo-RS, les solvants repris dans le tableau 1 ci-dessous ont été testés pour la distillation extractive d'un mélange comprenant 2-chloro-3,3,3-trifluoropropene (1233xf) et le 1,1,1,3,3-pentafluoropropane (245fa).

Tableau 1- Capacité et Facteur de séparation de l'agent d'extraction organique

| Agent extraction organique | Capacité d'absorption | Facteur de séparation |
|---|---|---|
| Propanone | 1,83 | 2,45 |
| Methylacétate | 2,12 | 2,35 |
| Ethylacétate | 2,17 | 1,89 |
| Butanone | 1,72 | 1,77 |
| Dioxane | 1,69 | 1,62 |
| Triméthoxyméthane | 2,07 | 1,83 |
| 1,3-dioxane | 1,83 | 1,76 |
| 1,3,5-trioxane | 1,04 | 2,58 |
| 1,2-diaminoéthane | 1,87 | 2,41 |
| 1-methoxy-2-propanol | 1,22 | 1,68 |

### Exemple 2

La séparation entre le 2-chloro-3,3,3-trifluoropropene (1233xf) et le E-1-chloro-3,3,3-trifluoro-1-propene (1233zdE) est considérée. Sur base des informations obtenues par le modèle Cosmo-RS, les solvants repris dans le tableau 2 ci-dessous ont été testés pour la distillation extractive d'une première composition comprenant 2-chloro-3,3,3-trifluoropropene (1233xf) et le E-1-chloro-3,3,3-trifluoro-1-propene (1233zdE).

Tableau 2 - Capacité et Facteur de séparation de l'agent d'extraction organique

| Agent extraction organique | Capacité d'absorption | Facteur de séparation |
|---|---|---|
| Diethylamine | 3,29 | 1,96 |
| Propanone | 1,15 | 1,68 |
| Méthylacétate | 1,42 | 1,70 |
| Tetrahydrofurane | 2,94 | 1,80 |
| Ethylacétate | 1,86 | 1,76 |
| Butanone | 1,46 | 1,63 |
| Diéthoxyméthane | 2,30 | 1,63 |

(suite)

| Agent extraction organique | Capacité d'absorption | Facteur de séparation |
|---|---|---|
| Isopropylacétate | 2,04 | 1,71 |
| Tert-butylacétate | 2,10 | 1,71 |
| Dioxane | 1,78 | 1,85 |
| 3-pentanone | 1,77 | 1,65 |
| 1,1-diethoxyéthane | 2,49 | 1,67 |
| 2-pentanone | 1,70 | 1,63 |
| n-pentylamine | 3,56 | 2,21 |
| 1,3-dioxane | 1,74 | 1,81 |
| Sec-butylacétate | 2,14 | 1,69 |
| 1,2-diaminoéthane | 2,74 | 3,81 |
| 1-methoxy-2-propanol | 2,06 | 1,72 |
| N-butylacétate | 2,08 | 1,72 |
| 1-ethoxy-2-propanol | 1,50 | 1,92 |

**Exemple 3**

La séparation entre d'une part le 2-chloro-3,3,3-trifluoropropene (1233xf) et d'autre part E-1-chloro-3,3,3-trifluoro-1-propene (1233zdE) et 1,1,1,3,3-pentafluoropropane (245fa) est considérée. Sur base des informations obtenues par le modèle Cosmo-RS, les solvants repris dans le tableau 3 ci-dessous ont été testés pour la distillation extractive d'une première composition comprenant 2-chloro-3,3,3-trifluoropropene (1233xf), 1,1,1,3,3-pentafluoropropane (245fa) et le E-1-chloro-3,3,3-trifluoro-1-propene (1233zdE).

Tableau 3 - Capacité et Facteur de séparation de l'agent d'extraction organique

| Agent extraction organique | Capacité d'absorption (par rapport au 245fa) | Facteur de séparation (par rapport au 245fa) | Capacité d'absorption (par rapport au 1233zdE) | Facteur de séparation (par rapport au 1233zdE) |
|---|---|---|---|---|
| Propanone | 1,83 | 2,45 | 1,15 | 1,68 |
| Methylacétate | 2,12 | 2,35 | 1,42 | 1,70 |
| Ethylacétate | 2,17 | 1,89 | 1,86 | 1,76 |
| Butanone | 1,72 | 1,77 | 1,46 | 1,63 |
| Dioxane | 1,69 | 1,62 | 1,78 | 1,85 |
| Trimethoxyméthane | 2,07 | 1,83 | 1,76 | 1,68 |
| 1,3-dioxane | 1,83 | 1,76 | 1,74 | 1,81 |
| 1,2-diaminoethane | 1,87 | 2,41 | 2,74 | 3,81 |
| 1-methoxy-2-propanol | 1,21 | 1,69 | 1,26 | 1,90 |
| 3-methoxy-1-butanol | 1,52 | 1,63 | 1,72 | 1,99 |
| Diacétone alcohol | 1,55 | 1,91 | 1,45 | 1,95 |

Les résultats ont été confirmés à partir d'un mélange comprenant 60 à 90 % en poids de 2-chloro-3,3,3-trifluoropropene (1233xf) 5 à 25 % en poids de 1-chloro-3,3,3-trifluoropropène (1233zd) et 5 à 15 % en poids de 1,1,1,3,3-pentafluoropropane (245fa) sur base du poids total de la composition. Le reste de la composition est formée par l'agent d'extraction organique testé.

**Revendications**

1. Procédé de purification du 2-chloro-3,3,3-trifluoropropène (1233xf) à partir d'une première composition comprenant du 2-chloro-3,3,3-trifluoropropène et 1,1,1,3,3-pentafluoropropane (245fa), ledit procédé comprenant les étapes de:

   a) mise en contact de ladite première composition avec au moins un agent d'extraction organique pour former une seconde composition ;
   b) distillation extractive de ladite seconde composition pour former :

   i) une troisième composition comprenant ledit agent d'extraction organique et 1,1,1,3,3-pentafluoropropane (245fa),
   ii) un courant comprenant le 2-chloro-3,3,3-trifluoropropène;

   c) récupération et séparation de ladite troisième composition pour former un courant comprenant ledit agent d'extraction organique et un courant comprenant 1,1,1,3,3-pentafluoropropane (245fa); **caractérisé en ce que** ledit agent d'extraction organique est sélectionné parmi le groupe consistant en éthanedial, propanone, methylacetate, chloromethoxymethane, isobutanal, methanol, isopropylformate, methylglyoxal, 2,2,2-trifluoroethanol, 1,1,1-trifluoro-2-propanol, ethylacetate, ethanol, butanone, n-propylformate, 2-propanol, acetonitrile, pentafluoro-1-propanol, tert-butanol, 1,2-dimethoxyethane, isopropylacetate, 2-methylbutanal, 2-methoxyethanamine, tert-butylacetate, propionitrile, 2-allyloxyethanol, ethylpropionate, dioxane, 3-pentanone, 2-pentanone, trimethoxymethane, 3,3-dimethyl-2-butanone, 1,3-dioxane, 2,2,3,3-tetraflouro-1-propanol, sec-butylacetate, n-methyl-1,2-ethanediamine, 1,3,5-trioxane, 4-methyl-2-pentanone, 1,2-diaminoethane, butyronitrile, 1-methoxy2-propanol, 1,2-propanediamine, perfluorobutanoicacid, 1-(dimethylamino)-2-propanol, 2-methoxyethanol, 4,4,4-trifluorobutanol, diethylcarbonate, n-butylacetate, 2-hexanone, 3-fluoropropanol, 5-hexen-2-one, 2-methoxy1-propanol, 1-ethoxy-2-propanol, 2-(dimethylamino)-ethanol, ethoxyethanol, 2-ethoxy-1-propanol, 1,3-propanediamine, 3,3,4,4,5,5,6,6-octafluoro-1-pentanol, valeronitrile, (methyleneamino)acetonitrile, 3-furfural, 4-methyl-2-hexanone, 1-methoxy-2-acetoxypropane, methylhexanoate, 2-propoxyethanol, 1-propoxy-2-propanol, dimethylethanolamine, 2-heptanone, dimethylformamide, 2-isopropoxyethanol, cyclohexanone, 2-ethoxyethanolacetate, 3-hydroxy-butanal, 2-(methylamino)-ethanol, 4-methoxy-4-methyl-pentan-2-one, 3-ethoxy-1-propanol, 3-methoxy-1-butanol, furfural, diglyme, 3-methoxypropanenitrile, 2-(ethylamino)ethanol, diacetone alcohol, 1-butoxy-2-propanol, 2-furanmethanol, methylacetoacetate, 2-aminopropanol, n,n-dimethylpropanamide, 2-amino-1-butanol, 2-methyl-2-nitro-1-propanol, 2-propanol-1-methoxy-propanoate, cycloheptanone, dimethylmalonate, 1,1,3,3-tetramethoxypropane, dimethylsulfate, dimethylsulfoxide, 2-butoxyethanolacetate, diethylsulfoxide, 2-(2-methoxyethoxy)ethanol, 1,2-butanediol, diethyleneglycolmonoethylether, trimethylphosphate, 2-methyl-2,4-pentanediol, methylbenzoate, diethylmalonate.

2. Procédé selon la revendication précédente **caractérisé en ce que** ledit agent d'extraction organique est sélectionné parmi le groupe consistant en éthanedial, propanone, methylacetate, methylglyoxal, ethylacetate, butanone, propionitrile, dioxane, trimethoxymethane, 1,3-dioxane, 1,3,5-trioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, methylacetoacetate, n,n-dimethylpropanamide, dimethylmalonate, diethylsulfoxide, 2-(2-methoxyethoxy)ethanol, trimethylphosphate, diethylmalonate ; de préférence ledit agent d'extraction organique est choisi parmi le groupe consistant en propanone, methylacetate, ethylacetate, butanone, dioxane, trimethoxymethane, 1,3-dioxane, 1,3,5-trioxane, 1,2-diaminoethane, 1-methoxy-2-propanol.

3. Procédé de purification du 2-chloro-3,3,3-trifluoropropène (1233xf) à partir d'une première composition comprenant du 2-chloro-3,3,3-trifluoropropène et E-1-chloro-3,3,3-trifluoro-1-propene (1233zdE), ledit procédé comprenant les étapes de:

   a) mise en contact de ladite première composition avec au moins un agent d'extraction organique pour former une seconde composition ;
   b) distillation extractive de ladite seconde composition pour former :

   i) une troisième composition comprenant ledit agent d'extraction organique et E-1-chloro-3,3,3-trifluoro-1-propene (1233zdE),

ii) un courant comprenant le 2-chloro-3,3,3-trifluoropropène;

c) récupération et séparation de ladite troisième composition pour former un courant comprenant ledit agent d'extraction organique et un courant comprenant E-1-chloro-3,3,3-trifluoro-1-propene (1233zdE) ; **caractérisé en ce que** ledit agent d'extraction organique est sélectionné parmi le groupe consistant en isopropylmethylamine, ethanedial, 2-chloro-2-methylpropane, 2-propanethiol, 2-ethoxy-propane, 1-chloro-2,2-difluoropropane, methyl-t-butylether, diethylamine, propanone, methylacetate, 1,1-dichloroethane, 4-methoxy-2-methyl-2-butanethiol, 2-bromopropane, chloromethoxymethane, difluorodiethylsilane, 2-butanamine, n-methylpropylamine, tert-butylthiol, isobutanal, tetrahydrofurane, 1-propanethiol, 2-chlorobutane, isopropylformate, diisopropylether, 1-bromopropane, methylglyoxal, 2-ethoxy-2-methyl-propane, 2-bromo-2-methylpropane, 1-chloro-3-fluoropropane, 1,1,1-trifluoro-2-propanol, 1-butylamine, ethylacetate, 1-chlorobutane, ethanol, butanone, n-propylformate, 2-ethoxy-butane, 2-propanol, acetonitrile, tert-butanol, 1-methoxy-2-methyl-butane, cyclohexene, 2,2-dimethoxypropane, 2-chloro-2-methylbutane, 1,2-dichloroethane, 1-ethoxy-2-methylpropane, diisopropylamine, 2-butanethiol, 1,2-dimethoxyethane, 3-methyl-2-butanamine, diethoxymethane, 1,1-dichloropropane, 2-methyl-1-propanethiol, isopropylacetate, 1,2-dichloro-2-fluoropropane, di-n-propylether,, 3-pentylamine, n-methylbutylamine, 2-bromobutane, diethylsulfide, 1-ethoxybutane, 1-fluorohexane, 1-methoxy-2-propanamine, 2,3-dichloro-1-propene, 2-methylbutanal, 2-methoxyethanamine, 1,2-dichloropropane, propanol, tert-butylacetate, propionitrile, 3-chloropentane, trichloroacetaldehyde, 2-allyloxyethanol, butanethiol, isoamylchloride, 1-methoxy-pentane, ethylpropionate, 2-butanol, 1,2-dimethoxypropane, isopropyl-isobutyl-ether, 1-chloro-4-fluorobutane, 2,4,4-trimethyl-1-pentene, 1-bromo-3-fluoropropane, dioxane, 1-bromobutane, 3-pentanone, 1,1-diethoxyethane, 2-pentanone, 2-methyl-2-butanol, 2-methoxy-1propanamine, trimethoxymethane, 2,2-dichlorobutane, cis-1,3-dichloropropene, n-pentylamine, 3,3-dimethyl-2-butanone, 1,3-dioxane, piperidine, isobutanol, 2-bromo-2-methylbutane, dipropylamine, 2-ethoxyethanamine, triethylfluorosilane, 1-methylcyclohexene, sec-butylacetate, trans-1,3-dichloropropene, 2,2-dimethyl-1-propanol, 1,1,1-trichloro-3-fluoropropane, n-methyl-1,2-ethanediamine, 2,2-diethoxypropane, 1,3,5-trioxane, 1-chloro-3,3-dimethylbutane, pyridine, n-methylmorpholine, 3-pentanol, 4-methyl-2-pentanone, 1,2-diaminoethane, isobutyl-tert-butylether, 2-bromopentane, butyronitrile, 1-butanol, 2,3-dichlorobutane, sec-butyl-tert-butylether, 1-methoxy2-propanol, 1,2-propanediamine, 2,6-dimethyl-5-heptenal, 1-bromo-3-methylbutane, 1,3-dichloro-trans-2-butene, 1,3-dichloropropane, 1-(dimethylamino)-2-propanol, tetrahydrothiophene, 3-methyl-3-pentanol, 1,1-diethoxypropane, 1,2,2-trichloropropane, 1-chloro-2-methyl-2-propanol, 2-methoxyethanol, 1,2-dichlorobutane, 4,4,4-trifluorobutanol, 2-ethylbutylamine, diethylcarbonate, n-butylacetate, 1-pentanethiol, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxy1-propanol, 1,2-dichloro-2-butene, hexanal, 1-ethoxy-2-propanol, 4-methyl-2-pentanol, bromoaceticacidmethylester, 1,2-octanediol, 4-methyl-2-hexanamine, hexylamine, methoxycyclohexane, 2-(dimethylamino)-ethanol, 1,3-dichlorobutane, cyclohexylamine, n-ethyl-2-dimethylaminoethylamine, ethoxyethanol, 3-hexanol, 2-hexanol, 2-methylpyrazine, 2-ethoxy-1-propanol, 1-pentanol, n-ethyl-morpholine, 1-methylpiperazine, 1,4-dimethylbenzene, 1,3-propanediamine, di-n-butylether, valeronitrile, (methyleneamino)acetonitrile, 2-heptanamine, 2,3-dimethylbutanol, 1-ethoxyhexane, 1-chloro-3-bromopropane, n,n-diethylethylenediamine, 3-furfural, 2,6-dimethylpyridine, 4-methyl-2-hexanone, 1,1,1-triethoxyethane, styrene, 1-methoxy-2-acetoxypropane, 4-methylpyridine, n,n'-diethyl-1,2-ethanediamine, 2,6-dimethylmorpholine, 2-ethyl-1-butanol, 1,2-dichloropentane, 2-methyl-1-pentanol, methylhexanoate, 2-propoxyethanol, 1-propoxy-2-propanol, dimethylethanolamine, isopropylchloroacetate, 2-heptanone, 1-hexanethiol, 1,2-propanedithiol, heptanal, dimethylformamide, 2,6-dimethylpyrazine, 2-isopropoxyethanol, diethyldisulfide, 2-methylpiperazine, 1-methylcyclohexanol, 1-bromohexane, cyclohexanone, 1-heptanamine, 2,3-dimethylpyrazine, 2-ethoxyethanolacetate, 3-hydroxy-butanal, 1-hexanol, 2-(methylamino)-ethanol, 1,4-butanediamine, 2,4-dimethylpyridine, 2-pyrimidinamine, 2-heptanol, 2-methoxy-3-methylpyrazine, dibutylamine, 4-methoxy-4-methyl-pentan-2-one, 1,4-dichloro-trans-2-butene, 3-ethoxy-1-propanol, cyclohexanol, 1,4-dichlorobutane, 3-methoxy-1-butanol, furfural, diglyme, 1,1,1-trichloro-2-propanol, 2-(diethylamino)-ethanol, 3-methoxypropanenitrile, 2,2-diethoxyethanamine, 1,3,5-trimethylbenzene, 2-methoxy-n-(2-methoxyethyl)ethanamine, 2-chloropyridine, bromocyclohexane, 2,3-heptanedione, 2-(ethylamino)ethanol, 3-methylcyclohexanol, 1,3-dibromopropane, 2-methylcyclohexanol, 3-octanone, diacetone alcohol, diethylaminopropylamine, 2-ethylhexylamine, 1,3-propanedithiol, ethoxybenzene, 1-butoxy-2-propanol, 2-furanmethanol, 2-butoxyethanol, methylacetoacetate, 2-octanone, 2-aminopropanol, 1,4-dichlorobenzene, methylheptanoate, triethylenediamine, n,n-dimethylpropanamide, 2-chlorophenol, 2-amino-1-butanol, 1-heptanol, 2-methyl-2-nitro-1-propanol, 2-propanol-1-methoxy-propanoate, 1,5-pentanediamine, 2-octanol, cycloheptanone, 3,4-dimethylpyridine, 1-octanamine, benzylmethylamine, dimethylmalonate, 1-chlorooctane, cyclohexanemethanol, 1,1,3,3-tetramethoxypropane, 2-(ethylthio)-ethanol, 1-ethoxy-2-methylbenzene, aniline, 2-ethyl-1-hexanol, tert-butylcyclohexylamine, dihexylphthalate, 1-fluorodecane, 2-chloro-1,4-dimethylbenzene, dimethylsulfate, dimethylsulfoxide, diethylpropanolamine, 2-methylphenol, 2-butoxyethanolacetate, diethylsulfoxide, 1-octanol, 2-bromopyridine, 2-(2-methoxyethoxy)ethanol, 1,2-butanediol, 2-bromophenol, 4-methylbenzenemethanamine, diethyleneglycolmonoethylether, 1,2,4,5-tetramethylbenzene, 2-propylcyclohexanone, 1,4-dibromobutane, trimethylphosphate, 2-methyl-2,4-pentanediol, 1,3-diethenylbenzene, methylbenzoate, 1-octanethiol, diethylmalonate, 2-methoxypyrimidine.

4. Procédé selon la revendication précédente **caractérisé en ce que** ledit agent d'extraction organique est sélectionné parmi le groupe consistant en isopropylmethylamine, methyl-t-butylether, diethylamine, propanone, methylacetate, 2-butanamine, n-methylpropylamine, tetrahydrofurane, 1-butylamine, ethylacetate, butanone, n-propylformate, dimethoxypropane, diisopropylamine, 1,2-dimethoxyethane, 3-methyl-2-butanamine, diethoxymethane, isopropylacetate, 3-pentylamine, n-methylbutylamine, 1-methoxy-2-propanamine, 2-methoxyethanamine, tert-butylacetate, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 1,2-diaminoethane, 1-methoxy2-propanol, 1,2-propanediamine, 2,6-dimethyl-5-heptenal, 1-(dimethylamino)-2-propanol, 3-methyl-3-pentanol, 2-ethylbutylamine, diethylcarbonate, n-butylacetate, 2-hexanone, n-ethylethylenediamine, 2-methoxy1-propanol, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-ethoxy-1-propanol, 1-methylpiperazine, 1,3-propanediamine, 2-heptanamine, n,n-diethylethylenediamine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol, 2-heptanone, dimethylformamide, 2-isopropoxyethanol, 2-methylpiperazine, cyclohexanone, 1-heptanamine, 2-ethoxyethanolacetate, 1,4-butanediamine, 2,4-dimethylpyridine, 2-methoxy-3-methylpyrazine, 4-methoxy-4-methyl-pentan-2-one, 3-ethoxy-1-propanol, 3-methoxy-1-butanol, diglyme, 2-(diethylamino)-ethanol, 2,2-diethoxyethanamine, 2-methoxy-n-(2-methoxyethyl)ethanamine, 2-(ethylamino)ethanol, 3-octanone, diacetone alcohol, diethylaminopropylamine, 2-ethylhexylamine, 1-butoxy-2-propanol, 2-butoxyethanol, 2-octanone, methylheptanoate, triethylenediamine, n,n-dimethylpropanamide, 2-propanol-1-methoxy-propanoate, 1,5-pentanediamine, cycloheptanone, 3,4-dimethylpyridine, 1-octanamine, benzylmethylamine, 1,1,3,3-tetramethoxypropane, dihexylphthalate, diethylpropanolamine, 2-butoxyethanolacetate, diethylsulfoxide, 2-(2-methoxyethoxy)ethanol, 4-methylbenzenemethanamine, diethyleneglycolmonoethylether, 2-propylcyclohexanone, trimethylphosphate, 2-methyl-2,4-pentanediol, methylbenzoate, diethylmalonate, 2-methoxypyrimidine ; de préférence ledit agent d'extraction organique est choisi parmi le groupe consistant en diethylamine, propanone, methylacetate, tetrahydrofurane, ethylacetate, butanone, diethoxymethane, isopropylacetate, tert-butylacetate, dioxane, 3-pentanone, 1,1-diethoxyethane, 2-pentanone, n-pentylamine, 1,3-dioxane, sec-butylacetate, 1,2-diaminoethane, 1-methoxy2-propanol, n-butylacetate, 1-ethoxy-2-propanol.

5. Procédé selon l'une quelconque des revendications précédentes **caractérisé en ce que** le courant comprenant l'agent d'extraction organique séparé à l'étape c) est recyclé à l'étape a).

6. Procédé selon l'une quelconque des revendications précédentes **caractérisé en ce que** ledit courant comprenant le 2-chloro-3,3,3-trifluoropropène formé à l'étape b) est récupéré en tête de colonne de distillation et recyclé optionnellement dans un procédé de production du 2,3,3,3-tétrafluoropropène.

7. Procédé selon l'une quelconque des revendications précédentes **caractérisé en ce que** ledit agent d'extraction organique est choisi parmi le groupe consistant en propanone, methylacetate, ethylacetate, butanone, dioxane, trimethoxymethane, 1,3-dioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, n,n-dimethylpropanamide, diethylsulfoxide, 2-(2-methoxyethoxy)ethanol, trimethylphosphate, diethylmalonate ; de préférence ledit agent d'extraction organique est choisi parmi le groupe consistant propanone, methylacetate, ethylacetate, butanone, dioxane, trimethoxymethane, 1,3-dioxane, 1,2-diaminoethane, 1-methoxy2-propanol, 3-methoxy-1-butanol, diacetone alcohol.

8. Procédé selon l'une quelconque des revendications précédentes **caractérisé en ce que** la première composition est une composition azéotropique ou quasi-azéotropique comprenant 2-chloro-3,3,3-trifluoropropène et E-1-chloro-3,3,3-trifluoro-1-propene (1233zdE) ; ou la première composition est une composition azéotropique ou quasi-azéotropique comprenant 2-chloro-3,3,3-trifluoropropène et 1,1,1,3,3-pentafluoropropane (245fa).

9. Procédé de production de 2,3,3,3-tetrafluoro-1-propène comprenant les étapes de :

A) dans un réacteur, fluoration en présence d'un catalyseur d'un composé de formule (I) CX(Y)$_2$-CX(Y)$_m$-CH$_m$XY dans laquelle X et Y représentent indépendamment un atome d'hydrogène, de fluor ou de chlore et m = 0 ou 1 ; et/ou fluoration catalytique en présence d'un catalyseur d'un composé de formule (CX$_n$Y$_{3-n}$)CH$_p$X$_{1-p}$CH$_m$X$_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant issu d'une purge de la boucle réactionnelle de recyclage comprenant 1,1,1,2,2-pentafluoropropane, 2-chloro-3,3,3-trifluoropropène, et au moins un des composés choisis parmi le groupe consistant en E-1-chloro-3,3,3-trifluoro-1-propene (1233zdE) et 1,1,1,3,3-pentafluoropropane (245fa);

C) distillation du courant récupéré à l'étape B) et récupération en tête de colonne de distillation d'un courant comprenant du 1,1,1,2,2-pentafluoropropane et en bas de colonne de distillation d'un courant comprenant du 2-chloro-3,3,3-trifluoropropène (1233xf) et au moins un des composés choisis parmi le groupe consistant en E-1-chloro-3,3,3-trifluoro-1-propene (1233zdE) et 1,1,1,3,3-pentafluoropropane (245fa);

D) mise en oeuvre du procédé de purification du 2-chloro-3,3,3-trifluoropropène selon l'une quelconque des revendications 1 à 8 à partir du courant récupéré à l'étape C) et comprenant du 2-chloro-3,3,3-trifluoropropène (1233xf) et au moins un des composés choisis parmi le groupe consistant en E-1-chloro-3,3,3-trifluoro-1-propene (1233zdE) et 1,1,1,3,3-pentafluoropropane (245fa); et

E) recyclage à l'étape A) du courant comprenant le 2-chloro-3,3,3-trifluoropropène formé et récupéré à l'étape b) du procédé de purification mis en oeuvre à l'étape D).

10. Procédé selon la revendication précédente **caractérisé en ce que** le courant récupéré à l'étape B) comprenant également HF, 1,1,1,2,2-pentafluoropropane et 1,3,3,3-tetrafluoro-1-propene (1234ze), ceux-ci étant, préalablement à la mise en oeuvre de la distillation de l'étape C), traités selon les étapes suivantes :

i) séparation à basse température de ladite composition liquide pour former une première phase riche en HF et une seconde phase organique contenant 1,1,1,2,2-pentafluoropropane et 1,3,3,3-tetrafluoro-1-propene (1234ze), 2-chloro-3,3,3-trifluoropropène (1233xf) et au moins un des composés choisis parmi le groupe consistant en E-1-chloro-3,3,3-trifluoro-1-propene (1233zdE) et 1,1,1,3,3-pentafluoropropane (245fa);

ii) distillation de ladite seconde phase organique pour former et récupérer, avantageusement en tête de colonne de distillation, un premier courant contenant 1,1,1,2,2-pentafluoropropane et 1,3,3,3-tetrafluoro-1-propene (1234ze), et pour former et récupérer, avantageusement en bas de colonne de distillation, un second courant comprenant du 2-chloro-3,3,3-trifluoropropène (1233xf) et au moins un des composés choisi parmi le groupe consistant en E-1-chloro-3,3,3-trifluoro-1-propene (1233zdE) et 1,1,1,3,3-pentafluoropropane (245fa);

iii) récupération dudit second courant et mise en oeuvre de l'étape D) à partir de celui-ci.

11. Composition comprenant du 2-chloro-3,3,3-trifluoropropène (1233xf), 1,1,1,3,3-pentafluoropropane (245fa) et un agent d'extraction organique sélectionné parmi le groupe consistant en éthanedial, propanone, methylacetate, methylglyoxal, ethylacetate, butanone, propionitrile, dioxane, trimethoxymethane, 1,3-dioxane, 1,3,5-trioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, methylacetoacetate, n,n-dimethylpropanamide, dimethylmalonate, diethylsulfoxide, 2-(2-methoxyethoxy)ethanol, trimethyl phosphate, diethylmalonate ; en particulier ledit agent d'extraction organique est sélectionné parmi le groupe consistant en propanone, methylacetate, ethylacetate, butanone, dioxane, trimethoxymethane, 1,3-dioxane, 1,3,5-trioxane, 1,2-diaminoethane, 1-methoxy-2-propanol.

12. Composition comprenant du 2-chloro-3,3,3-trifluoropropène (1233xf), E-1-chloro-3,3,3-trifluoro-1-propene (1233zdE) et un agent d'extraction organique sélectionné parmi le groupe consistant en isopropylmethylamine, methyl-t-butylether, diethylamine, propanone, methylacetate, 2-butanamine, n-methylpropylamine, tetrahydrofurane, 1-butylamine, ethylacetate, butanone, n-propylformate, -dimethoxypropane, diisopropylamine, 1,2-dimethoxyethane, 3-methyl-2-butanamine, diethoxymethane, isopropylacetate, 3-pentylamine, n-methylbutylamine, 1-methoxy-2-propanamine, 2-methoxyethanamine, tert-butylacetate, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 1,2-diaminoethane, 1-methoxy2-propanol, 1,2-propanediamine, 2,6-dimethyl-5-heptenal, 1-(dimethylamino)-2-propanol, 3-methyl-3-pentanol, 2-ethylbutylamine, diethylcarbonate, n-butylacetate, 2-hexanone, n-ethylethylenediamine, 2-methoxy1-propanol, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-ethoxy-1-propanol, 1-methylpiperazine, 1,3-propanediamine, 2-heptanamine, n,n-diethylethylenediamine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol, 2-heptanone, dimethylformamide, 2-isopropoxyethanol, 2-methylpiperazine, cyclohexanone, 1-heptanamine, 2-ethoxyethanolacetate, 1,4-butanediamine, 2,4-dimethylpyridine, 2-methoxy-3-methylpyrazine, 4-methoxy-4-methyl-pentan-2-one, 3-ethoxy-1-propanol, 3-methoxy-1-butanol, diglyme, 2-(diethylamino)-ethanol, 2,2-diethoxyethanamine, 2-methoxy-n-(2-methoxyethyl)ethanamine, 2-(ethylamino)ethanol, 3-octanone, diacetone alcohol, diethylaminopropylamine, 2-ethylhexylamine, 1-butoxy-2-propanol, 2-butoxyethanol, 2-octanone, methylheptanoate, triethylenediamine, n,n-dimethylpropanamide, 2-propanol-1-methoxy-propanoate, 1,5-pentanediamine, cycloheptanone, 3,4-dimethylpyridine, 1-octanamine, benzylmethylamine, 1,1,3,3-tetramethoxypropane, dihexylphthalate, diethylpropanolamine, 2-butoxyethanolacetate, diethylsulfoxide, 2-(2-methoxyethoxy)ethanol, 4-methylbenzenemethanamine, diethyleneglycolmonoethylether, 2-propylcyclohexanone, trimethylphosphate, 2-methyl-2,4-pentanediol, methylbenzoate, diethylmalonate, 2-methoxypyrimidine ; en particulier ledit agent d'extraction organique est choisi parmi le groupe consistant en diethylamine, propanone, methylacetate, tetrahydrofurane, ethylacetate, butanone, diethoxymethane, isopropylacetate, tert-butylacetate, dioxane, 3-pentanone, 1,1-diethoxyethane, 2-pentanone, n-pentylamine, 1,3-dioxane, sec-butylacetate, 1,2-diaminoethane, 1-methoxy2-propanol, n-butylacetate, 1-ethoxy-2-propanol.

## Patentansprüche

1. Verfahren zur Reinigung von 2-Chlor-3,3,3-trifluorpropen (1233xf) ausgehend von einer ersten Zusammensetzung, die 2-Chlor-3,3,3-trifluorpropen und 1,1,1,3,3-Pentafluorpropan (245fa) umfasst, wobei das Verfahren die folgenden Schritte umfasst:

a) Inkontaktbringen der ersten Zusammensetzung mit mindestens einem organischen Extraktionsmittel unter Herstellung einer zweiten Zusammensetzung;
b) extraktive Destillation der zweiten Zusammensetzung unter Herstellung:

i) einer dritten Zusammensetzung, die das organische Extraktionsmittel und 1,1,1,3,3-Pentafluorpropan (245fa) umfasst,
ii) eines Stroms, der 2-Chlor-3,3,3-trifluorpropen umfasst;

c) Gewinnung und Trennung der dritten Zusammensetzung unter Herstellung eines Stroms, der das organische Extraktionsmittel umfasst, und eines Stroms, der 1,1,1,3,3-Pentafluorpropan (245fa) umfasst, **dadurch gekennzeichnet, dass** das organische Extraktionsmittel aus der aus Ethandial, Propanon, Methylacetat, Chlormethoxymethan, Isobutanal, Methanol, Isopropylformiat, Methylglyoxal, 2,2,2-Trifluorethanol, 1,1,1-Trifluor-2-propanol, Ethylacetat, Ethanol, Butanon, n-Propylformiat, 2-Propanol, Acetonitril, Pentafluor-1-propanol, tert-Butanol, 1,2-Dimethoxyethan, Isopropylacetat, 2-Methylbutanal, 2-Methoxyethanamin, tert-Butylacetat, Propionitril, 2-Allyloxyethanol, Ethylpropionat, Dioxan, 3-Pentanon, 2-Pentanon, Trimethoxymethan, 3,3-Dimethyl-2-butanon, 1,3-Dioxan, 2,2,3,3-Tetrafluor-1-propanol, sek-Butylacetat, N-Methyl-1,2-ethandiamin, 1,3,5-Trioxan, 4-Methyl-2-pentanon, 1,2-Diaminoethan, Butyronitril, 1-Methoxy-2-propanol, 1,2-Propandiamin, Perfluorbuttersäure, 1-(Dimethylamino)-2-propanol, 2-Methoxyethanol, 4,4,4-Trifluorbutanol, Diethylcarbonat, n-Butylacetat, 2-Hexanon, 3-Fluorpropanol, 5-Hexen-2-on, 2-Methoxy-1-propanol, 1-Ethoxy-2-propanol, 2-(Dimethylamino)ethanol, Ethoxyethanol, 2-Ethoxy-1-propanol, 1,3-Propandiamin, 3,3,4,4,5,5,6,6-Octafluor-1-pentanol, Valeronitril, (Methylenamino)acetonitril, 3-Furfural, 4-Methyl-2-hexanon, 1-Methoxy-2-acetoxypropan, Methylhexanoat, 2-Propoxyethanol, 1-Propoxy-2-propanol, Dimethylethanolamin, 2-Heptanon, Dimethylformamid, 2-Isopropoxyethanol, Cyclohexanon, 2-Ethoxyethanolacetat, 3-Hydroxybutanal, 2-(Methylamino)ethanol, 4-Methoxy-4-methylpentan-2-on, 3-Ethoxy-1-propanol, 3-Methoxy-1-butanol, Furfural, Diglym, 3-Methoxypropannitril, 2-(Ethylamino)ethanol, Diacetonalkohol, 1-Butoxy-2-propanol, 2-Furanmethanol, Methylacetoacetat, 2-Aminopropanol, N,N-Dimethylpropanamid, 2-Amino-1-butanol, 2-Methyl-2-nitro-1-propanol, 2-Propanol-1-methoxypropanoat, Cycloheptanon, Dimethylmalonat, 1,1,3,3-Tetramethoxypropan, Dimethylsulfat, Dimethylsulfoxid, 2-Butoxyethanolacetat, Diethylsulfoxid, 2-(2-Methoxyethoxy)ethanol, 1,2-Butandiol, Diethylenglykolmonoethylether, Trimethylphosphat, 2-Methyl-2,4-pentandiol, Methylbenzoat, Diethylmalonat bestehenden Gruppe ausgewählt ist.

2. Verfahren nach dem vorhergehenden Anspruch, **dadurch gekennzeichnet, dass** das organische Extraktionsmittel aus der aus Ethandial, Propanon, Methylacetat, Methylglyoxal, Ethylacetat, Butanon, Propionitril, Dioxan, Trimethoxymethan, 1,3-Dioxan, 1,3,5-Trioxan, 1,2-Diaminoethan, 1-Methoxy-2-propanol, Diethylcarbonat, 2-Methoxy-1-propanol, 1-Methoxy-2-acetoxypropan, Dimethylformamid, 3-Methoxy-1-butanol, Diacetonalkohol, Methylacetoacetat, N,N-Dimethylpropanamid, Dimethylmalonat, Diethylsulfoxid, 2-(2-Methoxyethoxy)ethanol, Trimethylphosphat, Diethylmalonat bestehenden Gruppe ausgewählt ist; vorzugsweise ist das organische Extraktionsmittel aus der aus Propanon, Methylacetat, Ethylacetat, Butanon, Dioxan, Trimethoxymethan, 1,3-Dioxan, 1,3,5-Trioxan, 1,2-Diaminoethan, 1-Methoxy-2-propanol bestehenden Gruppe ausgewählt.

3. Verfahren zur Reinigung von 2-Chlor-3,3,3-trifluorpropen (1233xf) ausgehend von einer ersten Zusammensetzung, die 2-Chlor-3,3,3-trifluorpropen und E-1-Chlor-3,3,3-trifluor-1-propen (1233zdE) umfasst, wobei das Verfahren die folgenden Schritte umfasst:

a) Inkontaktbringen der ersten Zusammensetzung mit mindestens einem organischen Extraktionsmittel unter Herstellung einer zweiten Zusammensetzung;
b) extraktive Destillation der zweiten Zusammensetzung unter Herstellung:

i) einer dritten Zusammensetzung, die das organische Extraktionsmittel und E-1-Chlor-3,3,3-trifluor-1-propen (1233zdE) umfasst,
ii) eines Stroms, der 2-Chlor-3,3,3-trifluorpropen umfasst;

c) Gewinnung und Trennung der dritten Zusammensetzung unter Herstellung eines Stroms, der das organische Extraktionsmittel umfasst, und eines Stroms, der E-1-Chlor-3,3,3-trifluor-1-propen (1233zdE) umfasst, **dadurch gekennzeichnet, dass** das organische Extraktionsmittel aus der aus Isopropylmethylamin, Ethandial, 2-Chlor-2-methylpropan, 2-Propanthiol, 2-Ethoxypropan, 1-Chlor-2,2-difluorpropan, Methyl-t-butylether, Diethylamin, Propanon, Methylacetat, 1,1-Dichlorethan, 4-Methoxy-2-methyl-2-butanthiol, 2-Brompropan, Chlormethoxymethan, Difluordiethylsilan, 2-Butanamin, N-Methylpropylamin, tert-Butylthiol, Isobutanal, Tetrahydrofuran, 1-Propanthiol, 2-Chlorbutan, Isopropylformiat, Diisopropylether, 1-Brompropan, Methylglyoxal, 2-Ethoxy-2-methylpropan, 2-Brom-2-methylpropan, 1-Chlor-3-fluorpropan, 1,1,1-Trifluor-2-propanol, 1-Butylamin, Ethylacetat, 1-Chlorbutan, Ethanol, Butanon, n-Propylformiat, 2-Ethoxybutan, 2-Propanol, Acetonitril, tert-Butanol, 1-Methoxy-2-methylbutan, Cyclohexen, 2,2-Dimethoxypropan, 2-Chlor-2-methylbutan, 1,2-Dichlorethan, 1-Ethoxy-2-methylpropan, Diisopropylamin, 2-Butanthiol, 1,2-Dimethoxyethan, 3-Methyl-2-butanamin, Diethoxymethan, 1,1-Dichlorpropan, 2-Methyl-1-propanthiol, Isopropylacetat, 1,2-Dichlor-2-fluorpropan, Di-n-propylether, 3-Pentylamin, N-Methylbutylamin, 2-Brombutan, Diethylsulfid, 1-Ethoxybutan, 1-Fluorhexan, 1-Methoxy-2-propanamin, 2,3-Dichlor-1-propen, 2-Methylbutanal, 2-Methoxyethanamin, 1,2-Dichlorpropan, Propanol, tert-Butylacetat, Propionitril, 3-Chlorpentan, Trichloracetaldehyd, 2-Allyloxyethanol, Butanthiol, Isoamylchlorid, 1-Methoxypentan, Ethylpropionat, 2-Butanol, 1,2-Dimethoxypropan, Isopropylisobutylether, 1-Chlor-4-fluorbutan, 2,4,4-Trimethyl-1-penten, 1-Brom-3-fluorpropan, Dioxan, 1-Brombutan, 3-Pentanon, 1,1-Diethoxyethan, 2-Pentanon, 2-Methyl-2-butanol, 2-Methoxy-1-propanamin, Trimethoxymethan, 2,2-Dichlorbutan, cis-1,3-Dichlorpropen, n-Pentylamin, 3,3-Dimethyl-2-butanon, 1,3-Dioxan, Piperidin, Isobutanol, 2-Brom-2-methylbutan, Dipropylamin, 2-Ethoxyethanamin, Triethylfluorsilan, 1-Methylcyclohexen, sek-Butylacetat, trans-1,3-Dichlorpropen, 2,2-Dimethyl-1-propanol, 1,1,1-Trichlor-3-fluorpropan, N-Methyl-1,2-ethandiamin, 2,2-Diethoxypropan, 1,3,5-Trioxan, 1-Chlor-3,3-dimethylbutan, Pyridin, N-Methylmorpholin, 3-Pentanol, 4-Methyl-2-pentanon, 1,2-Diaminoethan, Isobutyl-tert-butylether, 2-Brompentan, Butyronitril, 1-Butanol, 2,3-Dichlorbutan, sek-Butyl-tert-butylether, 1-Methoxy-2-propanol, 1,2-Propandiamin, 2,6-Dimethyl-5-heptenal, 1-Brom-3-methylbutan, 1,3-Dichlor-trans-2-buten, 1,3-Dichlorpropan, 1-(Dimethylamino)-2-propanol, Tetrahydrothiophen, 3-Methyl-3-pentanol, 1,1-Diethoxypropan, 1,2,2-Trichlorpropan, 1-Chlor-2-methyl-2-propanol, 2-Methoxyethanol, 1,2-Dichlorbutan, 4,4,4-Trifluorbutanol, 2-Ethylbutylamin, Diethylcarbonat, n-Butylacetat, 1-Pentanthiol, 2-Chlor-1,1-dimethoxyethan, 2-Hexanon, N-Ethylethylendiamin, 3-Fluorpropanol, 5-Hexen-2-on, 2,3-Dichlor-2-methylbutan, 1,1-Diethoxy-N,N-dimethylmethanamin, 2-Methylpyridin, 1-Brompentan, 2-Methoxy-1-propanol, 1,2-Dichlor-2-buten, Hexanal, 1-Ethoxy-2-propanol, 4-Methyl-2-pentanol, Bromessigsäuremethylester, 1,2-Octandiol, 4-Methyl-2-hexanamin, Hexylamin, Methoxycyclohexan, 2-(Dimethylamino)ethanol, 1,3-Dichlorbutan, Cyclohexylamin, N-Ethyl-2-dimethylaminoethylamin, Ethoxyethanol, 3-Hexanol, 2-Hexanol, 2-Methylpyrazin, 2-Ethoxy-1-propanol, 1-Pentanol, N-Ethylmorpholin, 1-Methylpiperazin, 1,4-Dimethylbenzol, 1,3-Propandiamin, Di-n-Butylether, Valeronitril, (Methylenamino)acetonitril, 2-Heptanamin, 2,3-Dimethylbutanol, 1-Ethoxyhexan, 1-Chlor-3-brompropan, N,N-Diethylethylendiamin, 3-Furfural, 2,6-Dimethylpyridin, 4-Methyl-2-hexanon, 1,1,1-Triethoxyethan, Styrol, 1-Methoxy-2-acetoxypropan, 4-Methylpyridin, N,N'-Diethyl-1,2-ethandiamin, 2,6-Dimethylmorpholin, 2-Ethyl-1-butanol 1,2-Dichlorpentan, 2-Methyl-1-pentanol, Methylhexanoat, 2-Propoxyethanol, 1-Propoxy-2-propanol, Dimethylethanolamin, Isopropylchloracetat, 2-Heptanon, 1-Hexanthiol, 1,2-Propandithiol, Heptanal, Dimethylformamid, 2,6-Dimethylpyrazin, 2-Isopropoxyethanol, Diethyldisulfid, 2-Methylpiperazin, 1-Methylcyclohexanol, 1-Bromhexan, Cyclohexanon, 1-Heptanamin, 2,3-Dimethylpyrazin, 2-Ethoxyethanolacetat, 3-Hydroxybutanal, 1-Hexanol, 2-(Methylamino)ethanol, 1,4-Butandiamin, 2,4-Dimethylpyridin, 2-Pyrimidinamin, 2-Heptanol, 2-Methoxy-3-methylpyrazin, Dibutylamin, 4-Methoxy-4-methylpentan-2-on, 1,4-Dichlor-trans-2-buten, 3-Ethoxy-1-propanol, Cyclohexanol, 1,4-Dichlorbutan, 3-Methoxy-1-butanol, Furfural, Diglym, 1,1,1-Trichlor-2-propanol, 2-(Diethylamino)ethanol, 3-Methoxypropannitril, 2,2-Diethoxyethanamin, 1,3,5-Trimethylbenzol, 2-Methoxy-N-(2-methoxyethyl)ethanamin, 2-Chlorpyridin, Bromcyclohexan, 2,3-Heptandion, 2-(Ethylamino)ethanol, 3-Methylcyclohexanol, 1,3-Dibrompropan, 2-Methylcyclohexanol, 3-Octanon, Diacetonalkohol, Diethylaminopropylamin, 2-Ethylhexylamin, 1,3-Propandithiol, Ethoxybenzol, 1-Butoxy-2-propanol, 2-Furanmethanol, 2-Butoxyethanol, Methylacetoacetat, 2-Octanon, 2-Aminopropanol, 1,4-Dichlorbenzol, Methylheptanoat, Triethylendiamin, N,N-Dimethylpropanamid, 2-Chlorphenol, 2-Amino-1-butanol, 1-Heptanol, 2-Methyl-2-nitro-1-propanol 2-Propanol-1-methoxypropanoat, 1,5-Pentandiamin, 2-Octanol, Cycloheptanon, 3,4-Dimethylpyridin, 1-Octanamin, Benzylmethylamin, Dimethylmalonat, 1-Chloroctan, Cyclohexanmethanol, 1,1,3,3-Tetramethoxypropan, 2-(Ethylthio)ethanol, 1-Ethoxy-2-methylbenzol, Anilin, 2-Ethyl-1-hexanol, tert-Butylcyclohexylamin, Dihexylphthalat, 1-Fluordecan, 2-Chlor-1,4-dimethylbenzol, Dimethylsulfat, Dimethylsulfoxid, Diethylpropanolamin, 2-Methylphenol, 2-Butoxyethanolacetat, Diethylsulfoxid, 1-Octanol, 2-Brompyridin, 2-(2-Methoxyethoxy)ethanol, 1,2-Butandiol, 2-Bromphenol, 4-Methylbenzolmethanamin, Diethylenglykolmonoethylether, 1,2,4,5-Tetramethylbenzol, 2-Propylcyclohexanon, 1,4-Dibrombutan, Trimethylphosphat, 2-Methyl-2,4-pentandiol, 1,3-Diethenylbenzol, Methylbenzoat, 1-Octanthiol, Diethylmalonat, 2-Methoxypyrimidin bestehenden Gruppe ausgewählt ist.

4. Verfahren nach dem vorhergehenden Anspruch, **dadurch gekennzeichnet, dass** das organische Extraktionsmittel aus der aus Isopropylmethylamin, Methyl-t-butylether, Diethylamin, Propanon, Methylacetat, 2-Butanamin, N-Methylpropylamin, Tetrahydrofuran, 1-Butylamin, Ethylacetat, Butanon, n-Propylformiat, Dimethoxypropan, Diisopropylamin, 1,2-Dimethoxyethan, 3-Methyl-2-butanamin, Diethoxymethan, Isopropylacetat, 3-Pentylamin, N-Methylbutylamin, 1-Methoxy-2-propanamin, 2-Methoxyethanamin, tert-Butylacetat, Ethylpropionat, 1,2-Dimethoxypropan, Dioxan, 3-Pentanon, 1,1-Diethoxyethan, 2-Pentanon, 2-Methoxy-1-propanamin, Trimethoxymethan, n-Pentylamin, 3,3-Dimethyl-2-butanon, 1,3-Dioxan, Piperidin, 2-Ethoxyethanamin, sek-Butylacetat, N-Methyl-1,2-ethandiamin, 2,2-Diethoxypropan, 1,2-Diaminoethan, 1-Methoxy-2-propanol, 1,2-Propandiamin, 2,6-Dimethyl-5-heptenal, 1-(Dimethylamino)-2-propanol, 3-Methyl-3-pentanol, 2-Ethylbutylamin, Diethylcarbonat, n-Butylacetat, 2-Hexanon, N-Ethylethylendiamin, 2-Methoxy-1-propanol, 1-Ethoxy-2-propanol, 4-Methyl-2-hexanamin, Hexylamin, Methoxycyclohexan, 2-(Dimethylamino)ethanol, Cyclohexylamin, N-Ethyl-2-dimethylaminoethylamin, Ethoxyethanol, 2-Ethoxy-1-propanol, 1-Methylpiperazin, 1,3-Propandiamin, 2-Heptanamin, N,N-Diethylethylendiamin, 4-Methyl-2-hexanon, 1,1,1-Triethoxyethan, 1-Methoxy-2-acetoxypropan, 4-Methylpyridin, N,N'-Diethyl-1,2-ethandiamin, 2,6-Dimethylmorpholin, Methylhexanoat, 2-Propoxyethanol, 1-Propoxy-2-propanol, 2-Heptanon, Dimethylformamid, 2-Isopropoxyethanol, 2-Methylpiperazin, Cyclohexanon, 1-Heptanamin, 2-Ethoxyethanolacetat, 1,4-Butandiamin, 2,4-Dimethylpyridin, 2-Methoxy-3-methylpyrazin, 4-Methoxy-4-methylpentan-2-on, 3-Ethoxy-1-propanol, 3-Methoxy-1-butanol, Diglym, 2-(Diethylamino)ethanol, 2,2-Diethoxyethanamin, 2-Methoxy-N-(2-methoxyethyl)ethanamin, 2-(Ethylamino)ethanol, 3-Octanon, Diacetonalkohol, Diethylaminopropylamin, 2-Ethylhexylamin, 1-Butoxy-2-propanol, 2-Butoxyethanol, 2-Octanon, Methylheptanoat, Triethylendiamin, N,N-Dimethylpropanamid, 2-Propyl-1-methoxypropanoat, 1,5-Pentandiamin, Cycloheptanon, 3,4-Dimethylpyridin, 1-Octanamin, Benzylmethylamin, 1,1,3,3-Tetramethoxypropan, Dihexylphthalat, Diethylpropanolamin, 2-Butoxyethanolacetat, Diethylsulfoxid, 2-(2-Methoxyethoxy)ethanol, 4-Methylbenzolmethanamin, Diethylenglykolmonoethylether, 2-Propylcyclohexanon, Trimethylphosphat, 2-Methyl-2,4-pentandiol, Methylbenzoat, Diethylmalonat und 2-Methoxypyrimidin bestehenden Gruppe ausgewählt ist; vorzugsweise ist das organische Extraktionsmittel aus der aus Diethylamin, Propanon, Methylacetat, Tetrahydrofuran, Ethylacetat, Butanon, Diethoxymethan, Isopropylacetat, tert-Butylacetat, Dioxan, 3-Pentanon, 1,1-Diethoxyethan, 2-Pentanon, n-Pentylamin, 1,3-Dioxan, sek-Butylacetat, 1,2-Diaminoethan, 1-Methoxy-2-propanol, n-Butylacetat, 1-Ethoxy-2-propanol bestehenden Gruppe ausgewählt.

5. Verfahren nach einem der vorhergehenden Ansprüche, **dadurch gekennzeichnet, dass** der im Schritt c) abgetrennte Strom, der das organische Extraktionsmittel umfasst, zum Schritt a) rückgeführt wird.

6. Verfahren nach einem der vorhergehenden Ansprüche, **dadurch gekennzeichnet, dass** im Schritt b) hergestellte Strom, der 2-Chlor-3,3,3-trifluorpropen umfasst, am Kopf der Destillationskolonne gewonnen und gegebenenfalls in ein Verfahren zur Produktion von 2,3,3,3-Tetrafluorpropen rückgeführt wird.

7. Verfahren nach einem der vorhergehenden Ansprüche, **dadurch gekennzeichnet, dass** das organische Extraktionsmittel aus der aus Propanon, Methylacetat, Ethylacetat, Butanon, Dioxan, Trimethoxymethan, 1,3-Dioxan, 1,2-Diaminoethan, 1-Methoxy-2-propanol, Diethylcarbonat, 2-Methoxy-1-propanol, 1-Methoxy-2-acetoxypropan, Dimethylformamid, 3-Methoxy-1-butanol, Diacetonalkohol, N,N-Dimethylpropanamid, Diethylsulfoxid, 2-(2-Methoxyethoxy)ethanol, Trimethylphosphat, Diethylmalonat bestehenden Gruppe ausgewählt ist; vorzugsweise ist das organische Extraktionsmittel aus der aus Propanon, Methylacetat, Ethylacetat, Butanon, Dioxan, Trimethoxymethan, 1,3-Dioxan, 1,2-Diaminoethan, 1-Methoxy-2-propanol, 3-Methoxy-1-butanol, Diacetonalkohol bestehenden Gruppe ausgewählt.

8. Verfahren nach einem der vorhergehenden Ansprüche, **dadurch gekennzeichnet, dass** die erste Zusammensetzung eine azeotrope oder quasi-azeotrope Zusammensetzung ist, die 2-Chlor-3,3,3-trifluorpropen und E-1-Chlor-3,3,3-trifluor-1-propen (1233zdE) umfasst, oder die erste Zusammensetzung eine azeotrope oder quasi-azeotrope Zusammensetzung ist, die 2-Chlor-3,3,3-trifluorpropen und 1,1,1,3,3-Pentafluorpropan (245fa) umfasst.

9. Verfahren zur Produktion von 2,3,3,3-Tetrafluor-1-propen, umfassend die folgenden Schritte:

A) Fluorierung in einem Reaktor in Gegenwart eines Katalysators einer Verbindung der Formel (I) $CX(Y)_2-CX(Y)_m-CH_mXY$, worin X und Y unabhängig ein Wasserstoff-, Fluor- oder Chloratom darstellen und m = 0 oder 1; und/oder katalytische Fluorierung in Gegenwart eines Katalysators einer Verbindung der Formel $(CX_nY_{3-n})CH_pX_{1-p}CH_mX_{2-m}$ (II), worin X unabhängig voneinander Cl, F, I oder Br ist; Y unabhängig voneinander H, Cl, F, I oder Br ist; n 1, 2 oder 3 ist und m 0, 1 oder 2 ist und p 0 oder 1 ist;
B) Gewinnung eines von einer Spülung des Rückführungsreaktionskreislaufs herrührenden Stroms, der 1,1,1,2,2-Pentafluorpropan, 2-Chlor-3,3,3-trifluorpropen und mindestens eine der aus der aus E-1-Chlor-3,3,3-trifluor-1-propen (1233zdE) und 1,1,1,3,3-Pentafluorpropan (245fa) bestehenden Gruppe ausgewählten Verbindungen umfasst;
C) Destillation des im Schritt B) gewonnenen Stroms und Gewinnung eines Stroms am Kopf der Destillationskolonne, der 1,1,1,2,2-Pentafluorpropan umfasst, und eines Stroms am Boden der Destillationskolonne, der 2-Chlor-3,3,3-trifluorpropen (1233xf) und mindestens eine der aus der aus E-1-Chlor-3,3,3-trifluor-1-propen (1233zdE) und 1,1,1,3,3-Pentafluorpropan (245fa) bestehenden Gruppe ausgewählten Verbindungen umfasst;
D) Durchführen des Verfahrens zur Reinigung von 2-Chlor-3,3,3-trifluorpropen nach einem der Ansprüche 1 bis 8 ausgehend von dem im Schritt C) gewonnenen Strom, der 2-Chlor-3,3,3-trifluorpropen (1233xf) und mindestens eine der aus der aus E-1-Chlor-3,3,3-trifluor-1-propen (1233zdE) und 1,1,1,3,3-Pentafluorpropan (245fa) bestehenden Gruppe ausgewählten Verbindungen umfasst; und
E) Rückführen des im Schritt b) des im Schritt D) durchgeführten Reinigungsverfahrens hergestellten und gewonnenen Stroms, der 2-Chlor-3,3,3-trifluorpropen umfasst, zum Schritt A).

10. Verfahren nach dem vorhergehenden Anspruch, **dadurch gekennzeichnet, dass** der im Schritt B) gewonnene Strom auch HF, 1,1,1,2,2-Pentafluorpropan und 1,3,3,3-Tetrafluor-1-propen (1234ze) umfasst, die vor der Durchführung der Destillation von Schritt C) gemäß den folgenden Schritten behandelt werden:

i) Abtrennung der flüssigen Zusammensetzung bei niedriger Temperatur unter Herstellung einer ersten an HF reichen Phase und einer zweiten organischen Phase, die 1,1,1,2,2-Pentafluorpropan und 1,3,3,3-Tetrafluor-1-propen (1234ze), 2-Chlor-3,3,3-trifluorpropen (1233xf) und mindestens eine der aus der aus E-1-Chlor-3,3,3-trifluor-1-propen (1233zdE) und 1,1,1,3,3-Pentafluorpropan (245fa) bestehenden Gruppe ausgewählten Verbindungen enthält;
ii) Destillation der zweiten organischen Phase, um vorteilhafterweise am Kopf der Destillationskolonne einen ersten Strom herzustellen und zu gewinnen, der 1,1,1,2,2-Pentafluorpropan und 1,3,3,3-Tetrafluor-1-propen (1234ze) enthält, und um vorteilhafterweise am Boden der Destillationskolonne einen zweiten Strom herzustellen und zu gewinnen, der 2-Chlor-3,3,3-trifluorpropen (1233xf) und mindestens eine der aus der aus E-1-Chlor-3,3,3-trifluor-1-propen (1233zdE) und 1,1,1,3,3-pentafluorpropan (245fa) bestehenden Gruppe ausgewählten Verbindungen enthält;
iii) Gewinnen des zweiten Stroms und Durchführen von Schritt D) ausgehend von demselben.

11. Zusammensetzung, umfassend 2-Chlor-3,3,3-trifluorpropen (1233xf), 1,1,1,3,3-Pentafluorpropan (245fa) und ein aus der aus Ethandial, Propanon, Methylacetat, Methylglyoxal, Ethylacetat, Butanon, Propionitril, Dioxan, Trimethoxymethan, 1,3-Dioxan, 1,3,5-Trioxan, 1,2-Diaminoethan, 1-Methoxy-2-propanol, Diethylcarbonat, 2-Methoxy-1-propanol, 1-Methoxy-2-acetoxypropan, Dimethylformamid, 3-Methoxy-1-butanol, Diacetonalkohol, Methylacetoacetate, N,N-Dimethylpropanamid, Dimethylmalonat, Diethylsulfoxid, 2-(2-Methoxyethoxy)ethanol, Trimethylphosphat, Diethylmalonat bestehenden Gruppe ausgewähltes organisches Extraktionsmittel; insbesondere ist das organische Extraktionsmittel aus der aus Propanon, Methylacetat, Ethylacetat, Butanon, Dioxan, Trimethoxymethan, 1,3-Dioxan, 1,3,5-Trioxan, 1,2-Diaminoethan, 1-Methoxy-2-propanol bestehenden Gruppe ausgewählt.

12. Zusammensetzung, umfassend 2-Chlor-3,3,3-trifluorpropen (1233xf), E-1-Chlor-3,3,3-trifluor-1-propen (1233zdE) und ein aus der aus Isopropylmethylamin, Methyl-t-butylether, Diethylamin, Propanon, Methylacetat, 2-Butanamin, N-Methylpropylamin, Tetrahydrofuran, 1-Butylamin, Ethylacetat, Butanon, n-Propylformiat, Dimethoxypropan, Diisopropylamin, 1,2-Dimethoxyethan, 3-Methyl-2-butanamin, Diethoxymethan, Isopropylacetat, 3-Pentylamin, N-Methylbutylamin, 1-Methoxy-2-propanamin, 2-Methoxyethanamin, tert-Butylacetat, Ethylpropionat, 1,2-Dimethoxypropan, Dioxan, 3-Pentanon, 1,1-Diethoxyethan, 2-Pentanon, 2-Methoxy-1-propanamin, Trimethoxymethan, n-Pentylamin, 3,3-Dimethyl-2-butanon, 1,3-Dioxan, Piperidin, 2-Ethoxyethanamin, sek-Butylacetat, N-Methyl-1,2-ethandiamin, 2,2-Diethoxypropan, 1,2-Diaminoethan, 1-Methoxy-2-propanol, 1,2-Propandiamin, 2,6-Dimethyl-5-heptenal, 1-(Dimethylamino)-2-propanol, 3-Methyl-3-pentanol, 2-Ethylbutylamin, Diethylcarbonat, n-Butylacetat, 2-Hexanon, N-Ethylethylendiamin, 2-Methoxy-1-propanol, 1-Ethoxy-2-propanol, 4-Methyl-2-hexanamin, Hexylamin, Methoxycyclohexan, 2-(Dimethylamino)ethanol, Cyclohexylamin, N-Ethyl-2-dimethylaminoethylamin, Ethoxyethanol, 2-Ethoxy-1-propanol, 1-Methylpiperazin, 1,3-Propandiamin, 2-Heptanamin, N,N-Diethylethylendiamin, 4-Methyl-2-hexanon, 1,1,1-Triethoxyethan, 1-Methoxy-2-acetoxypropan, 4-Methylpyridin, N,N'-Diethyl-1,2-ethandiamin, 2,6-Dimethylmorpholin, Methylhexanoat, 2-Propoxyethanol, 1-Propoxy-2-propanol, 2-Heptanon, Dimethylformamid, 2-Isopropoxyethanol, 2-Methylpiperazin, Cyclohexanon, 1-Heptanamin, 2-Ethoxyethanolacetat, 1,4-Butandiamin, 2,4-Dimethylpyridin, 2-Methoxy-3-methylpyrazin, 4-Methoxy-4-methylpentan-2-on, 3-Ethoxy-1-propanol, 3-Methoxy-1-butanol, Diglym, 2-(Diethylamino)ethanol, 2,2-Diethoxyethanamin, 2-Methoxy-N-(2-methoxyethyl)ethanamin, 2-(Ethylamino)ethanol, 3-Octanon, Diacetonalkohol, Diethylaminopropylamin, 2-Ethylhexylamin, 1-Butoxy-2-propanol, 2-Butoxyethanol, 2-Octanon, Methylheptanoat, Triethylendiamin, N,N-Dimethylpropanamid, 2-Propanol-1-methoxypropanoat, 1,5-Pentandiamin, Cycloheptanon, 3,4-Dimethylpyridin, 1-Octanamin, Benzylmethylamin, 1,1,3,3-Tetramethoxypropan, Dihexylphthalat, Diethylpropanolamin, 2-Butoxyethanolacetat, Diethylsulfoxid, 2-(2-Methoxyethoxy)ethanol, 4-Methylbenzolmethanamin, Diethylenglykolmonoethylether, 2-Propylcyclohexanon, Trimethylphosphat, 2-Methyl-2,4-pentandiol, Methylbenzoat, Diethylmalonat, 2-Methoxypyrimidin bestehenden Gruppe ausgewähltes organisches Extraktionsmittel; insbesondere ist das organische Extraktionsmittel aus der aus Diethylamin, Propanon, Methylacetat, Tetrahydrofuran, Ethylacetat, Butanon, Diethoxymethan, Isopropylacetat, tert-Butylacetat, Dioxan, 3-Pentanon, 1,1-Diethoxyethan, 2-Pentanon, n-Pentylamin, 1,3-Dioxan, sek-Butylacetat, 1,2-Diaminoethan, 1-Methoxy-2-propanol, n-Butylacetat, 1-Ethoxy-2-propanol bestehenden Gruppe ausgewählt.

**Claims**

1. Process for purifying 2-chloro-3,3,3-trifluoropropene (1233xf) from a first composition comprising 2-chloro-3,3,3-trifluoropropene and 1,1,1,3,3-pentafluoropropane (245fa), said process comprising the steps of:

   a) placing said first composition in contact with at least one organic extracting agent to form a second composition;
   b) extractive distillation of said second composition to form:

      i) a third composition comprising said organic extracting agent and 1,1,1,3,3-pentafluoropropane (245fa),
      ii) a stream comprising 2-chloro-3,3,3-trifluoropropene;

   c) recovering and separating out said third composition to form a stream comprising said organic extracting agent and a stream comprising 1,1,1, 3, 3-pentafluoropropane (245fa) ; **characterized in that** said organic extracting agent is chosen from the group consisting of ethanedial, propanone, methyl acetate, chloromethoxymethane, isobutanal, methanol, isopropyl formate, methylglyoxal, 2,2,2-trifluoroethanol, 1,1,1-trifluoro-2-propanol, ethyl acetate, ethanol, butanone, n-propyl formate, 2-propanol, acetonitrile, pentafluoro-1-propanol, tert-butanol, 1,2-dimethoxyethane, isopropyl acetate, 2-methylbutanal, 2-methoxyethanamine, tert-butyl acetate, propionitrile, 2-allyloxyethanol, ethyl propionate, dioxane, 3-pentanone, 2-pentanone, trimethoxymethane, 3,3-dimethyl-2-butanone, 1,3-dioxane, 2,2,3,3-tetrafluoro-1-propanol, sec-butyl acetate, N-methyl-1,2-ethanediamine, 1,3,5-trioxane, 4-methyl-2-pentanone, 1,2-diaminoethane, butyronitrile, 1-methoxy-2-propanol, 1,2-propanediamine, perfluorobutanoic acid, 1-(dimethylamino)-2-propanol, 2-methoxyethanol, 4,4,4-trifluorobutanol, diethyl carbonate, n-butyl acetate, 2-hexanone, 3-fluoropropanol, 5-hexen-2-one, 2-methoxy-1-propanol, 1-ethoxy-2-propanol, 2-(dimethylamino)ethanol, ethoxyethanol, 2-ethoxy-1-propanol, 1,3-propanediamine, 3,3,4,4,5,5,6,6-octafluoro-1-pentanol, valeronitrile, (methyleneamino)acetonitrile, 3-furfural, 4-methyl-2-hexanone, 1-methoxy-2-acetoxypropane, methyl hexanoate, 2-propoxyethanol, 1-propoxy-2-propanol, dimethylethanolamine, 2-heptanone, dimethylformamide, 2-isopropoxyethanol, cyclohexanone, 2-ethoxyethyl acetate, 3-hydroxybutanal, 2-(methylamino)ethanol, 4-methoxy-4-methylpentan-2-one, 3-ethoxy-1-propanol, 3-methoxy-1-butanol, furfural, diglyme, 3-methoxypropanenitrile, 2-(ethylamino)ethanol, 3-octanone, diacetone alcohol, 1-butoxy-2-propanol, 2-furanmethanol, methyl acetoacetate, 2-aminopropanol, N,N-dimethylpropanamide, 2-amino-1-butanol, 2-methyl-2-nitro-1-propanol, 2-propyl-1-methoxypropanoate, cycloheptanone, dimethyl malonate, 1,1,3,3-tetramethoxypropane, dimethyl sulfate, dimethyl sulfoxide, 2-butoxyethyl acetate, diethyl sulfoxide, 2-(2-methoxyethoxy)ethanol, 1,2-butanediol, diethylene glycol monoethyl ether, trimethyl phosphate, 2-methyl-2,4-pentanediol, methyl benzoate and diethyl malonate.

2. Process according to the preceding claim, **characterized in that** said organic extracting agent is chosen from the group consisting of ethanedial, propanone, methyl acetate, methylglyoxal, ethyl acetate, butanone, propionitrile, dioxane, trimethoxymethane, 1,3-dioxane, 1,3,5-trioxane, 1,2-diaminoethane, 1-methoxy-2-propanol, diethyl carbonate, 2-methoxy-1-propanol, 1-methoxy-2-acetoxypropane, dimethylformamide, 3-methoxy-1-butanol, diacetone alcohol, methyl acetoacetate, N,N-dimethylpropanamide, dimethyl malonate, diethyl sulfoxide, 2-(2-methoxyethoxy)ethanol, trimethyl phosphate and diethyl malonate; preferably, said organic extracting agent is chosen from the group consisting of propanone, methyl acetate, ethyl acetate, butanone, dioxane, trimethoxymethane, 1,3-dioxane, 1,3,5-trioxane, 1,2-diaminoethane and 1-methoxy-2-propanol.

3. Process for purifying 2-chloro-3,3,3-trifluoropropene (1233xf) from a first composition comprising 2-chloro-3,3,3-trifluoropropene and E-1-chloro-3,3,3-trifluoro-1-propene (1233zdE), said process comprising the steps of:

   a) placing said first composition in contact with at least one organic extracting agent to form a second composition;
   b) extractive distillation of said second composition to form:

      i) a third composition comprising said organic extracting agent and E-1-chloro-3,3,3-trifluoro-1-propene (1233zdE),
      ii) a stream comprising 2-chloro-3,3,3-trifluoropropene;

   c) recovering and separating out said third composition to form a stream comprising said organic extracting agent and a stream comprising E-1-chloro-3,3,3-trifluoro-1-propene (1233zdE); **characterized in that** said organic extracting agent is chosen from the group consisting of isopropylmethylamine, ethanedial, 2-chloro-2-methylpropane, 2-propanethiol, 2-ethoxypropane, 1-chloro-2,2-difluoropropane, methyl t-butyl ether, diethylamine, propanone, methyl acetate, 1,1-dichloroethane, 4-methoxy-2-methyl-2-butanethiol, 2-bromopropane, chloromethoxymethane, difluorodiethylsilane, 2-butanamine, N-methylpropylamine, tert-butylthiol, isobutanal, tetrahydrofuran, 1-propanethiol, 2-chlorobutane, isopropyl formate, diisopropyl ether, 1-bromopropane, methylglyoxal, 2-ethoxy-2-methylpropane, 2-bromo-2-methylpropane, 1-chloro-3-fluoropropane, 1,1,1-trifluoro-2-propanol, 1-butylamine, ethyl acetate, 1-chlorobutane, ethanol, butanone, n-propyl formate, 2-ethoxybutane, 2-propanol, acetonitrile, tert-butanol, 1-methoxy-2-methylbutane, cyclohexene, 2,2-dimethoxypropane, 2-chloro-2-methylbutane, 1,2-dichloroethane, 1-ethoxy-2-methylpropane, diisopropylamine, 2-butanethiol, 1,2-dimethoxyethane, 3-methyl-2-butanamine, diethoxymethane, 1,1-dichloropropane, 2-methyl-1-propanethiol, isopropyl acetate, 1,2-dichloro-2-fluoropropane, di-n-propyl ether, 3-pentylamine, N-methylbutylamine, 2-bromobutane, diethyl sulfide, 1-ethoxybutane, 1-fluorohexane, 1-methoxy-2-propanamine, 2,3-dichloro-1-propene, 2-methylbutanal, 2-methoxyethanamine, 1,2-dichloropropane, propanol, tert-butyl acetate, propionitrile, 3-chloropentane, trichloroacetaldehyde, 2-allyloxyethanol, butanethiol, isoamyl chloride, 1-methoxypentane, ethyl propionate, 2-butanol, 1,2-dimethoxypropane, isopropyl isobutyl ether, 1-chloro-4-fluorobutane, 2,4,4-trimethyl-1-pentene, 1-bromo-3-fluoropropane, dioxane, 1-bromobutane, 3-pentanone, 1,1-diethoxyethane, 2-pentanone, 2-methyl-2-butanol, 2-methoxy-1-propanamine, trimethoxymethane, 2,2-dichlorobutane, cis-1,3-dichloropropene, n-pentylamine, 3,3-dimethyl-2-butanone, 1,3-dioxane, piperidine, isobutanol, 2-bromo-2-methylbutane, dipropylamine, 2-ethoxyethanamine, triethylfluorosilane, 1-methylcyclohexene, sec-butyl acetate, trans-1,3-dichloropropene, 2,2-dimethyl-1-propanol, 1,1,1-trichloro-3-fluoropropane, N-methyl-1,2-ethanediamine, 2,2-diethoxypropane, 1,3,5-trioxane, 1-chloro-3,3-dimethylbutane, pyridine, N-methylmorpholine, 3-pentanol, 4-methyl-2-pentanone, 1,2-diaminoethane, isobutyl tert-butyl ether, 2-bromopentane, butyronitrile, 1-butanol, 2,3-dichlorobutane, sec-butyl tert-butyl ether, 1-methoxy-2-propanol, 1,2-propanediamine, 2,6-dimethyl-5-heptenal, 1-bromo-3-methylbutane, 1,3-dichloro-trans-2-butene, 1,3-dichloropropane, 1-(dimethylamino)-2-propanol, tetrahydrothiophene, 3-methyl-3-pentanol, 1,1-diethoxypropane, 1,2,2-trichloropropane, 1-chloro-2-methyl-2-propanol, 2-methoxyethanol, 1,2-dichlorobutane, 4,4,4-trifluorobutanol, 2-ethylbutylamine, diethyl carbonate, n-butyl acetate, 1-pentanethiol, 2-chloro-1,1-dimethoxyethane, 2-hexanone, N-ethylethylenediamine, 3-fluoropropanol, 5-hexen-2-one, 2,3-dichloro-2-methylbutane, 1,1-diethoxy-N,N-dimethylmethanamine, 2-methylpyridine, 1-bromopentane, 2-methoxy-1-propanol, 1,2-dichloro-2-butene, hexanal, 1-ethoxy-2-propanol, 4-methyl-2-pentanol, bromoacetic acid methyl ester, 1,2-octanediol, 4-methyl-2-hexanamine, hexylamine, methoxycyclohexane, 2-(dimethylamino)ethanol, 1,3-dichlorobutane, cyclohexylamine, N-ethyl-2-dimethylaminoethylamine, ethoxyethanol, 3-hexanol, 2-hexanol, 2-methylpyrazine, 2-ethoxy-1-propanol, 1-pentanol, N-ethylmorpholine, 1-methylpiperazine, 1,4-dimethylbenzene, 1,3-propanediamine, di-n-butyl ether, valeronitrile, (methyleneamino)acetonitrile, 2-heptanamine, 2,3-dimethylbutanol, 1-ethoxyhexane, 1-chloro-3-bromopropane, N,N-diethylethylenediamine, 3-furfural, 2,6-dimethylpyridine, 4-methyl-2-hexanone, 1,1,1-triethoxyethane, styrene, 1-methoxy-2-acetoxypropane, 4-methylpyridine, N,N'-diethyl-1,2-ethanediamine, 2,6-dimethylmorpholine, 2-ethyl-1-butanol, 1,2-dichloropentane, 2-methyl-1-pentanol, methyl hexanoate, 2-propoxyethanol, 1-propoxy-2-propanol, dimethylethanolamine, isopropyl chloroacetate, 2-heptanone, 1-hexanethiol, 1,2-propanedithiol, heptanal, dimethylformamide, 2,6-dimethylpyrazine, 2-isopropoxyethanol, diethyl disulfide, 2-methylpiperazine, 1-methylcyclohexanol, 1-bromohexane, cyclohexanone, 1-heptanamine, 2,3-dimethylpyrazine, 2-ethoxyethyl acetate, 3-hydroxybutanal, 1-hexanol, 2-(methylamino)ethanol, 1,4-butanediamine, 2,4-dimethylpyridine, 2-pyrimidinamine, 2-heptanol, 2-methoxy-3-methylpyrazine, dibutylamine, 4-methoxy-4-methylpentan-2-one, 1,4-dichloro-trans-2-butene, 3-ethoxy-1-propanol, cyclohexanol, 1,4-dichlorobutane, 3-methoxy-1-butanol, furfural, diglyme, 1,1,1-trichloro-2-propanol, 2-(diethylamino)ethanol, 3-methoxypropanenitrile, 2,2-diethoxyethanamine, 1,3,5-trimethylbenzene, 2-methoxy-N-(2-methoxyethyl)ethanamine, 2-chloropyridine, bromocyclohexane, 2,3-heptanedione, 2-(ethylamino)ethanol, 3-methylcyclohexanol, 1,3-dibromopropane, 2-methylcyclohexanol, 3-octanone, diacetone alcohol, diethylaminopropylamine, 2-ethylhexylamine, 1,3-propanedithiol, ethoxybenzene, 1-butoxy-2-propanol, 2-furanmethanol, 2-butoxyethanol, methyl acetoacetate, 2-octanone, 2-aminopropanol, 1,4-dichlorobenzene, methyl heptanoate, triethylenediamine, N,N-dimethylpropanamide, 2-chlorophenol, 2-amino-1-butanol, 1-heptanol, 2-methyl-2-nitro-1-propanol, 2-propyl-1-methoxypropanoate, 1,5-pentanediamine, 2-octanol, cycloheptanone, 3,4-dimethylpyridine, 1-octanamine, benzylmethylamine, dimethyl malonate, 1-chlorooctane, cyclohexanemethanol, 1,1,3,3-tetramethoxypropane, 2-(ethylthio)ethanol, 1-ethoxy-2-methylbenzene, aniline, 2-ethyl-1-hexanol, tert-butylcyclohexylamine, dihexyl phthalate, 1-fluorodecane, 2-chloro-1,4-dimethylbenzene, dimethyl sulfate, dimethyl sulfoxide, diethylpropanolamine, 2-methylphenol, 2-butoxyethyl acetate, diethyl sulfoxide, 1-octanol, 2-bromopyridine, 2-(2-methoxyethoxy)ethanol, 1,2-butanediol, 2-bromophenol, 4-methylbenzenemethanamine, diethylene glycol monoethyl ether, 1,2,4,5-tetramethylbenzene, 2-propylcyclohexanone, 1,4-dibromobutane, trimethyl phosphate, 2-methyl-2,4-pentanediol, 1,3-diethenylbenzene, methyl benzoate, 1-octanethiol, diethyl malonate and 2-methoxypyrimidine.

4. Process according to the preceding claim, **characterized in that** said organic extracting agent is chosen from the group consisting of isopropylmethylamine, methyl t-butyl ether, diethylamine, propanone, methyl acetate, 2-butanamine, N-methylpropylamine, tetrahydrofuran, 1-butylamine, ethyl acetate, butanone, n-propyl formate, dimethoxypropane, diisopropylamine, 1,2-dimethoxyethane, 3-methyl-2-butanamine, diethoxymethane, isopropyl acetate, 3-pentylamine, N-methylbutylamine, 1-methoxy-2-propanamine, 2-methoxyethanamine, tert-butyl acetate, ethyl propionate, 1,2-dimethoxypropane, dioxane, 3-pentanone, 1,1-diethoxyethane, 2-pentanone, 2-methoxy-1-propanamine, trimethoxymethane, n-pentylamine, 3,3-dimethyl-2-butanone, 1,3-dioxane, piperidine, 2-ethoxyethanamine, sec-butyl acetate, N-methyl-1,2-ethanediamine, 2,2-diethoxypropane, 1,2-diaminoethane, 1-methoxy-2-propanol, 1,2-propanediamine, 2,6-dimethyl-5-heptenal, 1-(dimethylamino)-2-propanol, 3-methyl-3-pentanol, 2-ethylbutylamine, diethyl carbonate, n-butyl acetate, 2-hexanone, N-ethylethylenediamine, 2-methoxy-1-propanol, 1-ethoxy-2-propanol, 4-methyl-2-hexanamine, hexylamine, methoxycyclohexane, 2-(dimethylamino)ethanol, cyclohexylamine, N-ethyl-2-dimethylaminoethylamine, ethoxyethanol, 2-ethoxy-1-propanol, 1-methylpiperazine, 1,3-propanediamine, 2-heptanamine, N,N-diethylethylenediamine, 4-methyl-2-hexanone, 1,1,1-triethoxyethane, 1-methoxy-2-acetoxypropane, 4-methylpyridine, N,N'-diethyl-1,2-ethanediamine, 2,6-dimethylmorpholine, methyl hexanoate, 2-propoxyethanol, 1-propoxy-2-propanol, 2-heptanone, dimethylformamide, 2-isopropoxyethanol, 2-methylpiperazine, cyclohexanone, 1-heptanamine, 2-ethoxyethyl acetate, 1,4-butanediamine, 2,4-dimethylpyridine, 2-methoxy-3-methylpyrazine, 4-methoxy-4-methylpentan-2-one, 3-ethoxy-1-propanol, 3-methoxy-1-butanol, diglyme, 2-(diethylamino)ethanol, 2,2-diethoxyethanamine, 2-methoxy-N-(2-methoxyethyl)ethanamine, 2-(ethylamino)ethanol, 3-octanone, diacetone alcohol, diethylaminopropylamine, 2-ethylhexylamine, 1-butoxy-2-propanol, 2-butoxyethanol, 2-octanone, methyl heptanoate, triethylenediamine, N,N-dimethylpropanamide, 2-propyl-1-methoxypropanoate, 1,5-pentanediamine, cycloheptanone, 3,4-dimethylpyridine, 1-octanamine, benzylmethylamine, 1,1,3,3-tetramethoxypropane, dihexyl phthalate, diethylpropanolamine, 2-butoxyethyl acetate, diethyl sulfoxide, 2-(2-methoxyethoxy)ethanol, 4-methylbenzenemethanamine, diethylene glycol monoethyl ether, 2-propylcyclohexanone, trimethyl phosphate, 2-methyl-2,4-pentanediol, methyl benzoate, diethyl malonate and 2-methoxypyrimidine; preferably, said organic extracting agent is chosen from the group consisting of diethylamine, propanone, methyl acetate, tetrahydrofuran, ethyl acetate, butanone, diethoxymethane, isopropyl acetate, tert-butyl acetate, dioxane, 3-pentanone, 1,1-diethoxyethane, 2-pentanone, n-pentylamine, 1,3-dioxane, sec-butyl acetate, 1,2-diaminoethane, 1-methoxy-2-propanol, n-butyl acetate and 1-ethoxy-2-propanol.

5. Process according to any one of the preceding claims, **characterized in that** the stream comprising the organic extracting agent separated out in step c) is recycled into step a).

**6.** Process according to any one of the preceding claims, **characterized in that** said stream comprising 2-chloro-3,3,3-trifluoropropene formed in step b) is recovered at the top of the distillation column and optionally recycled into a process for producing 2,3,3,3-tetrafluoropropene.

**7.** Process according to any one of the preceding claims, **characterized in that** said organic extracting agent is chosen from the group consisting of propanone, methyl acetate, ethyl acetate, butanone, dioxane, trimethoxymethane, 1,3-dioxane, 1,2-diaminoethane, 1-methoxy-2-propanol, diethyl carbonate, 2-methoxy-1-propanol, 1-methoxy-2-acetoxypropane, dimethylformamide, 3-methoxy-1-butanol, diacetone alcohol, N,N-dimethylpropanamide, diethyl sulfoxide, 2-(2-methoxyethoxy)ethanol, trimethyl phosphate and diethylmalonate; preferably, said organic extracting agent is chosen from the group consisting of propanone, methyl acetate, ethyl acetate, butanone, dioxane, trimethoxymethane, 1,3-dioxane, 1,2-diaminoethane, 1-methoxy-2-propanol, 3-methoxy-1-butanol and diacetone alcohol.

**8.** Process according to any one of the preceding claims, **characterized in that** the first composition is an azeotropic or quasi-azeotropic composition comprising 2-chloro-3,3,3-trifluoropropene and E-1-chloro-3,3,3-trifluoro-1-propene (1233zdE); or the first composition is an azeotropic or quasi-azeotropic composition comprising 2-chloro-3,3,3-trifluoropropene and 1,1,1,3,3-pentafluoropropane (245fa) .

**9.** Process for producing 2,3,3,3-tetrafluoro-1-propene, comprising the steps of:

A) in a reactor, fluorination in the presence of a catalyst for a compound of formula (I) $CX(Y)_2$-$CX(Y)_m$-$CH_mXY$ in which X and Y independently represent a hydrogen, fluorine or chlorine atom and m = 0 or 1; and/or catalytic fluorination in the presence of a catalyst for a compound of formula $(CX_nY_{3-n})CH_pX_{1-p}CH_mX_{2-m}$ (II) in which X is, independently of each other, Cl, F, I or Br; Y is, independently of each other, H, Cl, F, I or Br; n is 1, 2 or 3; and m is 0, 1 or 2; and p is 0 or 1;

B) recovery of a stream derived from purging of the recycling reaction loop, comprising 1,1,1,2,2-pentafluoropropane, 2-chloro-3,3,3-trifluoropropene, and at least one compound chosen from the group consisting of E-1-chloro-3,3,3-trifluoro-1-propene (1233zdE) and 1,1,1,3,3-pentafluoropropane (245fa);

C) distillation of the stream recovered in step B) and recovery, at the top of the distillation column, of a stream comprising 1,1,1,2,2-pentafluoropropane and, at the bottom of the distillation column, of a stream comprising 2-chloro-3,3,3-trifluoropropene (1233xf) and at least one compound chosen from the group consisting of E-1-chloro-3,3,3-trifluoro-1-propene (1233zdE) and 1,1,1,3,3-pentafluoropropane (245fa) ;

D) performing the process for purifying 2-chloro-3,3,3-trifluoropropene according to any one of Claims 1 to 8 using the stream recovered in step C) and comprising 2-chloro-3,3,3-trifluoropropene (1233xf) and at least one compound chosen from the group consisting of E-1-chloro-3,3,3-trifluoro-1-propene (1233zdE) and 1,1,1,3,3-pentafluoropropane (245fa); and

E) recycling into step A) the stream comprising the 2-chloro-3,3,3-trifluoropropene formed and recovered in step b) of the purification process performed in step D).

**10.** Process according to the preceding claim, **characterized in that** the stream recovered in step B) also comprising HF, 1,1,1,2,2-pentafluoropropane and 1,3,3,3-tetrafluoro-1-propene (1234ze), the latter being, prior to performing the distillation of step C), treated according to the following steps:

i) low-temperature separation of said liquid composition to form a first HF-rich phase and a second organic phase containing 1,1,1,2,2-pentafluoropropane, 1,3,3,3-tetrafluoro-1-propene (1234ze), 2-chloro-3,3,3-trifluoropropene (1233xf) and at least one compound chosen from the group consisting of E-1-chloro-3,3,3-trifluoro-1-propene (1233zdE) and 1,1,1,3,3-pentafluoropropane (245fa);

ii) distillation of said second organic phase to form and recover, advantageously at the top of the distillation column, a first stream containing 1,1,1,2,2-pentafluoropropane and 1,3,3,3-tetrafluoro-1-propene (1234ze), and to form and recover, advantageously at the bottom of the distillation column, a second stream comprising 2-chloro-3,3,3-trifluoropropene (1233xf) and at least one compound chosen from the group consisting of E-1-chloro-3,3,3-trifluoro-1-propene (1233zdE) and 1,1,1,3,3-pentafluoropropane (245fa);

iii) recovery of said second stream and implementation of step D) using same.

**11.** Composition comprising 2-chloro-3,3,3-trifluoropropene (1233xf), 1,1,1,3,3-pentafluoropropane (245fa) and an organic extracting agent chosen from the group consisting of ethanedial, propanone, methyl acetate, methylglyoxal, ethyl acetate, butanone, propionitrile, dioxane, trimethoxymethane, 1,3-dioxane, 1,3,5-trioxane, 1,2-diaminoethane, 1-methoxy-2-propanol, diethyl carbonate, 2-methoxy-1-propanol, 1-methoxy-2-acetoxypropane, dimethylformamide, 3-methoxy-1-butanol, diacetone alcohol, methyl acetoacetate, N,N-dimethylpropanamide, dimethyl malonate, diethyl sulfoxide, 2-(2-methoxyethoxy)ethanol, trimethyl phosphate and diethyl malonate; in particular, said organic extracting agent is chosen from the group consisting of propanone, methyl acetate, ethyl acetate, butanone, dioxane, trimethoxymethane, 1,3-dioxane, 1,3,5-trioxane, 1,2-diaminoethane and 1-methoxy-2-propanol.

12. Composition comprising 2-chloro-3,3,3-trifluoropropene (1233xf), E-1-chloro-3,3,3-trifluoro-1-propene (1233zdE) and an organic extracting agent chosen from the group consisting of isopropylmethylamine, methyl t-butyl ether, diethylamine, propanone, methyl acetate, 2-butanamine, N-methylpropylamine, tetrahydrofuran, 1-butylamine, ethyl acetate, butanone, n-propyl formate, dimethoxypropane, diisopropylamine, 1,2-dimethoxyethane, 3-methyl-2-butanamine, diethoxymethane, isopropyl acetate, 3-pentylamine, n-methylbutylamine, 1-methoxy-2-propanamine, 2-methoxyethanamine, tert-butyl acetate, ethyl propionate, 1,2-dimethoxypropane, dioxane, 3-pentanone, 1,1-diethoxyethane, 2-pentanone, 2-methoxy-1-propanamine, trimethoxymethane, n-pentylamine, 3,3-dimethyl-2-butanone, 1,3-dioxane, piperidine, 2-ethoxyethanamine, sec-butyl acetate, N-methyl-1,2-ethanediamine, 2,2-diethoxypropane, 1,2-diaminoethane, 1-methoxy-2-propanol, 1,2-propanediamine, 2,6-dimethyl-5-heptenal, 1-(dimethylamino)-2-propanol, 3-methyl-3-pentanol, 2-ethylbutylamine, diethyl carbonate, n-butyl acetate, 2-hexanone, N-ethylethylenediamine, 2-methoxy-1-propanol, 1-ethoxy-2-propanol, 4-methyl-2-hexanamine, hexylamine, methoxycyclohexane, 2-(dimethylamino)ethanol, cyclohexylamine, N-ethyl-2-dimethylaminoethylamine, ethoxyethanol, 2-ethoxy-1-propanol, 1-methylpiperazine, 1,3-propanediamine, 2-heptanamine, N,N-diethylethylenediamine, 4-methyl-2-hexanone, 1,1,1-triethoxyethane, 1-methoxy-2-acetoxypropane, 4-methylpyridine, N,N'-diethyl-1,2-ethanediamine, 2,6-dimethylmorpholine, methyl hexanoate, 2-propoxyethanol, 1-propoxy-2-propanol, 2-heptanone, dimethylformamide, 2-isopropoxyethanol, 2-methylpiperazine, cyclohexanone, 1-heptanamine, 2-ethoxyethyl acetate, 1,4-butanediamine, 2,4-dimethylpyridine, 2-methoxy-3-methylpyrazine, 4-methoxy-4-methylpentan-2-one, 3-ethoxy-1-propanol, 3-methoxy-1-butanol, diglyme, 2-(diethylamino)ethanol, 2,2-diethoxyethanamine, 2-methoxy-N-(2-methoxyethyl)ethanamine, 2-(ethylamino)ethanol, 3-octanone, diacetone alcohol, diethylaminopropylamine, 2-ethylhexylamine, 1-butoxy-2-propanol, 2-butoxyethanol, 2-octanone, methyl heptanoate, triethylenediamine, N,N-dimethylpropanamide, 2-propyl-1-methoxypropanoate, 1,5-pentanediamine, cycloheptanone, 3,4-dimethylpyridine, 1-octanamine, benzylmethylamine, 1,1,3,3-tetramethoxypropane, dihexyl phthalate, diethylpropanolamine, 2-butoxyethyl acetate, diethyl sulfoxide, 2-(2-methoxyethoxy)ethanol, 4-methylbenzenemethanamine, diethylene glycol monoethyl ether, 2-propylcyclohexanone, trimethyl phosphate, 2-methyl-2,4-pentanediol, methyl benzoate, diethyl malonate and 2-methoxypyrimidine; in particular, said organic extracting agent is chosen from the group consisting of diethylamine, propanone, methyl acetate, tetrahydrofuran, ethyl acetate, butanone, diethoxymethane, isopropyl acetate, tert-butyl acetate, dioxane, 3-pentanone, 1,1-diethoxyethane, 2-pentanone, n-pentylamine, 1,3-dioxane, sec-butyl acetate, 1,2-diaminoethane, 1-methoxy-2-propanol, n-butyl acetate and 1-ethoxy-2-propanol.

FIG. 1a

FIG. 1b

FIG. 1c

EP 3 394 017 B1

FIG. 1d

FIG. 2

**EP 3 394 017 B1**

**Documents brevets cités dans la description**

- EP 0864554 A
- WO 03068716 A
- WO 9819982 A
- WO 2008054781 A
- WO 2007079431 A
- EP 939071 A
- WO 2008040969 A
- US 4902838 A
- WO 2009118628 A